# EUROPEAN PATENT SPECIFICATION

(11) **EP 4 146 340 B1**
(45) Date of publication and mention of the grant of the patent: **21.10.2026**
(21) Application number: 21799718.8
(22) Date of filing: 07.05.2021
(51) Int. Cl.: A61K 31/57, A61P 9/10, G01N 33/68, A61K 31/427

(54) **STATINS ADMINISTERED INTRANASALLY OR BY INHALATION FOR USE IN THE TREATMENT OF VIRAL RESPIRATORY DISEASES SELECTED FROM CORONAVIRUS, SARS-COV-2 INFECTION OR COVID-19**
INTRANASAL ODER INHALATIV VERABREICHTE STATINE ZUR VERWENDUNG BEI DER BEHANDLUNG VON VIRALEN ATEMWEGSERKRANKUNGEN, DIE AUS CORONAVIRUS, SARS-COV-2-INFEKTION ODER COVID-19 AUSGEWÄHLT SIND
STATINES ADMINISTREES PAR VOIE INTRANASALE OU PAR INHALATION POUR LE TRAITEMENT DE MALADIES RESPIRATOIRES VIRALES SELECTIONNEES DU CORONAVIRUS, DE L'INFECTION SARS-COV-2 OU DU COVID-19

(30) Priority: 07.05.2020 US 202063021618 P; 08.03.2021 US 202163158144 P
(43) Date of publication of application: 15.03.2023
(73) Proprietor: THE REGENTS OF THE UNIVERSITY OF CALIFORNIA, Oakland, CA 94607-5200 (US); The United States Government as represented by the Department of Veterans Affairs, Washington, DC 20402 (US); Beth Israel Deaconess Medical Center, Inc., Boston, MA 02215 (US); Invixa, Inc., Rancho Cordova, CA 95742 (US)
(72) Inventor: ZEKI, Amir A., Davis, California 95618 (US); KRISHNAN, Ramaswamy, Boston, Massachusetts 02215 (US); GHOSH, Chandra C., Rancho Cordova, California 95742 (US)
(74) Representative: HGF
(86) International application number: PCT/US2021/031327
(87) International publication number: WO 2021/226479

(56) References cited:
- WO-A1-2008/034832
- WO-A1-2020/214650
- US-A1- 2009 318 379
- US-A1- 2015 361 432
- CHANG SEOK LEE: "Simvastatin suppresses RANTES-mediated neutrophilia in polyinosinic-polycytidylic acid-induced pneumonia", EUROPEAN RESPIRATORY JOURNAL, vol. 41, no. 5, 26 July 2012 (2012-07-26), GB, pages 1147 - 1156, XP093156421, ISSN: 0903-1936, DOI: 10.1183/09031936.00050612
- SONGQUAN WU; YANG RUHUI; WANG GUANGLI: "Anti-asthmatic efect of pitavastatin through aerosol inhalation is associated with CD 4+ CD 25+ Foxp3+ T cells in an asthma mouse model", SCIENTIFIC REPORTS, vol. 7, 6084, 20 July 2017 (2017-07-20), pages 1 - 12, XP055744603, ISSN: 2045-2322, DOI: 10.1038/s41598-017-06476-6
- CASTIGLIONE VINCENZO, CHIRIACÒ MARTINA, EMDIN MICHELE, TADDEI STEFANO, VERGARO GIUSEPPE: "Statin therapy in COVID-19 infection", EUROPEAN HEART JOURNAL - CARDIOVASCULAR PHARMACOTHERAPY, vol. 6, no. 4, 1 July 2020 (2020-07-01), pages 258 - 259, XP055870555, ISSN: 2055-6837, DOI: 10.1093/ehjcvp/pvaa042

## Description

### CROSS-REFERENCES TO RELATED APPLICATIONS

This application claims priority to U.S. Provisional Application No. 63/021,618 filed May 7, 2020 and U.S. Provisional Application No. 63/158,144 filed March 8.

### STATEMENT AS TO RIGHTS TO INVENTIONS MADE UNDER FEDERALLY

### SPONSORED RESEARCH AND DEVELOPMENT

This invention was made with Government support. The Government has certain rights in this invention.

### BACKGROUND

A previously unknown coronavirus emerged in late 2019, and by early 2020 had spread into a global pandemic. The virus, SARS-CoV-2, can cause serious pulmonary complications, including severe respiratory failure, acute lung injury (ALI), Acute Respiratory Distress Syndrome (ARDS), pneumonia, sepsis, blood clots, and death, but in many cases is asymptomatic. The disease caused by the virus is known as COVID-19. By late April 2020, the COVID-19 pandemic had globally exceeded three million confirmed cases, with more than 200,000 deaths attributed to that infection.

The virus is believed to spread via respiratory droplets and/or aerosols, initially infecting epithelial cells of the nasopharynx, then the airways and lungs. SARS-CoV-2 gains access to epithelial cell cytoplasm by binding to a cell surface receptor, Angiotensin-Converting Enzyme 2 (ACE2, UniProtKB Q9BYF1). Viral entry requires both binding of the S ("spike") protein to ACE2, and its cleavage by TMPRSS2 (transmembrane serine protease 2, UniProtKB O15393), a serine protease also found on the extracellular surface of epithelial cells (M. Hoffman et al., Cell (2020) 181:271-80).

SARS-CoV-2 spreads easily from person to person, and many COVID-19 infections appear to be asymptomatic even though large amounts of virus are being shed from the nasopharynx. Some COVID-19 infections, however, cause severe disease, often requiring hospitalization and intensive care, and are sometimes fatal. Although there are a number of candidate therapeutic agents, and vaccines are in development, at present there is no effective treatment for COVID-19.

### BRIEF SUMMARY OF THE INVENTION

The invention is defined in the appended claims. Any references in the description to methods of treatment refer to the compounds, pharmaceutical compositions and medicaments of the present invention for use in a method for treatment of the human (or animal) body by therapy (or for diagnosis).

In some embodiments, the present invention provides a method for reducing viral respiratory infection in a subject in need thereof, the method comprising: administering a formulation intranasally or by inhalation to the subject having a viral respiratory infection, wherein the formulation comprises a therapeutically effective amount of a statin; and a pharmaceutically acceptable carrier.

In some embodiments, the present invention provides a method for treating a viral respiratory infection in a subject in need thereof, the method comprising: administering a formulation intranasally or by inhalation to the subject suffering from the viral respiratory infection or who may be exposed to the viral respiratory infection, wherein the formulation comprises a therapeutically effective amount of a statin; and a pharmaceutically acceptable carrier.

In some embodiments, the present invention provides a pharmaceutical composition comprising: a therapeutically effective amount of a statin; at least one additional therapeutic agent; and a pharmaceutically acceptable carrier.

In some embodiments, the present invention provides a pharmaceutical formulation for the treatment of a viral respiratory disease, the composition comprising: a therapeutically effective amount of a statin, or an isomer, enantiomer, or diastereomer thereof, and a pharmaceutically acceptable carrier suitable for administration by inhalation.

In some embodiments, the present invention provides a method for treating a SARS-CoV-2 virus infection in a subject in need thereof, the method comprising: administering a formulation intranasally or by inhalation to a subject suffering from the viral respiratory infection, wherein the formulation comprises a therapeutically effective amount of a statin; and a pharmaceutically acceptable carrier.

In some embodiments, the present invention provides a method for treating a SARS-CoV-2 virus infection in a subject in need thereof, the method comprising: administering a formulation intranasally or by inhalation to a subject who may be exposed to a SARS-CoV-2 virus, wherein the formulation comprises a therapeutically effective amount of a statin; and a pharmaceutically acceptable carrier.

In some embodiments, the present invention provides a method for reducing the severity of COVID-19 in a subject infected with SARS-CoV-2, the method comprising: administering a formulation intranasally or by inhalation to the infected subject, wherein the formulation comprises a therapeutically effective amount of a statin; and a pharmaceutically acceptable carrier.

In some embodiments, the present invention provides a method for blocking viral entry into a cell comprising administering a therapeutically effective amount of a statin, and wherein the virus is a SARS virus.

### BRIEF DESCRIPTION OF THE DRAWINGS

**FIG. 1** shows the reduction of cellular cholesterol in human bronchial epithelial cells (HBE1) after treatment with simvastatin for 48 hours. Simvastatin was applied at concentrations of 50, 100, 200, and 400 nM. Significant inhibition (p < 0.05) is indicated by an asterisk (*).
**FIG. 2** shows the reduction of cellular cholesterol in human bronchial epithelial cells (HBE1) after treatment with simvastatin for 48 hours. Simvastatin was applied at concentrations of 1, 5, 10, and 20 µM. Significant inhibition (p < 0.05) is indicated by an asterisk (*).
**FIGs. 3A-3C** show data from the ACE2 assay. **FIG. 3A** shows the capillary scan, **FIG. 3B** shows the MST Traces, and **FIG. 3C** shows the dose-response curve.
**FIG. 4** shows data from the statin ligands assay.
**FIG. 5** shows cell viability data of INS-102 and INS-103 after cells were treated with the compounds for 72 hours
**FIG. 6** shows cell viability data of INS-102 and INS-103 after cells were pre-treated with compounds for 6 to 24 hours.
**FIG. 7** shows cell viability data of INS-102 and INS-103 after cells were infected with the virus for 1 hour, and then INS-102 or INS-103 was added.
**FIG. 8** shows cell viability data of INS-102 and INS-103 after cells were infected with the virus for 24 hours, and then INS-102 or INS-103 was added. The compound was in contact for 48 hours with the infected cell.
**FIG. 9** shows cell viability data of INS-102 and INS-103 after cells were infected with the virus for 48 hours, and then INS-102 or INS-103 was added. The compound was in contact for 24 hours with the infected cell.
**FIG. 10** shows cell viability data of INS-102 and INS-103 where the cells were pre-treated with INS-102 or INS-103 for 6 hours, and then infected with the virus for 72 hours.
**FIG. 11** shows cell viability data of INS-102 and INS-103 where the cells were pre-treated with INS-102 or INS-103 for 24 hours, and then infected with the virus for 72 hours.
**FIG. 12** shows cell viability data of INS-102 and INS-103 where the cells were pre-treated with INS-102 or INS-103 for 1 hour, and then infected with the virus for 72 hours.
**FIG. 13** shows viral load data for INS-102.
**FIG. 14** shows viral load data for INS-103.
**FIG. 15** shows the Luminex experiment IL-6 production for INS-102 and INS-103.
**FIG. 16** shows the Luminex experiment IL-8 production for INS-102 and INS-103.
**FIG. 17** shows the Luminex experiment IL-10 production for INS-102 and INS-103.
**FIG. 18** shows the Luminex experiment IL-1α production for INS-102 and INS-103.
**FIG. 19** shows the ELISA experiment IL-6 production for INS-102 and INS-103.
**FIG. 20** shows the schematic study design of the hamster model.
**FIG. 21** shows animals treated with control group maintained relatively constant weight. In contrast, animals treated with SARS-CoV-2 and no drug experienced weight loss, whereas animals treated with pitavastatin had less weight loss.
**FIG. 22** shows viral titers from nasal swabs.
**FIG. 23** shows comparison of the viral titers.
**FIG. 24** shows the viral titer in the nasal swabs (left panel) and tracheas (right panel) of hamsters treated with pitavastatin and controls on Day 3 post-infection.
**FIG. 25** shows the viral titer in lung samples (R2- right lung lobes- right medial; R4 right lung lobes - right post-caval) of hamsters treated with pitavastatin and controls on Day 3 post-infection.
**FIG. 26** shows the histopathology of lungs from the treated and control samples.
**FIG. 27** shows the blinded scoring of lung inflammation grade of all the infected animals based on the average +/- SEM of the lung histopathology graded according to severity of inflammation.
**FIG. 28** shows lung histopathology scoring based on the percentage of affected lung.
**FIG. 29** shows data for INS-102 administered as a pre-treatment 6 hours before SARS-CoV-2 (MOI 0.01) infection. Viral load is measured by RT-PCR (ORF1ab gene) conducted at 24 hours post infection. The decrease in viral load is enhanced with the combination of remdesivir or dexamethasone and the statin as compared to the monotherapies.
**FIG. 30** shows data for INS-103 and SARS-CoV-2 (MOI 0.01) mixed 1 hour at room temperature before addition to cells. Viral load is measured by RT-PCR (ORF1ab gene) conducted at 24 hours post infection. The decrease in viral load is enhanced with the combination of remdesivir or dexamethasone and the statin as compared to the monotherapies.
**FIG. 31** shows data for INS-104 and SARS-CoV-2 (MOI 0.01) mixed 1 hour at room temperature before addition to cells. Viral load is measured by RT-PCR (ORF1ab gene) conducted at 24 hours post infection. Statin effect is synergistic to dexamethasone and remdesivir. INS-104 alone decreases viral load by 22% of the control. Dexamethasone alone decreases by 46%.
**FIG. 32** shows details of the cell set-up and treatment of INS-102.
**FIG. 33** shows statistical analysis of cell treatment with INS-102.
**FIG. 34** shows details of the cell set-up and treatment of INS-103.
**FIG. 35** shows statistical analysis of cell treatment with INS-103.
**FIG. 36** shows cell studies set-up for INS102 and INS103.
**FIG. 37** shows statistical analysis of the Luminex assay on IL-6 production.
**FIG. 38** shows statistical analysis of the Luminex assay on IL-8 production.
**FIG. 39** shows statistical analysis of the Luminex assay on IL-10 production.
**FIG. 40** shows statistical analysis of the Luminex assay on IL-1α production.
**FIG. 41** shows statistical analysis of the ELISA assay on IL-6 production.
**FIG. 42A** shows INS-102 combination data wherein the statin is at 1 µM. **FIG. 42B** shows INS-102 combination data wherein the statin is at 0.1 µM. **FIG. 42C** shows INS-102 combination data wherein the statin is at 10 µM.
**FIG. 43** shows INS-103 combination data wherein the statin is at 1 µM.
**FIG. 44A** shows INS-104 combination data wherein the statin is at 5 µM, dexamethasone is at 1 nM, and remdesivir is at 1 nM. **FIG. 44B** shows INS-104 combination data wherein the statin is at 5 µM, dexamethasone is at 1 nM, and remdesivir is at 10 nM.
**FIG. 45** shows low dose INS-102 pre-treatment for 6 hrs followed by SARS-CoV-2 Infection, with measurement 24 hours post-infection.
**FIG. 46** shows low dose INS-103 pre-treatment for 6 hrs followed by SARS-CoV-2 Infection, with measurement 24 hours post-infection.
**FIG. 47** shows high dose INS-104 Pre-treatment for 6 hrs followed by SARS-CoV-2 Infection, with measurement 24 hours post-infection.
**FIG. 48** shows low dose INS-102 pre-mixed with SARS-CoV-2 for 1 hr at Room Temperature prior to incubating with Calu-3 cells, with measurement 24 hours post-infection.
**FIG. 49** shows medium dose INS-103 pre-mixed with SARS-CoV-2 for 1 hr at Room Temperature prior to incubating with Calu-3 cells, with measurement 24 hours post-infection.
**FIG. 50** shows low dose INS-104 pre-mixed with SARS-CoV-2 for 1 hr at Room Temperature prior to incubating with Calu-3 cells, with measurement 24 hours post infection.
**FIG. 51** shows medium dose INS-104 pre-mixed with SARS-CoV-2 for 1 hr at Room Temperature prior to incubating with Calu-3 cells, with measurement 24 hours post infection.
**FIG. 52** shows low-dose INS-103 pre-treatment for 6 hrs followed by SARS-CoV-2 Infection, with measurement 72 hours post-infection.
**FIG. 53** shows high-dose INS-103 pre-treatment for 6 hrs followed by SARS-CoV-2 Infection, with measurement 72 hours post-infection.
**FIG. 54** shows INS-103 Pre-mixed with SARS-CoV-2 for 1 hr at Room Temperature prior to incubating with Calu-3 cells, with measurement 72 hours post-infection.
**FIG. 55** shows INS-104 pre-mixed with SARS-CoV-2 for 1 hr at Room Temperature prior to incubating with Calu-3 cells, with measurement 72 hours post-infection.

### DETAILED DESCRIPTION

### I. GENERAL

The need for novel antiviral agents is met by new methods using statins, which offer a new mechanism to inhibit or prevent viral entry into cells and reduce symptoms by delivering statins directly to the nasal passages and airways by inhalation.

Both ACE2 and TMPRSS2, and other receptors, are known to associate with lipid rafts in the cell membrane. Lipid rafts are membrane microdomains that that are more rigid and tightly-packed than the surrounding membrane. These rafts contain elevated concentrations of cholesterol and sphingolipids. Without being bound by any particular theory, it is currently believed that lipid rafts are necessary for the support and function of at least some surface receptors.

Statins are 3-hydroxy-3-methylglutaryl-coenzyme A reductase (HMG-CoA reductase) inhibitors that block the biosynthesis of mevalonate (MA) and the downstream isoprenoid lipids farnesyl-pyrophosphate (FPP) and geranylgeranyl-pyrophosphate (GGPP). At present, in the United States statins are only approved for oral administration as lipid lowering agents.

Administration of statins directly to the airways delivers an effective amount of the statin to airway epithelium and the airway smooth muscle which is not attained using oral administration. When inhaled, statins reduce intracellular cholesterol synthesis in airway epithelial cells, which reduces lipid rafts. ACE2 activity is inhibited when not supported by a lipid raft, thus reducing or eliminating the entry pathway for SARS-CoV-2 and other viruses that rely on ACE2 for entry. This reduces the rate of infection and the resulting symptoms. Similarly, viruses that rely on other surface proteins for entry are also inhibited or reduced by administration of inhaled statins if the surface protein relies on lipid rafts for their structure and/or function. WO2008034832 discloses the use of statins for treating viral infections including influenza, hepatitis and RSV.

### II. DEFINITIONS

Unless specifically indicated otherwise, all technical and scientific terms used herein have the same meaning as commonly understood by those of ordinary skill in the art to which this invention belongs. In addition, any method or material similar or equivalent to a method or material described herein can be used in the practice of the present invention. For purposes of the present invention, the following terms are defined.

"A," "an," or "the" as used herein not only include aspects with one member, but also include aspects with more than one member. For instance, the singular forms "a," "an," and "the" include plural referents unless the context clearly dictates otherwise. Thus, for example, reference to "a cell" includes a plurality of such cells and reference to "the agent" includes reference to one or more agents known to those skilled in the art, and so forth. "A and/or B" is used herein to include all of the following alternatives: "A", "B", "A or B", and "A and B."

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges, and are also encompassed within the disclosure, subject to any specifically excluded limit in the stated range. Where the stated range includes one or both of the limits, ranges excluding either or both of those included limits are also included in the disclosure.

All ranges disclosed herein also encompass any and all possible sub-ranges and combinations of sub-ranges thereof. Any listed range can be recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination. All combinations of the embodiments pertaining to the disclosure are specifically embraced by the present disclosure and are disclosed herein just as if each and every combination was individually and explicitly disclosed. In addition, all subcombinations of the various embodiments and elements thereof are also specifically embraced by the present disclosure and are disclosed herein just as if each and every such sub-combination was individually and explicitly disclosed herein.

"Statins" are small molecule HMG-CoA reductase inhibitors. Statins were designed to block the mevalonate metabolic pathway, and thereby reduce the production of FPP, GGPP, and cholesterol in the body. Suitable statins of the disclosure include, without limitation, simvastatin, pitavastatin, rosuvastatin, atorvastatin, lovastatin, fluvastatin, mevastatin, cerivastatin, tenivastatin, and pravastatin, and isomers, enantiomers, and diastereomers thereof. Hydrophobic statins include simvastatin, pitavastatin, and other statins with a similar hydrophobicity. Hydrophilic statins include pravastatin, and other statins with a similar hydrophilicity.

The term "therapeutically effective amount" refers to the amount of statin (or an isomer, enantiomer, diastereomer) or mixture thereof that is sufficient for reducing viral respiratory infection when administered by inhalation. The reduction of viral respiratory infection can include reduction in damage to airway epithelium, reduction or prevention of symptoms, including for example severe symptoms such as ARDS, viral pneumonia, pulmonary emboli, respiratory failure, sepsis, acute lung injury (ALI), or death. Subjects who are infected with some viruses, such as for example SARS-CoV-2, may exhibit no symptoms, or only mild symptoms, which leads to the unwitting infection of others who are contacted by such subjects. Accordingly, another measurable reduction of viral respiratory infection comprises a reduction in the viral burden (the amount of virus in the body of a subject, e.g., as measured or estimated using a PCR-based assay), or of the amount of virus shed by a subject infected with a respiratory viral disease.

The term "prophylactically" or "prophylactic treatment" refers to a preventative treatment method, which can guard against the development or progression of a disease or symptoms of the disease, and/or minimize the adverse effects of a disease. In some cases, a prophylactic treatment includes the prevention or a substantial reduction of infection (e.g., viral entry into cells or tissue), and thus prevent or substantially reduce the disease.

A "sub-therapeutic dose" refers to the dose of one or more agents in a synergistic or potentiated combination formulation, method, or system, wherein the dose of the agent is reduced to a level that would be insufficient or sub-therapeutic when administered alone or as part of a non-synergistic or combination formulation, method, or system, but is sufficient for therapeutic use when administered as part of the synergistic or combination formulation, method, or system. The sub-therapeutic dose of an agent can be about 90%, 80%, 75%, 70%, 65%, 60%, 55%, 50%, 45%, 40%, 35%, 30%, 25%, 20%, 19%, 18%, 17%, 16%, 15%, 14%, 13%, 12%, 11%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, or 0.1% of the effective dose of the agent when administered by inhalation as part of a non-synergistic formulation, method, or system according to the present disclosure.

The term "pharmaceutically acceptable carrier" refers to an excipient that is non-toxic to the subject at the amount and concentration in which it is administered, within which the statin may be dissolved and/or suspended. In the practice of the instant disclosure, pharmaceutically acceptable carriers are suitable for administration by inhalation. The pharmaceutically acceptable carrier can aid in the administration of an active agent to and absorption by a subject. Pharmaceutical excipients useful in the present invention include, but are not limited to, binders, fillers, disintegrants, lubricants, coatings, sweeteners, flavors and colors. One of skill in the art will recognize that other pharmaceutical excipients are useful in the present invention.

The term "non-viral airway disease" refers to a non-viral disease or disorder in which obstruction, or restriction or interference with airflow into and out of the lung is a substantial symptom. This obstruction may result from constriction of airway smooth muscle (bronchoconstriction) and/or over-secretion of mucus and/or inflammation. Non-viral lung airway diseases include, without limitation, asthma; exercise-induced bronchoconstriction (or exercise-induced asthma); chronic obstructive pulmonary disease (COPD) which may include emphysema, chronic bronchitis, and/or alpha-1 antitrypsin deficiency (AATD); asthma-COPD overlap syndrome (ACOS) (also known as asthma-COPD overlap or ACO); cystic fibrosis; acute bronchitis; eosinophilic bronchitis; constrictive bronchiolitis; infectious bronchiolitis; and bronchiectasis.

The term "viral respiratory infection" refers to a disease or disorder in which infection of airway epithelial cells and/or airway smooth muscle is a substantial symptom. Non-limiting exemplary viral respiratory infections include pulmonary infections by coronaviruses (including, for example, SARS-CoV, MERS-CoV, and SARS-CoV-2), morbillivirus (including, for example, measles and distemper), bunyavirus (including, for example, hantavirus and Crimean-Congo hemorrhagic fever virus), arenavirus (including, for example, Lassa virus and Junin virus), influenza, rhinovirus (including the "common cold"), and adenovirus (including, for example, HAdV-B and HAdV-C). The invention is limited to coronavirus, SARS-CoV-2 or COVID-19.

An "antiviral" agent is a compound capable of inhibiting the growth, replication, infectivity, or other factors that reduce or eliminate the effect of a virus on a mammalian subject.

"Reduce" or "inhibits" refers to the ability of a compound to lessen the symptoms associated with an infection. For example, the compound can lower the virus titer or viral load after administration to a subject in need thereof. In another non-limiting example, the compound can reduce or inhibit the level of proteins, cytokines, or immune responses in a subject after administration of the compound.

The term "subject" refers to animals such as mammals, including, but not limited to, primates (*e.g*., humans), cows, sheep, goats, horses, dogs, cats, rabbits, rats, mice and the like. In certain embodiments, the subject is a human

The term "administering" refers to oral administration, administration as a suppository, topical contact, parenteral, intravenous, intraperitoneal, intramuscular, intralesional, intranasal or subcutaneous administration, intrathecal administration, or the implantation of a slow-release device e.g., a mini-osmotic pump, to the subject.

"Treat", "treating" and "treatment" refers to any indicia of success in the treatment or amelioration of an injury, pathology, condition, or symptom (e.g., pain), including any objective or subjective parameter such as abatement; remission; diminishing of symptoms or making the symptom, injury, pathology or condition more tolerable to the patient; decreasing the frequency or duration of the symptom or condition; or, in some situations, preventing the onset of the symptom. The treatment or amelioration of symptoms can be based on any objective or subjective parameter; including, e.g., the result of a physical examination.

The term "virus titer" or "viral load" refers to the quantity of a virus in an amount of fluid, which may be measured volumetrically. Viral load can be expressed as viral or infectious particles per mL. A higher virus titer or viral load may correlate with the severity of an active viral infection. Tests for determining viral load may include, but are not limited to reverse transcription-polymerase chain reaction (RT-PCR) tests, branched DNA (bDNA) tests, Qualitative Transcription-Mediated Amplification Assays, and nucleic acid sequence-based amplification (NASBA) tests.

### III. FORMULATIONS

The disclosed compositions are formulated to be suitable for inhalation, in which the composition is inhaled or sprayed into the nasopharynx and lungs. Ideally, the composition is administered in such a manner that it is distributed evenly throughout the nasal passages and airways, providing an effective amount of statin directly to the nasopharyngeal and airway epithelium. This is generally accomplished by administering the formulation as a population of small particles suspended in air or a gas, where the distribution of particle sizes affects the distance that the particles will penetrate distal to the trachea. The composition may be in the form of a solution, suspension, powder, or other suitable form for pulmonary administration. See, for example, H.M. Mansour et al., Int J Nanomed (2009) 4:299-319. These compositions are administered to the lungs, for example, in an aerosol, atomized, nebulized, or vaporized form through appropriate devices known in the art. The amount of the composition administered can be controlled by providing a valve to deliver a metered amount, as in a metered dose inhaler (MDI) that delivers a fixed dose in a spray with each actuation of the device. In this way, an appropriate dose (e.g., a therapeutically effective amount) of the composition can be delivered reliably from a device that contains multiple doses.

The formulation employed for delivery will typically be designed to work with a particular mode of administration, such as an aerosol formulation, a nebulizer formulation, or a dry powder formulation.

Formulations of the disclosure contain a therapeutically effective amount of a statin. In some embodiments, the therapeutically effective amount is at least about 0.005, 0.01, 0.02, 0.03, 0.04, 0.05, 0.06, 0.07, 0.08, 0.09, 0.1, 0.2, 0.3, 0.4, 0.5, 0.6, 0.7, 0.75, 0.8, 0.9, 1.0, 1.5, 2.0, 3.0, 4.0, 5.0, 6.0, 7.0, 8.0, 9.0, 10, 12, 14, 15, 17, 20, 25, 30, 35, 40, 45, 50, 55, 60, 65, 70, 75, 80, 85, 90, 95, or 100 µg. In some embodiments, the therapeutically effective amount is at least about 0.1, 0.2, 0.3, 0.4, 0.5, 0.6, 0.7, 0.75, 0.8, 0.9, 1.0, 1.5, 2.0, 3.0, 4.0, 5.0, 6.0, 7.0, 8.0, 9.0, 10, 12, 14, 15, 17, 20, 25, 30, 35, 40, 45, 50, 55, 60, 65, 70, 75, 80, 85, 90, 95, or 100 mg. In some embodiments, the therapeutically effective amount will be no greater than about 100, 95, 90, 85, 80, 75, 70, 65, 60, 55, 50, 45, 40, 35, 30, 25, 20, 15, 10, 9, 8, 7, 6, 5, 4, 3, 2, 1, 0.9, 0.8, 0.7, 0.6, 0.5, 0.4, 0.3, 0.2, 0.1, 0.09, 0.08, 0.07, 0.06, 0.05, 0.04, 0.03, 0.02, 0.01, or 0.005 mg.

In some embodiments, the formulation further comprises an additional therapeutic agent. As the additional therapeutic agent is also not subject to hepatic first pass metabolism, it too may be administered at doses that are generally lower than the dose effective in oral or parenteral administration. In some embodiments, the effective dose when administered by inhalation is less than about 90%, 80%, 75%, 70%, 65%, 60%, 55%, 50%, 45%, 40%, 35%, 30%, 25%, 20%, 19%, 18%, 17%, 16%, 15%, 14%, 13%, 12%, 11%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, or 0.1% of the dose normally recommended for oral administration.

In some embodiments, the formulation for inhalation is designed to deliver the statin and/or additional therapeutic agent to the lower respiratory tract. In some embodiments, the formulation is designed to deliver the statin and/or additional therapeutic agent to the systemic circulation by absorption through the lower respiratory tract. Techniques and methods for making formulations for inhalation that target the lower respiratory tract or the systemic circulation are known: see, e.g., J.G. Weers et al., AAPS Pharm Sci Tech (2019) 20(3):103; J.S. Patton et al., Proc Am Thorac Soc (2004) 1(4):338-44. Systemically targeted formulations for inhalation are useful for reducing viral infection in non-respiratory tissues, as some viruses target tissues other than airway epithelium. Tissues that can be targeted by delivery of inhaled statins to the systemic circulation include, without limitation, the circulatory system including the heart, arteries, veins, and capillaries; the gut, including the esophagus, stomach, small and large intestine; and others.

The formulation can contain any pharmaceutically active statin or a mixture thereof. In some embodiments, the statin is selected from the group consisting of simvastatin, pitavastatin, rosuvastatin, atorvastatin, lovastatin, fluvastatin, mevastatin, cerivastatin, tenivastatin, and pravastatin, and isomers, enantiomers, and diastereomers thereof. In some embodiments, the statin is selected from the group consisting of simvastatin, pitavastatin, atorvastatin, lovastatin, and pravastatin. In some embodiments, the statin is selected from the group consisting of simvastatin and pitavastatin. In some embodiments, the statin is simvastatin. In some embodiments, the statin is pitavastatin.

Statins can be formulated as spongy porous microspheres. Suitable microspheres are prepared by a two-step process. In the first step, a submicron oil-in-water (O/W) emulsion is prepared by high pressure homogenization of long-chain saturated phospholipids (for example, distearoylphosphatidylcholine) in water or phosphate-buffered saline. This results in phospholipids being incorporated as emulsifiers at the oil/water interface.

The second step involves mixing the API dropwise along with matrix forming agents such as sodium alginate (controlled gelation with calcium), chitosan, trehalose, raffinose, leucine, hydroxypropylmethylcellulose, hydroxypropyl-β-cyclodextrin and/or a dispersing agent such as Pluronics^{®} F-68 (a polyoxyethylene-polyoxypropylene diblock copolymer) into the oil in water emulsion. The resulting mixture is nebulized for administration, or is spray dried for administration in a dry powder formulation.

Formulations of the disclosure may further include an additional therapeutic agent, which can be selected from antiviral agents, such as RNA polymerase inhibitors, TMPRSS2 inhibitors, inhibitors of viral proteases, inhibitors of viral regulatory proteins, inhibitors of viral capsid assembly, inhibitors of viral entry, inhibitors of viral membrane coating or uncoating, and immune stimulatory agents, for example IFNy. Examples of antiviral agents include, without limitation, chloroquine or a salt thereof, hydroxychloroquine or a salt thereof, amantadine, rimantadine, lopinavir, ritonavir, umifenovir, remdesivir, favipiravir, nelfinavir mesylate, azithromycin, bafilomycin, camostat or a salt thereof, darunavir, oseltamivir, and ribavirin. In some embodiments, the formulation comprises an additional antiviral agent, selected from RNA polymerase inhibitors, TMPRSS2 inhibitors, inhibitors of viral proteases, inhibitors of viral regulatory proteins, inhibitors of viral capsid assembly, inhibitors of viral entry, and inhibitors of viral membrane coating or uncoating. In some embodiments, the additional antiviral agent is chloroquine phosphate, hydroxychloroquine sulfate, amantadine, rimantadine, lopinavir, ritonavir, umifenovir, remdesivir, favipiravir, nelfinavir mesylate, azithromycin, bafilomycin, camostat mesylate, darunavir, oseltamivir, or ribavirin. In some embodiments, the additional antiviral agent is chloroquine phosphate, hydroxychloroquine sulfate, remdesivir, favipiravir, nelfinavir mesylate, azithromycin, bafilomycin, camostat mesylate, or darunavir. In some embodiments, the additional antiviral agent is chloroquine or a salt or ester thereof. In some embodiments, the salt is chloroquine phosphate. In some embodiments, the additional antiviral agent is hydroxychloroquine or a salt or ester thereof. In some embodiments, the salt is hydroxychloroquine sulfate. In some embodiments, the additional antiviral agent is camostat or a salt or ester thereof. In some embodiments, the salt is camostat mesylate. In some embodiments, a combination of two or more additional antiviral agents is included. In some embodiments, the combination comprises azithromycin and chloroquine or a salt or ester thereof. In some embodiments, the combination comprises azithromycin and hydroxychloroquine or a salt or ester thereof. In some embodiments, the combination comprises azithromycin and camostat or a salt or ester thereof. In some embodiments, the additional therapeutic agent is remdesivir. In some embodiments, the additional therapeutic agent is dexamethasone. In some embodiments, the additional therapeutic agent is dexamethasone, and further comprises remdesivir.

Other compounds and treatments that are found to inhibit or interact with viral proteins, or that prevent viral use of host proteins, can also be used. In the case of SARS-CoV-2, interactions between viral proteins and host proteins and processes has recently been reported (see, e.g., D.E. Gordon et al., Nature (2020) doi. org/10.1038/s41586-020-2286-9). Compounds have been identified that interfere with viral processes *in vitro,* including without limitation bromodomain inhibitors, Sigma 1 and/or Sigma 2 receptor targeting drugs, antihistamines, protein translation inhibitors, antipsychotic drugs, antidepressants, and anti-anxiety drugs. In some embodiments, the additional antiviral agent is a bromodomain inhibitor (BETS), a Sigma 1 and/or Sigma 2 receptor targeting drug (for example without limitation, PB28), an antihistamine (for example without limitation, clemastine and/or cloperastin), a protein translation inhibitor (for example without limitation, zotatifin, ternatin-4, and/or plitidepsin), an antipsychotic drug (for example without limitation, haloperidol and/or cloperazine); or siramesine (an antidepressant and anti-anxiety drug). In some embodiments, the antiviral agent is PB28, clemastine, cloperastin, zotatifin, ternatin-4, plitidepsin, haloperidol, cloperazine, or siramesine.

Formulations of the disclosure may further include an additional therapeutic agent, which can be selected from β-agonists; corticosteroids; muscarinic antagonists; RhoA inhibitors; GGTase-I or -II inhibitors; ROCK1 and/or ROCK2 inhibitors; soluble epoxide hydrolase inhibitors; fatty acid amide hydrolase inhibitors; leukotriene receptor antagonists; phosphodiesterase-4 inhibitors such as roflumilast; 5-lipoxygenase inhibitors such as zileuton; mast cell stabilizers such as nedocromil; squalene synthase inhibitors such as lapaquistat, zaragozic acid, and RPR 107393; inhibitors of farnesyl pyrophosphate synthase, including without limitation bisphosphonates such as alendronate, etidronate, clodronate, tiludronate, pamidronate, neridronate, olpadronate, ibadronate, risedronate, zoledronate; theophylline; anti-IL5 antibodies or antibody derivatives; anti-IgE antibodies or antibody derivatives; anti-IL5 receptor antibodies or antibody derivatives; anti-IL13/4 receptor antibodies or antibody derivatives; biologics such as mepolizumab, reslizumab, benralizumab, omalizumab, and dupilumab; β-agonist and muscarinic antagonist combinations, including both long- and short-acting formulations; β-agonist and corticosteroid combinations, including both long- and short-acting formulations; corticosteroids and muscarinic antagonist combinations, including both long- and short-acting formulations; and β-agonist, corticosteroid, and muscarinic antagonist combinations, including both long- and short-acting formulation.

An antibody derivative is a protein capable of binding an antigen that is similar to or based on an antibody. Examples of antibody derivatives include nanobodies, diabodies, triabodies, minibodies, F(ab')2 fragments, F(ab)v fragments, single chain variable fragments (scFv), single domain antibodies (sdAb), and functional fragments thereof.

Suitable corticosteroids for use as an additional therapeutic agent include, without limitation: beclomethasone, fluticasone, budesonide, mometasone, flunisolide, alclometasone, beclomethasone, betamethasone, clobetasol, clobetasone, clocortolone, desoximetasone, dexamethasone, diflorasone, diflucortolone, flurclorolone, flumetasone, fluocortin, fluocortolone, fluprednidene, fluticasone, fluticasone furoate, halometasone, meprednisone, mometasone, mometasone furoate, paramethasone, prednylidene, rimexolone, ulobetasol, amcinonide, ciclesonide, deflazacort, desonide, formocortal, fluclorolone acetonide, fludroxycortide, fluocinolone acetonide, fluocinonide, halcinonide, and triamcinolone acetonide.

Muscarinic antagonists are anticholinergic agents that block the muscarinic acetylcholine receptor, and can therefor block bronchoconstriction. Suitable muscarinic antagonists for use as an additional therapeutic agent include, without limitation: ipratropium bromide, tiotropium, glycopyrrolate, glycopyrronium bromide, revefenacin, umeclidinium bromide, aclidinium, trospium chloride, oxitropium bromide, oxybutynin, tolterodine, solifenacin, fesoterodine, and darifenacin.

Beta-agonists are compounds that activate β2-adrenergic receptors, and are used to relax airway smooth muscle. Suitable beta-agonists (β-agonists) for use as an additional therapeutic agent include, without limitation: albuterol, arformoterol, buphenine, clenbuterol, bopexamine, epinephrine, fenoterol, formoterol, isoetarine, isoproterenol, orciprenaline, levoalbutamol, levalbuterol, pirbuterol, procaterol, ritodrine, albuterol, salmeterol, terbutaline, arbutamine, brefonalol, bromoacetylalprenololmenthane, broxaterol, cimaterol, cirazoline, etilefrine, hexoprenaline, higenamine, isoxsuprine, mabuterol, methoxyphenamine, oxyfedrine, ractopamine, reproterol, rimiterol, tretoquinol, tulobuterol, zilpaterol, and zintero.

ROCK inhibitors inhibit the enzyme Rho Kinase (ROCK1 and/or ROCK2). Suitable ROCK inhibitors include, for example, 1-methyl-5-(1H-pyrrolo[2,3-b]pyridin-4-yl)-1H-indazole ("TS-f22", M. Shen et al., Sci Rep (2015) 5:16749), (1*S*)-2-amino-1-(4-chlorophenyl)-1-[4-(1*H*-pyrazol-4-yl)phenyl]ethanol ("AT13148", T.A. Yap et al., Clin Cancer Res (2012) 18(14):3912-23), N-(6-fluoro-1*H*-indazol-5-yl)-6-methyl-2-oxo-4-[4-(trifluoromethyl)phenyl]-3,4-dihydro-1*H*-pyridine-5-carboxamide ("GSK429286A", E. Ahler et al., Mol Cell (2019) 74(2):393-408e20), 1-[(3-hydroxyphenyl)methyl]-3-(4-pyridin-4-yl-1,3-thiazol-2-yl)urea ("RKI-1447," H. Wang et al., Cancer Res (2017) 77(8):2148-60), and 4-[(1*R*)-1-aminoethyl]-N-pyridin-4-ylcyclohexane-1-carboxamide ("Y-27632", Y-C. Liao et al., Cell (2019) 179(1):147-64.e20). Suitable RhoA inhibitors include compounds such as N-[1-(4-chloroanilino)-1-oxopropan-2-yl]oxy-3,5-bis(trifluoromethyl)benzamide ("CCG-1423", D.A. Lionarons et al., Cancer Cell (2019) 36(1):68-83.e9). Suitable GGTI inhibitors include compounds such as N-(1-amino-1-oxo-3-phenylpropan-2-yl)-4-[2-(3,4-dichlorophenyl)-4-(2-methylsulfanylethyl)-5-pyridin-3-ylpyrazol-3-yl]oxybutanamide ("GGTI-DU40", Y.K. Peterson et al., J Biol Chem (2006) 281:12445-50), and (2*S*)-2-[[4-[[(2*R*)-2-amino-3-sulfanylpropyl]amino]-2-naphthalen-1-ylbenzoyl]amino]-4-methylpentanoic acid 2,2,2-trifluoroacetic acid ("GGTI-297", P.A. Subramani et al., Bioinformation (2015) 11(5):248-53). Suitable soluble epoxide hydrolase inhibitors include compounds such as, for example, 1-(1-acetylpiperidin-4-yl)-3-(1-adamantyl)urea ("AR9281", R.H. Ingraham et al., Curr Med Chem (2011) 18(4):587-603), 1-(1-propanoylpiperidin-4-yl)-3-[4-(trifluoromethoxy)phenyl]urea ("TPPU", Y-M. Kuo et al., Mol Neurobiol (2019) 56:8451-74).

Suitable fatty acid amide hydrolase inhibitors include, without limitation, compounds such as 4-hydroxy-N-[(5Z,8Z,11Z,14Z)-icosa-5,8,11,14-tetraenyl]benzamide ("AM-1172", C.J. Hillard et al., J Mol Neurosci (2007) 33:18-24), N-Phenyl-4-(3-phenyl-1,2,4-thiadiazol-5-yl)-1-piperazinecarboxamide ("JNJ 1661010", T. Lowin et al., Arth Res Ther (2015) 17:321), and *N*-3-pyridinyl-4-[[3-[[5-(trifluoromethyl)-2-pyridinyl]oxy]phenyl]methyl]-1-piperidinecarboxamide ("PF-3845", S. Ghosh et al., J Pharmacol Exp (2015) 354(2):111-20). Suitable leukotriene receptor antagonists include, without limitation, compounds such as zafirlukast, montelukast, and zileuton.

### (a) Aerosol formulations

Aerosols are suspensions of small solid particles or liquid droplets, typically having an average diameter < 10 µm, suspended in air or another gas. Aerosol formulations for delivering drugs to the respiratory tract are known in the art. See for example, A. Adjei et al., J Pharm Res (1990) 1:565-69; P. Zanen et al., J Int J Pharm (1995) 114:111-15; I. Gonda, Crit Rev Ther Drug Carrier Syst (1990) 6:273-313; Anderson et al., Am Rev Respir Dis, (1989)140:1317-24.

Compositions for aerosol administration via pressurized metered dose inhalers (pMDIs) can be formulated as solutions or suspensions. Solution compositions can be more convenient to manufacture, as the active agent is completely dissolved in the propellant vehicle and avoids the physical stability problems (such as particle aggregation) sometimes associated with suspension compositions. If the agent is not sufficiently soluble in the propellant, a co-solvent such as ethanol can be used to provide enhanced solubility in a pharmaceutical composition for administration by pMDI. In some embodiments, the formulation comprises a statin dissolved in a propellant and a co-solvent.

Suspension formulations can include small, solid particles of the pharmaceutical agent, typically having an average diameter of less than about 10 µm. Such formulations can be prepared by grinding or milling a crystalline form of the agent, or by spray-drying a solution containing the agent. In some embodiments, the formulation comprises a powdered statin, a propellant, and a suspending vehicle. In some embodiments, the suspending vehicle is selected from a cyclodextrin, PEG400, PEG1000, and propylene glycol (1,2-propane diol).

The pharmaceutical compositions may be formulated with one or more suitable propellants, such as, for example, hydrofluoroalkanes, CO₂, or other suitable gases. In some embodiments, a surfactant may be added to reduce the surface and interfacial tension between the composition, the propellant, and the co-solvent, if present. The surfactant may be any suitable, non-toxic compound which is non-reactive with the other pharmaceutical composition components and which reduces the surface tension and/or interfacial tension between the composition, the propellant, and co-solvent to the desired degree. In some embodiments, the formulations do not require a surfactant to produce and/or maintain a stable pharmaceutical composition solution under normal operating conditions, and may be surfactant-free.

### (b) Nebulizer formulations

"Nebulization" refers to reduction of a liquid to a fine spray or mist. Small liquid droplets of uniform size are produced from a larger body of a liquid formulation in a controlled manner, typically having an average particle size of about 0.5 µm to about 10 µm. Nebulization can be achieved by any suitable means, including a mechanical nebulizer, such as a Respimat^{®} Soft Mist nebulizer in which the formulation is squeezed through nozzles under spring pressure; a jet nebulizer, in which a compressor compresses air or oxygen to flow through the liquid at high velocity, forming a mist; an ultrasonic wave nebulizer, in which a piezoelectric transducer oscillating at an ultrasonic frequency is placed in contact with the liquid formulation, the vibration forming a mist or aerosol; or a vibrating mesh nebulizer, in which a mesh or membrane with small holes is vibrated at the surface of the liquid reservoir, forming a fine mist. Nebulizers using any of these techniques are commercially available. When the active ingredients are adapted to be administered, either together or individually, via nebulizer(s) they can be in the form of a nebulized aqueous suspension or solution, with or without a suitable pH or tonicity adjustment, either as a unit dose or multidose device.

Formulations used in nebulizer administration are typically, but not necessarily, mainly aqueous solutions. In cases in which the agent to be administered is only sparingly soluble in water, pharmaceutically acceptable co-solvents such as ethanol can be added to dissolve or help dissolve the agent. Alternatively, the formulation can be a suspension of suitably sized particles suspended in a mainly aqueous carrier. Agents can also be formulated as solid lipid microparticles (SLM), solid lipid nanoparticles (SLN), or liposomes, and suspended in a liquid carrier for nebulization or aerosolization. See, e.g., M. Paranjpe et al., Int J Mol Sci (2014) 15:5852-73; M.J. de Jesús Valle et al., J Antibiot (Tokyo) (2013) 66(8):447-51. The particle size of the nebulized droplets can be adjusted by a number of parameters, including for example the formulation viscosity and surface tension, and the nebulizer characteristics, as is taught in the art.

### (c) Dry Powder formulations:

Dry powder formulations, as the name implies, do not have a liquid carrier. Instead, the active agent and excipients are ground or milled to a fine powder, having a particle size suitable for inhalation. The formulation is designed to be carried into the lungs by a sharp inhalation and/or a puff of compressed air or gas. Dry powder formulations are particularly convenient when administering agents that are difficult to dissolve or suspend in conventional liquid carriers.

Dry powder formulations often contain excipients in addition to the active agent or agents. These excipients are often included to improve the flow properties of the product, including the dispersion and absorption, as well as for chemical stability during storage. The formulations can be prepared, for example, by spray-drying (A.A. Ambike et al., Pharm Res (2005) 22(6):990-98), grinding or milling, extrusion, precipitation, and/or screening using methods known in the art to obtain an inhalable powder. The excipients used may also be mixtures of ground excipients which are obtained by mixing excipient fractions of different mean particle sizes.

Examples of physiologically acceptable excipients which may be used to prepare the inhalable powders for use in the inhalers (or cartridges thereof) include monosaccharides (e.g., glucose, fructose or arabinose), disaccharides (e.g., lactose, saccharose, maltose, trehalose), oligo- and polysaccharides (e.g., dextrans, dextrins, maltodextrin, starch, cellulose), polyalcohols (e.g., sorbitol, mannitol, xylitol), cyclodextrins (e.g., α-cyclodextrin, β-cyclodextrin, χ-cyclodextrin, methyl-β-cyclodextrin, hydroxypropyl-β-cyclodextrin, sulfobutyl-β-cyclodextrin (Captisol^{®}, Dexolve^{™})), amino acids (e.g., arginine hydrochloride), and salts (e.g., sodium chloride, calcium carbonate), or mixtures thereof. Lactose, glucose, and other compounds can be used in the form of their hydrates. The excipients can be combined with the statin before, during, or after the powdering process.

Within the scope of the inhalable powders, the excipients can have a maximum average particle size of up to about 250 µm, between 10 and 150 µm, or between 15 and 80 µm. Finer excipient fractions with an average particle size of 1 to 9 µm can also be added to the excipients mentioned above. The average particle size may be determined using methods known in the art (for example WO 02/30389). Finally, in order to prepare inhalable powders, a micronised crystalline statin, which can be characterized by an average particle size of about 0.5 to about 10 µm, or from about 1 to about 5 µm, is added to the excipient mixture (see, for example, WO 02/30389). Processes for grinding and micronizing active substances are known in the art. If no specifically prepared excipient mixture is used as the excipient, excipients which have a mean particle size of 10-50 µm and a 10% fine content of 0.5 to 6 µm can be used. In some embodiments, the maximum average particle size is less than about 250, 225, 200, 190, 180, 170, 160, 150, 140, 130, 125, 120, 115, 110, 105, 100, 95, 90, 85, 80, 75, 70, 65, 60, 55, 50, 45, 40, 35, 30, 25, 20, 19, 18, 17, 16, 15, 14, 13, 12, 11, 10, 9, 8, 7, 6, 5, 4, 3, 2, or 1 µm. In some embodiments, the average particle size is at least about 0.001, 0.005, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1.0, 2, 3, 4, 5, 6, 7, 8, 9, 10, 12, 15, 17, 19, 20, 25, 30, 35, 40, 45, or 50 µm. In some embodiments, the average particle size is less than about 250, 225, 200, 190, 180, 170, 160, 150, 140, 130, 125, 120, 115, 110, 105, 100, 95, 90, 85, 80, 75, 70, 65, 60, 55, 50, 45, 40, 35, 30, 25, 20, 19, 18, 17, 16, 15, 14, 13, 12, 11, 10, 9, 8, 7, 6, 5, 4, 3, 2, or 1 µm.

In one method for preparing a dry powder formulation, the excipient and the active agent are placed in a suitable mixing container. In some embodiments, the active agent has an average particle size of 0.5 to 10 µm, 1 to 6 µm, or 2 to 5 µm. The excipient and the active agent are added using a sieve or a granulating sieve with a mesh size of 0.1 to 2 mm, 0.3 to 1 mm, or 0.3 to 0.6 mm. The excipient may be added first, and then the active agent is added to the mixing container. During this mixing process the two components may be added in batches, and the two components sieved in alternate layers. The mixing of the excipient with the active agent may take place while the two components are still being added.

Inhalable powders can also be formulated as PulmoSpheres (see, e.g., J.G. Weers et al., Ther Deliv (2014) 5(3):277-95; J.G. Weers et al., AAPS PharSciTech (2019) 20(3):103; and US Pat. No. 9452139, in which suspensions of micronized drug particles are spray-dried to form a powder. Alternatively, powders and suspensions can be formulated from self-assembling nanoparticles (see for example, N.J. Kenyon et al., PLOS One (2013) doi. org/10.1371/ journal.pone.0077730).

### Inhalers

The three primary types of inhaler are the nebulizer, the pressurized metered-dose inhaler (pMDI), and the dry powder inhaler (DPI). Nebulizers convert a liquid solution or suspension of drug into a fine mist of droplets, which are then inhaled into the lungs. Nebulizers typically take longer to administer a drug than pMDIs or DPIs, and are less accurate in terms of the exact dose of drug that is absorbed, due to losses of drug in the device and to the surrounding air. However, they are typically the most easy to use, and can be used with subjects who are too young to operate pMDIs or DPIs, or who are unconscious. Nebulizers typically comprise a reservoir that contains the drug formulation, a nebulization chamber, a face mask, and a mechanism for nebulizing the formulation. In jet nebulizers, the mechanism comprises a nozzle through which air is passed at high velocity, which draws the liquid formulation up through a capillary tube. Droplets of the formulation are entrained in the air jet, and impacted against baffles which reduce the droplet size and/or screen out overly large droplets. The baffles also reduce the air speed, so that the resulting mist leaves the nebulizer at lower velocity and is more likely to reach the lower airways. The process of nebulization in these devices also usually reduces the temperature of the formulation, due to the evaporation of the droplets. Jet nebulizers typically require a compressor to generate the air flow, which makes them noisier and less portable than other inhalers.

Ultrasonic nebulizers employ an element that is vibrated at ultrasonic frequencies to break the liquid formulation into droplets. The vibrating element is often a stiff mesh or perforated membrane. These nebulizers are generally quieter than jet nebulizers, and do not require a compressor, although they do still require a power source. The ultrasonic vibration often raises the temperature of the formulation.

pMDIs contain a solution or suspension of drug in a propellant under pressure, and comprise a valve that delivers a precisely measured amount of the formulation when actuated. The propellant is often a gas such as a hydrofluoroalkane propellant, which is combined with the drug and optionally a co-solvent such as ethanol and/or a surfactant. The formulation is compressed into a liquid state, and loaded into the pMDI or a pMDI cartridge. A typical pMDI releases the formulation in liquid form into a metering chamber, which determines the amount of the dose. When the device is actuated, the measured formulation is released into an expansion chamber where the propellant is volatilized. For efficient and consistent delivery of the drug, the subject using the pMDI must coordinate his or her breathing with the device actuation, to insure that the greatest amount of aerosol possible reaches the lower airways. Modern pMDIs may further include valves or sensing mechanisms that release the aerosol only when the subject is inhaling. Most pMDIs also employ a spacer, which is essentially a tube between the pMDI and the subject, which improves the efficiency of aerosol delivery and permits more time for the propellant to evaporate (leading to smaller droplets).

DPIs, in general, contain a measured quantity of the drug as a dry powder, optionally having a dry powder carrier such as powdered lactose. DPIs rely on a sharp inhalation by the subject to dispense the powdered formulation, rather than forming a mist or aerosol. They are in general easier to use than pMDIs, although the efficiency of delivery depends in part on the airspeed that the subject is capable of producing. Newer DPIs that are breath-triggered but power assisted are in development.

Formulations of the disclosure can be administered using commercially available inhalation devices, such as nebulizers, for example without limitation, a Respimat^{®} Soft Mist^{™} inhaler; inhalers such as a RespiClick^{®} inhaler, Breezhaler^{®} inhaler, a Rotahaler^{®} inhaler, a Genuair^{®} inhaler, an Ellipta^{®} inhaler, a Staccato^{®} inhaler (Alexza Pharmaceuticals, Mountain View, CA), and the like. Inhalers can be provided pre-filled, containing one or multiple therapeutic doses of a formulation of the disclosure, or can be configured to accept a cartridge that is pre-filled with one or multiple therapeutic doses of a formulation of the disclosure.

Inhalable powders and aerosols may, for example, be administered using inhalers which meter a single dose from a reservoir by means of a measuring chamber (see, e.g., US 4,570,630) or by other means (see, e.g., DE 3625685). In some embodiments, the inhalable powders are packed into capsules or cartridges, which are used in inhalers such as those described in WO 94/28958.

Capsules and cartridges for use in an inhaler may be formulated containing a powder mix of the disclosed compounds or pharmaceutical compositions and a suitable powder base such as lactose or starch.

### Systems

Methods of the disclosure can also be practiced using systems of the disclosure, comprising a statin or a statin formulation, and one or more additional therapeutic agents or formulations comprising one or more additional therapeutic agents. In a system of the disclosure, the statin and the additional therapeutic agent(s) need not be present in the same formulation, and can be administered at different times. In some embodiments, the system comprises a statin selected from the group consisting of simvastatin, pitavastatin, rosuvastatin, atorvastatin, lovastatin, fluvastatin, mevastatin, cerivastatin, tenivastatin, and pravastatin, and isomers, enantiomers, and diastereomers thereof. In some embodiments, the statin is selected from the group consisting of simvastatin, pitavastatin, lovastatin, fluvastatin, mevastatin, cerivastatin, and tenivastatin. In some embodiments, the statin is a hydrophobic statin. In some embodiments, the statin is simvastatin or pitavastatin. In some embodiments, the statin is pitavastatin. In some embodiments, the statin is simvastatin.

In some embodiments, the formulation is a dry powder formulation. In some embodiments, the formulation is an aerosol formulation. In some embodiments, the formulation is a nebulizable formulation. In some embodiments, the nebulizable formulation comprises an aqueous solution of the statin. In some embodiments, the nebulizable formulation further comprises a pharmaceutically acceptable alcohol. In some embodiments, the pharmaceutically acceptable alcohol comprises ethanol.

In some embodiments, the system further comprises an additional therapeutic agent. The additional therapeutic agent may treat the same disease, disorder, or symptoms as a statin, or may treat different symptoms of the same disease or disorder. Combinations of one or more statins with one or more additional therapeutic agents in some cases exhibit additive effects, in which the degree of response due to the combination formulation is substantially the same as the sum of the degree of response from each agent when administered alone. Combinations can also produce sub-additive effects, in which the combination produces a degree of response that is less than the sum of the degree of responses from each agent when administered alone (but still greater than the response produced by either agent alone), or synergistic effects, in which the combination produces a degree of response that is greater than the sum of the degree of responses from each agent when administered alone. Thus, combinations of one or more statins and one or more additional therapeutic agents can be used to achieve a greater response while administering a given dose, to achieve the same response while administering a reduced dose, or any combination thereof.

If the degree of effect produced by the combination is greater than the degree desired or required, the dose of one or both agents can be reduced until the desired degree of effect is reached. The amount of dose reduction will not necessarily be the same amount or percentage for each agent. This can be used to reduce side effects, or minimize the probability of encountering side effects. Thus, the dose of one or more agents in a synergistic combination formulation may be reduced to a level that would be insufficient or sub-therapeutic when administered alone or as part of a non-synergistic combination formulation, but is sufficient for therapeutic use when administered as part of the synergistic combination formulation. The sub-therapeutic dose of an agent in a synergistic combination formulation can be about 90%, 80%, 75%, 70%, 65%, 60%, 55%, 50%, 45%, 40%, 35%, 30%, 25%, 20%, 19%, 18%, 17%, 16%, 15%, 14%, 13%, 12%, 11%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, or 0.1% of the effective dose of the agent when administered by inhalation as part of a non-synergistic formulation according to the present disclosure.

In some systems, the administration of an inhaled statin potentiates the effect of an additional therapeutic agent that is administered at a given time period later, and provides a greater therapeutic effect than either the statin or the additional therapeutic agent alone. In some systems, the administration of an inhaled statin potentiates an effect of an additional therapeutic agent that is other than the reduction of respiratory viral infection. In some systems, the additional therapeutic agent is administered later than the statin. In some embodiments, the time period is at least about 1, 2, 3, 4, 5, 6, 7, 8, 9, 10, 11, 12, 13, 14, 15, 16, 17, 18, 19, 20, 21, 22, 23, or 24 hours, or about 1, 2, or 3 days. In some embodiments, the time period is no more than about 72, 48, 36, 24, 23, 22, 21, 20, 19, 18, 17, 16, 15, 14, 13, 12, 11, 10, 9, 8, 7, or 6 hours.

The additional therapeutic agent may be any of the additional therapeutic agents described in the disclosure. In some embodiments, the additional therapeutic agent is chloroquine or a salt thereof, hydroxychloroquine or a salt thereof, amantadine, rimantadine, lopinavir, ritonavir, umifenovir, remdesivir, favipiravir, nelfinavir mesylate, azithromycin, bafilomycin, camostat or a salt thereof, darunavir, oseltamivir, or ribavirin. In some embodiments, the additional therapeutic agent includes two or more antiviral agents. In some embodiments, the additional antiviral agent is a bromodomain inhibitor (BETS), a Sigma 1 and/or Sigma 2 receptor targeting drug (for example without limitation, PB28), an antihistamine (for example without limitation, clemastine and/or cloperastin), a protein translation inhibitor (for example without limitation, zotatifin, ternatin-4, and/or plitidepsin), an antipsychotic drug (for example without limitation, haloperidol and/or cloperazine); or siramesine (an antidepressant and anti-anxiety drug). In some embodiments, the antiviral agent is PB28, clemastine, cloperastin, zotatifin, ternatin-4, plitidepsin, haloperidol, cloperazine, or siramesine.

Additional antiviral treatments that can be used with inhaled statin formulations and systems of the disclosure include convalescent plasma and/or antibodies derived therefrom; selinexor (a selective inhibitor of nuclear export (SINE) compound that blocks cellular protein XPO1; inhaled nitric oxide; exosome and microvesicle technology (allogenic cardiosphere-derived stem cells); and cord blood regulatory T cells.

ACE2 converts angiotensin II to angiotensin(1-7) (Ang₁₋₇), which has antiinflammatory, anti-oxidant, and anti-thrombotic effects. As reduction of these effects can be deleterious, some systems and treatments of the disclosure further include replacement or supplementation of this activity. This can be accomplished by administering Ang₁₋₇, soluble ACE2, and/or other enzymes that catalyze hydrolysis of angiotensin II to Ang₁₋₇. See, e.g., P. Verdecchia et al., Eur J Int Med (2020) doi. org/10.1016/j.ejim.2020.04.037 (in press). Alternatively or additionally, angiotensin II activity can be reduced, for example without limitation, by administering inhibitors of ACE (angiotensin converting enzyme), inhibitors of angiotensin II receptors (angiotensin II receptor blockers, or ARBs), or a combination thereof. Suitable ACE inhibitors include, without limitation, captopril, benazepril, zofenopril, perindopril, trandolapril, enalapril, lisinopril, and ramipril. Suitable ARBs block the activity of the angiotensin II type 1 receptor (AT1), and include, without limitation, losartan, valsartan, candesartan, telmisartan, and fimasartan.

In some embodiments, the additional therapeutic agent is beclomethasone, fluticasone, budesonide, mometasone, flunisolide, alclometasone, beclomethasone, betamethasone, clobetasol, clobetasone, clocortolone, desoximetasone, dexamethasone, diflorasone, diflucortolone, flurclorolone, flumetasone, fluocortin, fluocortolone, fluprednidene, fluticasone, fluticasone furoate, halometasone, meprednisone, mometasone, mometasone furoate, paramethasone, prednylidene, rimexolone, ulobetasol, amcinonide, ciclesonide, deflazacort, desonide, formocortal, fluclorolone acetonide, fludroxycortide, fluocinolone acetonide, fluocinonide, halcinonide, or triamcinolone acetonide, or a combination thereof. In some embodiments, the additional therapeutic agent is albuterol, arformoterol, buphenine, clenbuterol, bopexamine, epinephrine, fenoterol, formoterol, isoetarine, isoproterenol, orciprenaline, levoalbutamol, levalbuterol, pirbuterol, procaterol, ritodrine, albuterol, salmeterol, terbutaline, arbutamine, brefonalol, bromoacetylalprenololmenthane, broxaterol, cimaterol, cirazoline, etilefrine, hexoprenaline, higenamine, isoxsuprine, mabuterol, methoxyphenamine, oxyfedrine, ractopamine, reproterol, rimiterol, tretoquinol, tulobuterol, zilpaterol, or zintero, or a combination thereof. In some embodiments, the additional therapeutic agent is albuterol. In some embodiments, the additional therapeutic agent comprises both an antiviral agent and a corticosteroid.

In some embodiments, the additional therapeutic agent is ipratropium bromide, tiotropium, glycopyrrolate, glycopyrronium bromide, revefenacin, umeclidinium bromide, aclidinium, trospium chloride, oxitropium bromide, oxybutynin, tolterodine, solifenacin, fesoterodine, darifenacin, or a combination thereof. In some embodiments, the additional therapeutic agent is roflumilast, zileuton, nedocromil, squalene synthase inhibitors such as lapaquistat, zaragozic acid, and RPR 107393; inhibitors of farnesyl pyrophosphate synthase, including without limitation bisphosphonates such as alendronate, etidronate, clodronate, tiludronate, pamidronate, neridronate, olpadronate, ibadronate, risedronate, zoledronate; theophylline, an anti-IL5 antibody or antibody derivative, an anti-IgE antibody or antibody derivative, an anti-IL5 receptor antibody or antibody derivative, an anti-IL13/4 receptor antibody or antibody derivative, mepolizumab, reslizumab, benralizumab, omalizumab, dupilumab, or a combination thereof. In some embodiments, the additional therapeutic agent is TS-f22, AT13148, GSK429286A, RKI-1447, Y-27632, CCG-1423, GGTI-DU40, GGTI-297, AR9281, TPPU, AM-1172, JNJ 1661010, PF-3845, zafirlukast, montelukast, zileuton, or a combination thereof.

In some embodiments, the additional therapeutic agent is provided in a formulation comprising the additional therapeutic agent and a pharmaceutically acceptable carrier or vehicle. In some embodiments, the formulation is suitable for administration by inhalation. In some embodiments, the formulation is suitable for administration orally or by injection.

Additional antiviral treatments that can be used with inhaled statin formulations and systems of the disclosure include convalescent plasma or antibodies extracted therefrom; selinexor (a selective inhibitor of nuclear export (SINE) compound that blocks cellular protein XPO 1); inhaled nitric oxide; exosomes and/or microvesicles (for example, allogenic cardiosphere-derived stem cells); and cord blood regulatory T cells. In some embodiments, the system comprises a statin formulation of the disclosure, and convalescent plasma or antibodies extracted therefrom; selinexor; inhaled nitric oxide; exosomes and/or microvesicles; or cord blood regulatory T cells.

### IV. PHARMACEUTICAL COMPOSITIONS

In some embodiments, provided herein is a pharmaceutical composition comprising: a therapeutically effective amount of a statin; at least one additional therapeutic agent; and a pharmaceutically acceptable carrier.

In some embodiments, the statin is selected from the group consisting of simvastatin, pitavastatin, rosuvastatin, atorvastatin, lovastatin, fluvastatin, mevastatin, cerivastatin, tenivastatin, and pravastatin. In some embodiments, the statin is selected from the group consisting of simvastatin and pitavastatin. In some embodiments, the statin is simvastatin. In some embodiments the statin is pitavastatin. In some embodiments, the additional therapeutic agent is a β-agonist, a corticosteroid, a muscarinic antagonist, or any combination thereof. In some embodiments, the additional therapeutic agent is dexamethasone, amantadine, rimantadine, lopinavir, ritonavir, umifenovir, remdesivir, favipiravir, nelfinavir mesylate, azithromycin, bafilomycin, camostat or a salt thereof, darunavir, oseltamivir, ribavirin, convalescent plasma or antibodies extracted therefrom; selinexor; inhaled nitric oxide; exosomes and/or microvesicles; and cord blood regulatory T cells. In some embodiments, the statin is selected from the group consisting of pitavastatin and simvastatin; and the additional therapeutic agent is selected from the group consisting of remdesivir, dexamethasone, and a combination thereof.

In some embodiments, provided herein is a pharmaceutical formulation for the treatment of a viral respiratory disease, the composition comprising: a therapeutically effective amount of a statin, or an isomer, enantiomer, or diastereomer thereof, and a pharmaceutically acceptable carrier suitable for administration by inhalation. In some embodiments, provided herein is a pharmaceutical formulation for the treatment of a viral respiratory disease, the composition comprising: a therapeutically effective amount of a statin, or an isomer, enantiomer, or diastereomer thereof, and a pharmaceutically acceptable carrier suitable for administration by inhalation and/or intranasally.

In some embodiments, administration of the pharmaceutical formulation is by inhalation and/or intranasally. In some embodiments, the pharmaceutical formulation comprises statin and an additional therapeutic agent as described herein. In some embodiments, the additional therapeutic agent is remdesivir or dexamethasone. In some embodiments, the additional therapeutic agent is remdesivir. In some embodiments, the additional therapeutic agent is dexamethasone.

In some embodiments, the statin is administered by inhalation and/or intranasally, and the additional therapeutic agent is administered by inhalation and/or intranasally. In some embodiments, the statin is administered by inhalation and/or intranasally, and the additional therapeutic agent is remdesivir and administered by inhalation and/or intranasally. In some embodiments, the statin is administered by inhalation and/or intranasally, and the additional therapeutic agent is dexamethasone and administered by inhalation and/or intranasally. In some embodiments, the statin is administered by inhalation and/or intranasally, and the additional therapeutic agent is administered orally. In some embodiments, the statin is administered by inhalation and/or intranasally, and the additional therapeutic agent is remdesivir and administered orally. In some embodiments, the statin is administered by inhalation and/or intranasally, and the additional therapeutic agent is dexamethasone and administered orally.

The compositions of the present invention can be prepared in a wide variety of oral, parenteral and topical dosage forms. Oral preparations include tablets, pills, powder, dragees, capsules, liquids, lozenges, cachets, gels, syrups, slurries, suspensions, etc., suitable for ingestion by the patient. The compositions of the present invention can also be administered by injection, that is, intravenously, intramuscularly, intracutaneously, subcutaneously, intraduodenally, or intraperitoneally. Also, the compositions described herein can be administered by inhalation, for example, intranasally. Additionally, the compositions of the present invention can be administered transdermally. The compositions of this invention can also be administered by intraocular, intravaginal, and intrarectal routes including suppositories, insufflation, powders and aerosol formulations (for examples of steroid inhalants, see Rohatagi, J. Clin. Pharmacol. 35:1187-1193, 1995; Tjwa, Ann. Allergy Asthma Immunol. 75:107-111, 1995). Accordingly, the present invention also provides pharmaceutical compositions including a pharmaceutically acceptable carrier or excipient and the compound of the present invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances, which may also act as diluents, flavoring agents, binders, preservatives, tablet disintegrating agents, or an encapsulating material. Details on techniques for formulation and administration are well described in the scientific and patent literature, see, e.g., the latest edition of Remington's Pharmaceutical Sciences, Maack Publishing Co, Easton PA ("Remington's").

In powders, the carrier is a finely divided solid, which is in a mixture with the finely divided active component. In tablets, the active component is mixed with the carrier having the necessary binding properties in suitable proportions and compacted in the shape and size desired. The powders and tablets preferably contain from 5% or 10% to 70% of the compound the present invention.

Suitable solid excipients include, but are not limited to, magnesium carbonate; magnesium stearate; talc; pectin; dextrin; starch; tragacanth; a low melting wax; cocoa butter; carbohydrates; sugars including, but not limited to, lactose, sucrose, mannitol, or sorbitol, starch from corn, wheat, rice, potato, or other plants; cellulose such as methyl cellulose, hydroxypropylmethyl-cellulose, or sodium carboxymethylcellulose; and gums including arabic and tragacanth; as well as proteins including, but not limited to, gelatin and collagen. If desired, disintegrating or solubilizing agents may be added, such as the cross-linked polyvinyl pyrrolidone, agar, alginic acid, or a salt thereof, such as sodium alginate.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound (i.e., dosage). Pharmaceutical preparations of the invention can also be used orally using, for example, push-fit capsules made of gelatin, as well as soft, sealed capsules made of gelatin and a coating such as glycerol or sorbitol. Push-fit capsules can contain the compound of the present invention mixed with a filler or binders such as lactose or starches, lubricants such as talc or magnesium stearate, and, optionally, stabilizers. In soft capsules, the compound of the present invention may be dissolved or suspended in suitable liquids, such as fatty oils, liquid paraffin, or liquid polyethylene glycol with or without stabilizers.

For preparing suppositories, a low melting wax, such as a mixture of fatty acid glycerides or cocoa butter, is first melted and the compound of the present invention is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the compound of the present invention in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Also included are solid form preparations, which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

Oil suspensions can be formulated by suspending the compound of the present invention in a vegetable oil, such as arachis oil, olive oil, sesame oil or coconut oil, or in a mineral oil such as liquid paraffin; or a mixture of these. The oil suspensions can contain a thickening agent, such as beeswax, hard paraffin or cetyl alcohol. Sweetening agents can be added to provide a palatable oral preparation, such as glycerol, sorbitol or sucrose. These formulations can be preserved by the addition of an antioxidant such as ascorbic acid. As an example of an injectable oil vehicle, see Minto, J. Pharmacol. Exp. Ther. 281:93-102, 1997. The pharmaceutical formulations of the invention can also be in the form of oil-in-water emulsions. The oily phase can be a vegetable oil or a mineral oil, described above, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent.

The compositions of the present invention can also be delivered as microspheres for slow release in the body. For example, microspheres can be formulated for administration via intradermal injection of drug-containing microspheres, which slowly release subcutaneously (see Rao, J. Biomater Sci. Polym. Ed. 7:623-645, 1995; as biodegradable and injectable gel formulations (see, e.g., Gao Pharm. Res. 12:857-863, 1995); or, as microspheres for oral administration (see, e.g., Eyles, J. Pharm. Pharmacol. 49:669-674, 1997). Both transdermal and intradermal routes afford constant delivery for weeks or months.

In another embodiment, the compositions of the present invention can be formulated for parenteral administration, such as intravenous (IV) administration or administration into a body cavity or lumen of an organ. The formulations for administration will commonly comprise a solution of the compositions of the present invention dissolved in a pharmaceutically acceptable carrier. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables. These solutions are sterile and generally free of undesirable matter. These formulations may be sterilized by conventional, well known sterilization techniques. The formulations may contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of the compositions of the present invention in these formulations can vary widely, and will be selected primarily based on fluid volumes, viscosities, body weight, and the like, in accordance with the particular mode of administration selected and the patient's needs. For IV administration, the formulation can be a sterile injectable preparation, such as a sterile injectable aqueous or oleaginous suspension. This suspension can be formulated according to the known art using those suitable dispersing or wetting agents and suspending agents. The sterile injectable preparation can also be a sterile injectable solution or suspension in a nontoxic parenterally-acceptable diluent or solvent, such as a solution of 1,3-butanediol.

In another embodiment, the formulations of the compositions of the present invention can be delivered by the use of liposomes which fuse with the cellular membrane or are endocytosed, i.e., by employing ligands attached to the liposome, or attached directly to the oligonucleotide, that bind to surface membrane protein receptors of the cell resulting in endocytosis. By using liposomes, particularly where the liposome surface carries ligands specific for target cells, or are otherwise preferentially directed to a specific organ, one can focus the delivery of the compositions of the present invention into the target cells in vivo. (See, e.g., Al-Muhammed, J. Microencapsul. 13:293-306, 1996; Chonn, Curr. Opin. Biotechnol. 6:698-708, 1995; Ostro, Am. J. Hosp. Pharm. 46:1576-1587, 1989).

### V. METHODS OF TREATMENT

### (a) Administration by Inhalation or by intranasal administration

The methods of treatment of the disclosure are based on the administration of suitable statins by inhalation or by intranasal administration. The methods, formulations, and systems of the disclosure treat respiratory viral infections, thus providing therapies for diseases that are not effectively or completely treated with existing therapeutic agents. Administration by inhalation has the advantages of (a) direct contact with the respiratory airways, (b) avoidance of first-pass hepatic metabolism, and (c) avoidance of injection (J.L. Rau, Resp Care (2005) 50(3):367-82; M. Ibrahim et al., Med Dev Evidence Res (2015) 8:131-39). Because the drug is not subject to first-pass metabolism, and is administered locally to the lungs rather than systemically to the entire body, the doses for inhaled drugs are often smaller than the amount that would be administered orally.

As set forth herein, in some embodiments, formulations of the disclosure are administered with the aid of an inhalation device ("inhaler"), which can be a nebulizer, pMDI, DPI, or other device capable of conveying the formulation into the lower airways. In some embodiments, formulations of the disclosure are administered intranasally, for example using a spray applicator, nebulizer, or nose drops. The frequency of administration will depend on the clearance rate of the statin and/or additional therapeutic agent from the subject's lungs. In some embodiments, a statin formulation is administered no more than 8, 7, 6, 5, 4, 3, 2, or once per day, or no more than once every 2, 3, 4, 5, 6, or 7 days. In some embodiments, a statin formulation is administered at least once every 4, 3, or 2 days, or at least 1, 2, 3, 4, 5, or 6 times per day. The duration of treatment can be 1, 2, 3, 4, 5, 6, 7, 8, 9, 10, 11, 12, 13, 14, 15, 16, 17, 18, 19, or 20 days. In some embodiments, the treatment duration is 5 to 7 days. In some embodiments, the treatment duration is 1 to 10 days. In some embodiments, the treatment duration is 1 to 12 days. In some embodiments, the treatment duration is 1 to 14 days. In some embodiments, the formulation is administered to a subject while the subject is undergoing mechanical ventilation (e.g., a subject who has been intubated, and/or is receiving assistance with breathing). In some embodiments, a formulation of the disclosure is administered to a subject through a ventilator or respirator.

In the methods of the disclosure, the therapeutic composition is administered directly to the lungs (e.g. by inhalation or by intranasal delivery), and thus does not undergo first pass metabolism in the liver. As a result, the active agents in the formulation are not diluted across the subject's entire body, and are not metabolized by the liver, such that a smaller amount is required reach a therapeutic concentration in the subject's airways than would be required with conventional, oral administration. The therapeutically effective amount will depend on the condition to be treated, the severity of the infection, the general health and state of the subject, and the particular statin(s) (and/or isomer(s), enantiomer(s), and/or diastereomer(s)) selected. Thus, a therapeutically effective amount of a statin in the practice of the disclosure may be as low as about 0.005 µg, about 0.008 µg, about 0.01 µg, about 0.05 µg, about 0.08 µg, about 0.1 µg, about 0.5 µg, about 0.8 µg, about 1 µg, about 2 µg, about 3 µg, about 4 µg, about 5 µg, about 6 µg, about 7 µg, about 8 µg, about 9 µg, about 10 µg, about 11 µg, about 12 µg, about 14 µg, about 15 µg, about 16 µg, about 18 µg, or about 20 µg. A therapeutically effective amount of a statin in the practice of the disclosure may be as high as about 40 mg, 20 mg, 18 mg, 15 mg, 12 mg, 10 mg, 9 mg, 8 mg, 7 mg, 6 mg, 5 mg, 4 mg, 3 mg, 2 mg, or 1 mg.

In some embodiments, the therapeutically effective amount of the statin is at least about 0.005 µg/kg, about 0.008 µg/kg, about 0.01 µg/kg, about 0.05 µg/kg, about 0.08 µg/kg, about 0.1 µg/kg, about 0.5 µg/kg, about 0.8 µg/kg, about 1 µg/kg, about 2 µg/kg, about 3 µg/kg, about 4 µg/kg, about 5 µg/kg, about 6 µg/kg, about 7 µg/kg, about 8 µg/kg, about 9 µg/kg, about 10 µg/kg, about 11 µg/kg, about 12 µg/kg, about 14 µg/kg, about 15 µg/kg, about 16 µg/kg, about 18 µg/kg, or about 20 µg/kg. In some embodiments, the therapeutically effective amount of the statin is no higher than about 40 mg/kg, 20 mg/kg, 18 mg/kg, 15 mg/kg, 12 mg/kg, 10 mg/kg, 9 mg/kg, 8 mg/kg, 7 mg/kg, 6 mg/kg, 5 mg/kg, 4 mg/kg, 3 mg/kg, 2 mg/kg, or 1 mg/kg.

### (b) Inhibition of Viral Entry and Prophylaxis

As set forth herein, many viruses require a specific receptor in order to enter the host cell, and this receptor in many cases must be localized in a lipid raft in the host cell membrane in order to be functional. Statins are capable of depleting cholesterol from lipid rafts, resulting in the reduction of lipid rafts. Without being bound by any particular theory, the reduction in lipid rafts is believed to destabilize the receptors dependent on lipid rafts, and reduce or prevent viral entry into host cells, thereby reducing infectivity.

In some embodiments, the statins directly interact with the virus, such as coronavirus or SARS-CoV-2, and thereby reduce viral uptake into a cell. In some cases, the cell is an airway cell, a nasal cell, a cell of the mouth, or a lung cell, such as a lung epithelial cell. In some embodiments, administration of a statin prevents uptake of the virus, such as the SARS-CoV-2, entry into a cell and thereby inhibits, reduces or prevents viral infection. In some embodiments, administration of a statin prevents uptake of the virus into a cell and thereby reduces the overall amount (titer) of virus in a subject. In some cases, such administration reduces the severity of the infection and/or the resulting symptoms of the virus, such as reducing the severity or symptoms of COVID-19. In some embodiments, such administration reduces the transmissibility of the virus from an infected subject by reducing the level of the virus present in a subject or in a particular tissue (e.g., lung and/or airway epithelium) or orifice of a subject (such as the nose or mouth). In some embodiments, a statin is administered to a subject prophylactically, and such administration reduces, inhibits, blocks or prevents the virus infection, such as infection by SARS-CoV-2. In some embodiments, the statin is provided to a subject after a subject has tested positive for or become exposed to the virus, such as a SARS-CoV-2 virus, but before the subject has developed discernable symptoms of the infection.

The therapeutically effective amount for inhibition of viral entry, and thus reduction of respiratory viral infection, will depend on the identity of the virus and the host receptor(s) that is targeted by the virus, the severity of the condition, the general health and state of the subject, and the particular statin(s) (and/or isomer(s), enantiomer(s), and/or diastereomer(s)) selected. Treatments of the disclosure (formulations, methods, and systems of the disclosure) reduce viral entry and/or proliferation by at least 10, 20, 30, 40, 45, 50, 55, 60, 65, 70, 75, 80, 85, 90, 91, 92, 93, 94, 95, 96, 97, 98, 99, or about 100%. This efficacy can be measured using standard microbiological assays and tests. For example, a culture of relevant cells or tissue can be exposed to a virus, or to a sample suspected of containing a virus, in the presence or absence of a statin or statin formulation of the disclosure, incubated under physiologic conditions, and the amount of virus, viral nucleic acids, viral proteins, or a combination thereof is measured, quantified, or titered. The relevant cells or tissue can be cells, cell cultures, or tissue samples that are similar or identical to cells or tissues that are normally or expected to be infected by the virus under study. In the case of respiratory viral infections, relevant cells and tissues can be, for example without limitation, airway epithelial cells, lung slices, epithelial cell cultures, or other model cells or organisms. Alternatively, such tests can be conducted *in vivo,* using a model animal susceptible to the virus, or in humans, for example without limitation, in the context of a clinical trial.

A therapeutically effective amount of a statin in the practice of the disclosure may be as low as about 0.005 µg, about 0.008 µg, about 0.01 µg, about 0.05 µg, about 0.08 µg, about 0.1 µg, about 0.5 µg, about 0.8 µg, about 1 µg, about 2 µg, about 3 µg, about 4 µg, about 5 µg, about 6 µg, about 7 µg, about 8 µg, about 9 µg, about 10 µg, about 11 µg, about 12 µg, about 14 µg, about 15 µg, about 16 µg, about 18 µg, or about 20 µg. A therapeutically effective amount of a statin in the practice of the disclosure may be as high as about 40 mg, 20 mg, 18 mg, 15 mg, 12 mg, 10 mg, 9 mg, 8 mg, 7 mg, 6 mg, 5 mg, 4 mg, 3 mg, 2 mg, or 1 mg.

In some embodiments, the therapeutically effective amount of the statin is at least about 0.005 µg/kg, about 0.008 µg/kg, about 0.01 µg/kg, about 0.05 µg/kg, about 0.08 µg/kg, about 0.1 µg/kg, about 0.5 µg/kg, about 0.8 µg/kg, about 1 µg/kg, about 2 µg/kg, about 3 µg/kg, about 4 µg/kg, about 5 µg/kg, about 6 µg/kg, about 7 µg/kg, about 8 µg/kg, about 9 µg/kg, about 10 µg/kg, about 11 µg/kg, about 12 µg/kg, about 14 µg/kg, about 15 µg/kg, about 16 µg/kg, about 18 µg/kg, or about 20 µg/kg. In some embodiments, the therapeutically effective amount of the statin is no higher than about 40 mg/kg, 20 mg/kg, 18 mg/kg, 15 mg/kg, 12 mg/kg, 10 mg/kg, 9 mg/kg, 8 mg/kg, 7 mg/kg, 6 mg/kg, 5 mg/kg, 4 mg/kg, 3 mg/kg, 2 mg/kg, or 1 mg/kg.

In some conditions, the early treatment of infection can produce more effective results due to the exponential nature of viral replication. In some embodiments, a subject having a respiratory viral infection is treated (using a formulation, method, or system of the disclosure) as soon as possible following exposure to a virus, or to another subject having a respiratory viral infection. In some embodiments, the subject is treated within 48, 36, 24, 20, 19, 18, 17, 16, 15, 14, 13, 12, 11, 10, 9, 8, 7, 6, 5, 4, 3, 2, or 1 hours, or within less than one hour of exposure. In some cases, the time of exposure is not known, and the subject is treated as soon as possible after diagnosis of infection or the appearance of symptoms consistent with a respiratory viral infection, such as, for example, coughing, panting, shortness of breath, production of sputum, sneezing, fever, and others, depending on the identity of the virus. A diagnosis of infection can be made by using a nucleic acid detection method, for example using a PCR assay or CRISPR-based viral detection assay specific one or more viruses, by detection of anti-viral antibodies in biological samples obtained from the subject, by growth of the virus in cell culture, or by standard medical diagnostic practices. In some embodiments, the subject is treated within 48, 36, 24, 20, 19, 18, 17, 16, 15, 14, 13, 12, 11, 10, 9, 8, 7, 6, 5, 4, 3, 2, or 1 hours, or within less than one hour of diagnosis of infection or the appearance of symptoms.

In some cases, a subject may be at risk of exposure to a respiratory virus, and can be administered a treatment of the disclosure prior to or while experiencing that risk. For example, without limitation, healthcare workers, public health testing personnel, disease outbreak investigators and personnel, medical researchers, and others can be at risk of exposure to one or more respiratory viruses, and can be treated prior to exposure in order to prevent infection and/or to reduce the probability of infection. In some embodiments, the subject is treated within 48, 36, 24, 20, 19, 18, 17, 16, 15, 14, 13, 12, 11, 10, 9, 8, 7, 6, 5, 4, 3, 2, or 1 hours, or within less than one hour prior to experiencing a possible exposure to a respiratory virus. For example, hospital personnel can be treated prior to entering a hospital for work to limit or avoid respiratory viral infection. Teachers can be treated prior to the first day of school, to limit or avoid possible respiratory viral infections from students (and other teachers) returning to class.

In some embodiments, the respiratory virus is coronavirus or SARS-CoV-2. In some embodiments, the respiratory virus is SARS-CoV-2. In some embodiments, administration of the statins prevent uptake of SARS-CoV-2, entry of SARS-CoV-2 into a cell, and thereby inhibits, reduces, or prevents SARS-CoV-2 infection. In some embodiments, administration of the statins prevent uptake of SARS-CoV-2 into the cell, and thereby reduces the overall amount (titer) of SARS-CoV-2 in a subject. In some embodiments, such administration reduces the severity of the SARS-CoV-2 infection and/or the resulting symptoms. In some embodiments, such administration reduces the transmissibility of SARS-CoV-2 from an infected subject by reducing the level of the virus present in a subject or in a particular tissue (e.g., lung and/or airway epithelium) or orifice of a subject (such as the nose or mouth).

In some embodiments, the statins are administered prophylactically to subjects at risk of exposure to SARS-CoV-2 or after exposure to SARS-CoV-2. In some embodiments, the statins are administered prophylactically to subjects at risk of exposure to SARS-CoV-2. In some embodiments, the statins are administered after the exposure to SARS-CoV-2. In some embodiments, the statins are administered to the subject after the subject has tested positive for SARS-CoV-2. In some embodiments, the statins are administered after the subject has tested positive for SARS-COV-2, but before the subject has developed discernable symptoms of the infection.

In some embodiments, for a subject at risk for exposure or for whom exposure is suspected (due to contact with the virus or an infected individual), a statin formulation is administered no more than 8, 7, 6, 5, 4, 3, 2, or 1 time(s) per day, or no more than once every 2, 3, 4, 5, 6, or 7 days. In some embodiments, a statin formulation is administered at least once every 4, 3, or 2 days, or at least 1, 2, 3, 4, 5, or 6 times per day. The duration of treatment can be 1, 2, 3, 4, 5, 6, 7, 8, 9, 10, 11, 12, 13, 14, 15, 16, 17, 18, 19, or 20 days. In some embodiments, the treatment duration is 5 to 7 days. In some embodiments, the treatment duration is 1 to 10 days. In some embodiments, the treatment duration is 1 to 12 days. In some embodiments, the treatment duration is 1 to 14 days.

In pandemic situations, where a large number of subjects are at risk of infection, any subject can be treated: in such cases, the risk event can be considered the formal declaration of a pandemic, the declaration of an epidemic in the geographic area in which the subject lives, works, or has recently visited; the beginning of travel to a geographic area that is experiencing an epidemic, pandemic, or outbreak. In some cases, subjects are treated during a pandemic if they have an elevated risk of severe complications, for example without limitation, viral pneumonia, pulmonary emboli, respiratory failure, ARDS, sepsis, acute lung injury (ALI), or death. Such patients are sometimes at elevated risk due to existing comorbidities such as diabetes, obesity, heart disease, lung disease, liver disease, kidney disease, immunocompromised states, cancer, or other conditions that reduce a subject's ability to resist disease. In some embodiments, a formulation or system of the disclosure is administered to a subject having a respiratory viral infection, who concurrently has diabetes, obesity, heart disease, lung disease, liver disease, kidney disease, immunocompromise (including immune suppression by a virus and/or drugs such as chemotherapy), or cancer.

The reduction of respiratory viral infection can also reduce the progression from mild forms of infection (for example, a minor nose, mouth, and/or throat infection) to a more severe form of infection (for example, bronchitis, pneumonia, pulmonary emboli, respiratory failure, ARDS, sepsis, ALI, myocarditis, or death). The reduction of respiratory viral infection can also reduce the degree of invasive medical treatment required, such as intensive care unit (ICU) admission, intubation, mechanical ventilation, and/or extracorporeal membrane oxygenation (ECMO).

In some embodiments, the administration of an inhaled or intranasally administered statin, is administered in an early stage of infection, where the virus may be present in the nose or nose and throat, but has not substantially entered the lower airways and lungs.

In some embodiments, statins are administered to a subject to protect from viral-induced epithelial cell death. Such administration of statins preserve epithelial cell viability in the face of a viral infection, and thereby may reduce the severity of the infection and symptoms resulting therefrom. In some embodiments, administration of a statin protects from SARS-CoV-2-induced epithelial cell death.

In some embodiments, the administration of a statin, such as an inhaled or intranasal statin reduces an immune response that may cause a severe reaction to a viral infection, such as an infection by a Corona virus such as SARS-CoV-2. In some embodiments, the administration of the statin reduces the level of IL-6 in an infected subject, such as a subject infected with SARS-CoV-2. In some embodiments, the administration of the statin reduces, inhibits or prevents the cytokine storm in the infected subject with resultant severe systemic inflammation.

In some embodiments, the administration of a statin, such as an inhaled or intranasal statin reduces or protects against complications and/or damage caused by a respiratory virus such as SARS-CoV-2. Such complications or damage can include damage to the lungs and other tissues. In some embodiments, the administration of a statin, such as an inhaled or intranasal statin reduces or protects against acute respiratory distress syndrome (ARDS) and pulmonary scarring or fibrosis that can be associated with infection by a respiratory virus such as SARS-CoV-2. In some embodiments, the administration of a statin, such as an inhaled or intranasal statin reduces or protects against COVID-19 associated ARDS-induced thrombosis (blood clotting). In some embodiments, the administration of a statin, such as an inhaled or intranasal statin reduces or protects against "long haul" symptoms, such as long haul COVID-19 (also known as "long COVID-19"), which can include one or more of fatigue, cough, joint pain, shortness of breath, chest pain, muscle pain, headaches, cognitive difficulties, fever and depression.

The reduction in viral entry can also be affected outside the respiratory system. For example, in some cases viruses target tissues other than, or in addition to, the respiratory system. For example, without limitation, SARS-CoV-2 has been found to also target ACE2-bearing cells in the heart, vasculature, and gut, and can cause myocarditis. In such cases, viral entry can be reduced by administering by inhalation formulations or systems of the disclosure that are targeted to the systemic circulation. In some embodiments, a viral infection is reduced by administering by inhalation a systemic circulation-targeted formulation or system of the disclosure. In some embodiments, the viral infection also infects the respiratory system. In some embodiments, the viral infection does not also infect the respiratory system.

In some embodiments, provided herein is a method for reducing viral respiratory infection in a subject in need thereof, the method comprising: administering a formulation intranasally or by inhalation to the subject having a viral respiratory infection, wherein the formulation comprises a therapeutically effective amount of a statin; and a pharmaceutically acceptable carrier.

In some embodiments, provided herein is a method for treating a viral respiratory infection in a subject in need thereof, the method comprising: administering a formulation intranasally or by inhalation to the subject suffering from the viral respiratory infection or who may be exposed to the viral respiratory infection, wherein the formulation comprises a therapeutically effective amount of a statin; and a pharmaceutically acceptable carrier.

In some embodiments, the viral respiratory infection is coronavirus, SARS-CoV-2 or COVID-19.

In some embodiments, the formulation of the methods of the present invention is administered by any known method known by one of skill in the art. In some embodiments, the formulation is administered as described in the sections above. In some embodiments, the formulation is administered intranasally or by inhalation. In some embodiments, the formulation is administered intranasally. In some embodiments, the formulation is administered by inhalation. In some embodiments, the administration is by a mechanical inhaler. In some embodiments, the mechanical inhaler is a metered-dose powder inhaler, a pressurized aerosol inhaler, a dry powder inhaler, or a nebulizer. In some embodiments, the mechanical inhaler is selected from the group consisting of: Respimat^{®} Soft Mist^{™} inhaler, RespiClick^{®} inhaler, Breezhaler^{®} inhaler, Genuair^{®} inhaler, Rotahaler^{®} inhaler, Staccato^{®} inhaler, and Ellipta^{®} inhaler.

In some embodiments, the methods of the present invention comprises any statins known by one of skill in the art. In some embodiments, the statin is selected from the group consisting of simvastatin, pitavastatin, rosuvastatin, atorvastatin, lovastatin, fluvastatin, mevastatin, cerivastatin, tenivastatin, and pravastatin. In some embodiments, the statin is selected from the group consisting of simvastatin, pitavastatin, rosuvastatin, and atorvastatin. In some embodiments, the statin is selected from the group consisting of pitavastatin and simvastatin. In some embodiments, the statin is pitavastatin. In some embodiments, the statin is simvastatin.

In some embodiments, the methods of the present invention comprises administering the statin in any therapeutically effective amount known by one of skill in the art. The statin can be administered in any therapeutically effective amount as described in the sections above. In some embodiments, the therapeutically effective amount is between about 0.005 µg and about 40 mg. In some embodiments, the therapeutically effective amount is between about 0.1 µg and about 15 mg. In some embodiments, the therapeutically effective amount is between about 0.1 µg and about 5 mg. In some embodiments, the therapeutically effective amount is between about 0.5 µg and about 15 mg. In some embodiments, the therapeutically effective amount is between about 1.0 µg and about 10 mg. In some embodiments, the therapeutically effective amount is between about 1.0 µg and about 5 mg. In some embodiments, the therapeutically effective amount is between about 0.1 µg and about 100 µg.

In some embodiments, the methods of the present invention further comprises administering at least one additional therapeutic agent. In some embodiments, the method of the present invention comprises any additional therapeutic agent known by one of skill in the art. In some embodiments, each additional therapeutic agent is independently selected from the group consisting of RNA polymerase inhibitors; inhibitors of a viral protease; inhibitors of a host protease; TMPRSS2 inhibitors; antiviral agents; chloroquine or a salt thereof, hydroxychloroquine or a salt thereof, amantadine, rimantadine, lopinavir, ritonavir, umifenovir, remdesivir, favipiravir, nelfinavir mesylate, azithromycin, bafilomycin, camostat or a salt thereof, darunavir, oseltamivir, ribavirin, convalescent plasma or antibodies extracted therefrom; selinexor; inhaled nitric oxide; exosomes and/or microvesicles; and cord blood regulatory T cells. In some embodiments, each additional therapeutic agent is nelfinavir mesylate, azithromycin, bafilomycin, camostat mesylate, camostat or a camostat salt, arbidol, amantadine, rimantadine, lopinavir, darunavir, ribavirin, remdesivir, favipirvir, chloroquine, hydroxychloroquine, tocilizumab, or sarilumab. In some embodiments, the additional therapeutic agent is remdesivir.

In some embodiments, each additional therapeutic agent is selected from the group consisting of β-agonists; corticosteroids; muscarinic antagonists; RhoA inhibitors; GGTase-I or -II inhibitors; ROCK1 and/or ROCK2 inhibitors; soluble epoxide hydrolase inhibitors; fatty acid amide hydrolase inhibitors; leukotriene receptor antagonists; phosphodiesterase-4 inhibitors such as roflumilast; 5-lipoxygenase inhibitors such as zileuton; mast cell stabilizers such as nedocromil; squalene synthase inhibitors such as lapaquistat, zaragozic acid, and RPR 107393; inhibitors of farnesyl pyrophosphate synthase, including without limitation bisphosphonates such as alendronate, etidronate, clodronate, tiludronate, pamidronate, neridronate, olpadronate, ibadronate, risedronate, zoledronate; theophylline; anti-IL5 antibodies; anti-IgE antibodies; anti-IL5 receptor antibodies; anti-IL13/4 receptor antibodies; biologics such as mepolizumab, reslizumab, benralizumab, omalizumab, and dupilumab; β-agonist and muscarinic antagonist combinations, including both long- and short-acting formulations; β-agonist and corticosteroid combinations, including both long- and short-acting formulations; corticosteroids and muscarinic antagonist combinations, including both long- and short-acting formulations; and β-agonist, corticosteroid, and muscarinic antagonist combinations, including both long- and short-acting formulations. In some embodiments, each additional therapeutic agent is a β-agonist, a corticosteroid, a muscarinic antagonist, or any combination thereof. In some embodiments, the additional agent is dexamethasone. In some embodiments, the additional agent comprises dexamethasone and further comprises remdesivir.

The additional therapeutic agents of the present invention can be administered by any method and any dosage known by one of skill in the art. In some embodiments, the additional therapeutic agent is administered intranasally or by inhalation. In some embodiments, the additional therapeutic agent is administered intranasally. In some embodiments, the additional therapeutic agent is administered by inhalation. In some embodiments, the additional therapeutic agent is administered at a therapeutic dose or at a sub-therapeutic dose. In some embodiments, the additional therapeutic agent is administered at a therapeutic dose. In some embodiments, the additional therapeutic agent is administered at a sub-therapeutic dose.

In some embodiments, the formulation for the method of the present invention can be administered at any suitable time. In some embodiments, the formulation is administered prophylactically. In some embodiments, the formulation is administered prior to exposure to the viral respiratory infection. In some embodiments, the formulation is administered between 1 hour and 7 days before exposure to the viral respiratory infection. In some embodiments, the formulation is administered between 1 hour and 24 hours before exposure to the viral respiratory infection. In some embodiments, the formulation is administered between 3 hours and 12 hours before exposure to the viral respiratory infection. In some embodiments, the formulation is administered about 4 hours, 6 hours, 8 hours, or 10 hours before exposure to the viral respiratory infection. In some embodiments, the formulation is administered about 6 hours before exposure to the viral respiratory infection.

In some embodiments, the formulation is administered after exposure to the viral infection. In some embodiments, the formulation is administered after the subject is diagnosed with the infection. In some embodiments, the formulation is administered after a suspected exposure to the respiratory virus.

In some embodiments, the formulation is administered from 1 hour to 24 hours before the potential exposure to the viral respiratory infection, wherein the statin comprises pitavastatin or simvastatin, and wherein the formulation further comprises at least one of remdesivir or dexamethasone.

In some embodiments, provided herein is a method for treating a SARS-CoV-2 virus infection in a subject in need thereof, the method comprising: administering a formulation intranasally or by inhalation to a subject suffering from the viral respiratory infection, wherein the formulation comprises a therapeutically effective amount of a statin; and a pharmaceutically acceptable carrier.

In some embodiments, the provided herein is a method for treating a SARS-CoV-2 virus infection in a subject in need thereof, the method comprising: administering a formulation intranasally or by inhalation to a subject who may be exposed to a SARS-CoV-2 virus, wherein the formulation comprises a therapeutically effective amount of a statin; and a pharmaceutically acceptable carrier.

In some embodiments, provided herein is a method for reducing the severity of COVID-19 in a subject infected with SARS-CoV-2, the method comprising: administering a formulation intranasally or by inhalation to the infected subject, wherein the formulation comprises a therapeutically effective amount of a statin; and a pharmaceutically acceptable carrier.

In some embodiments, the formulations for the methods of the present invention can reduce or inhibit virus titer, viral load, symptoms of the virus infection, or pro-inflammatory responses. In some embodiments, the formulation inhibits an increase in virus titer. In some embodiments, the formulation reduces viral load in the subject. In some embodiments, the formulation reduces or inhibits one or more symptoms of the virus infection. In some embodiments, the formulation reduces or inhibits one or more pro-inflammatory responses.

In some embodiments, the pro-inflammatory response is a cytokine, chemokine, or increase in IL-6 level. In some embodiments, the pro-inflammatory response is a cytokine or a chemokine. In some embodiments, the formulation reduces or inhibits an increase in IL-6 level in the subject. In some embodiments, the formulation prevents, inhibits or reduces a cytokine storm in the subject.

In some embodiments, the formulation is administered after a suspected exposure to the respiratory virus. In some embodiments, the formulation is administered subsequent to the exposure to the SARS-CoV-2 virus. In some embodiments, the formulation is administered to the subject within 1 hour, 2 hours, 6 hours, or 24 hours after the suspected exposure. In some embodiments, the formulation is administered to the subject within 1-14 days after the suspected exposure. In some embodiments, the formulation is administered to the subject within 1 day, 2 days, 3 days, 4 days, 5 days, 6 days or 7 days after the suspected exposure. In some embodiments, the formulation is administered to the subject within 7-10 days after the suspected exposure.

In some embodiments, the formulation is administered prior to a potential exposure to the respiratory virus. In some embodiments, the formulation is administered prior to a potential exposure to the SARS-CoV-2 virus. In some embodiments, the formulation is administered to the subject within 1 hour, 2 hours, 6 hours, or 24 hours before a potential exposure.

The formulation of the methods of the present invention can be administered as described in the sections above. In some embodiments, the formulation is administered once, twice, three times, 4 times or 5 times to the subject. In some embodiments, the formulation is administered once, twice, three times, 4 times or 5 times after the subject is exposed to the SARS-CoV-2 virus. In some embodiments, the formulation is administered once, twice, three times, 4 times or 5 times prior to exposing the subject to the SARS-CoV-2 virus.

In some embodiments, the formulation is administered to the subject prior and/or after vaccination for the SARS-CoV-2 virus. In some embodiments, the formulation is administered to the subject prior to a vaccination for the SARS-CoV-2 virus. In some embodiments, the formulation is administered to the subject after a vaccination for the SARS-CoV-2 virus. In some embodiments, the formulation is administered to the subject in combination with a vaccine for the SARS-CoV-2 virus.

In some embodiments, the formulation is administered to the subject in combination with an additional COVID-19 treatment. The additional COVID-19 treatment can be any treatment known by one of skill in the art. In some embodiments, the additional COVID-19 treatment is remdesivir or dexamethasone.

The statins of the present invention can preserve cell viability. In some embodiments, the statin preserves epithelial cell viability in the subject. In some embodiments, the epithelial cell viability is preserved in lung tissue and/or throat tissue. In some embodiments, the epithelial cell viability is preserved in lung tissue.

In some embodiments, provided herein is a method for blocking viral entry into a cell comprising administering a therapeutically effective amount of a statin, and wherein the virus is a SARS virus.

The SARS virus can be any SARS virus known by one of skill in the art. In some embodiments, the virus is a SARS-CoV-2 virus.

The cell of the methods of the present invention can be any suitable cell known by one of skill in the art. In some embodiments, the cell is an airway epithelial cell. In some embodiments, the cell is an airway epithelial cell of the mouth, nose, trachea or lung.

In some embodiments, the epithelial cell is present in a SARS-CoV-2 -infected subject and the statin is administered as a formulation intranasally or by inhalation to the infected subject, wherein the formulation comprises a therapeutically effective amount of a statin and a pharmaceutically acceptable carrier.

### VI. EXAMPLES

The following examples are provided for guidance, and are not intended to limit the scope of the claims herein.

To enhance cellular uptake and predictable cellular relaxation property simvastatin was activated by alkaline hydrolysis to chemically convert simvastatin lactone to simvastatin acid (SA). *In vivo,* hydrolysis can also occur naturally inside cells via lactonases, paraoxonases, alkaline hydrolases, and carboxylesterases. Simvastatin is activated by opening its lactone ring to the hydroxyl acid, using the protocol provided by Merck. Briefly, 8 mg of simvastatin (0.019 mM) is dissolved in 0.2 mL of 100% ethanol, with subsequent addition of 0.3 mL of 0.1 N NaOH. The solution is then heated at 50°C for 2 hours in a sand bath, then neutralized with HCl to a pH of 7.2 (C.C. Ghosh et al., Crit Care Med (2015) 43(7):e230-40).

### EXAMPLE 1: DEPLETION OF CHOLESTEROL

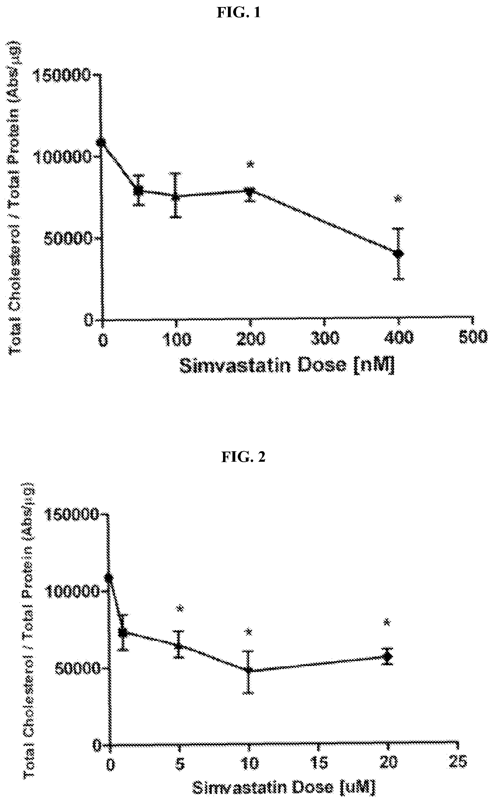

Normal human bronchial epithelial cells (cell line HBE1) were grown to confluence in an air-liquid interface (ALI), and were treated with simvastatin at 50, 100, 200, or 400 nM, and 1, 5, 10, or 20 µM for 48 hours. Total cellular cholesterol was measured by spectrophotometry, and plotted as the ratio of absorbance to total protein in µg. FIG. 1 shows the results of treatment with simvastatin at 50, 100, 200, and 400 nM. FIG. 2 shows the results of treatment with simvastatin at 1, 5, 10, and 20 µM. Significant reductions in cholesterol content (≥ 50%, p < 0.05) are indicated (*).

### EXAMPLE 2: ANTIVIRAL ACTIVITY

Airway epithelial cells are grown to confluence in biphasic ALI conditions, and treated with simvastatin, pitavastatin, rosuvastatin, atorvastatin, lovastatin, fluvastatin, mevastatin, cerivastatin, tenivastatin, and pravastatin for 24 to 72 hours and concentrations of 1 to 5 µM. Viral replication is measured in plaque assays, and viral levels are determined using quantitative RT-PCR of viral RNA.

Cell death is also determined by MTT assay, LDH release, and Alamar blue (C. Österlund et al., J Appl Toxicol (2005) 25:328-37; R. Hamid et al., Toxicol Vitr (2004) 18(5):703-10; and J. O'Brien et al., Eur J Biochem (2000) 267(17):5421-26). Expression of pro-inflammatory genes (IFNγ, IFNβ, TNFα, IL6, IL8, IL1β).

Pre- and post-drug experiments are conducted to demonstrate drug efficacy before infection and after SARS-CoV-2 infection in Calu-3 human epithelial cell line and primary human bronchial epithelial cells or precision cut human lung slices (PCLS). The expression of ACE2 and TMPRSS2 is measured using qRT-PCR and ELISA, and infectivity is determined via plaque assay. Cell death, viral levels, and inflammatory gene expression are determined as described above.

### EXAMPLE 3: FORMULATION

A spongy microsphere formulation of pitavastatin is prepared as follows. Distearoylphosphatidylcholine (1.24 g) is dispersed in 50 mL deionized water with a model T-25 Ultra-Turrax mixer at 8,000 rpm for 2 to 5 minutes (T = 60-70°C). The oil-in-water emulsion is mixed at 10,000 rpm for an additional period of not less than 4 minutes with a model T-25 Ultra-Turrax mixer (T = 60-70°C). The resulting coarse emulsions are then homogenized under high pressure with an Avestin (Ottawa, Canada) homogenizer at 18,000 psi for 5 passes.

To Pluronics^{®} F68 (BASF) (2.0 g) is added 500 mL deionized water, and the composition is mixed with a model T-25 Ultra-Turrax mixer at 10,000 rpm for 60 min (T = 60-70°C). Pitavastatin (0.4 g) is added to the Pluronics^{®} mixture in 10 mg increments spaced 5 min apart, maintaining the temperature at 60-70°C to form the drug feedstock.

The drug feedstock is then added dropwise to the O/W emulsion with continuous magnetic stirring, maintaining the temperature of the feedstock at 60°C. The combined feedstock can be UV or gamma sterilized prior to nebulization, in the event that filter clogging prevents sterile filtration. The final product is packaged for administration by nebulizer, or is spray dried for administration as a dry powder.

### EXAMPLE 4: IN SILICO 2BIND ASSAY

| **Instatin Compound** | **Chemical Name** | **Solvent** | **Concentration** |
|---|---|---|---|
| INS101 | Pravastatin Sodium | DMSO | 25mM |
| INS102 | Simvastatin | DMSO | 25mM |
| INS103 | Pitavastatin Calcium | DMSO | 25mM |
| INS104 | Atorvastatin | DMSO | 25mM |
| INS105 | Lovastatin | DMSO | 25mM |
| INS106 | Simvastatin Hydroxy Acid, Ammonium Salt | DMSO | 25mM |
| INS107 | ENALAPRILAT | DMSO | 25mM |
| INS102-S | Hydroxy Simvastatin | hydroalcoholic | 25mM |

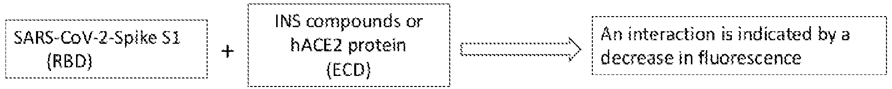

**Protein labelling and Interaction test:** SARS-CoV-2 S1 was labeled using Monolith His-Tag Labeling Kit RED-tris-NTA 2nd Generation (NanoTemper Technologies) according to the manufacturer's instructions in 1 C PBS pH 7.4, 0.005% Tween. To ensure stable interaction of His-tag and Dye was tested in a full dose response MST binding assay was performed. Constant 5 nM RED-tris-NTA Dye was supplied with a range of concentrations of SARS-CoV-2 S1 (0,0305 - 1000 nM) at 40% MST power, 10% LED power in premium capillaries on a Monolith NT.115 pico device at 25 °C (NanoTemper Technologies, Munich, Germany). Binding buffer was PBS pH 7.4, 0.005% Tween.

**MicroScale Thermophoresis (MST) binding assay SARS-CoV-2 S1 (labelled) vs ACE2.** MicroScale Thermophoresis (MST) binding experiments were carried out with 25 nM RED-tris-NTA -labeled SARS-CoV-2 S1 in binding buffer (PBS pH 7.4, 0.005% Tween) with a range of concentrations of ACE2 (0,2332 - 7.640 nM) at 40% MST power, 10% LED power in premium capillaries on a Monolith NT.115 pico device at 25°C (NanoTemper Technologies, Munich, Germany). Data was analyzed using MO.Affinity Analysis software (version v2.3, NanoTemper Technologies) at the standard MST-on time of 15s Data fits possessing amplitudes >5 units combined with Signal to Noise levels >5 units were defined as binding events.

**MicroScale Thermophoresis (MST) binding assay SARS-CoV-2 S1 (labelled) vs ligands.** MicroScale Thermophoresis (MST) binding experiments were carried out with 25 nM RED-tris-NTA -labeled SARS-CoV-2 S1 in binding buffer (PBS pH 7.4, 0.005% Tween + 2% DMSO) with a range of concentrations of each ligand (6.1035- 200.000 nM) at 40% MST power, 10% LED power in premium capillaries on a Monolith NT.115 pico device at 25°C (NanoTemper Technologies, Munich, Germany). Data was analyzed using MO.Affinity Analysis software (version v2.3, NanoTemper Technologies) at the different suitable MST-on times (1-20sec) for each data set. Data fits possessing amplitudes >5 units combined with Signal to Noise levels >5 units were defined as binding events.

### Comprehensive MST binding assays:

Target: SARS-CoV-2 S1, used at constant 25 nM
Ligand: INS101-INS107 titrated from 200 µM down in 16 1:1 dilution steps
Instrument : Monolith NT.115 Pico
Buffer: 1x PBS pH 7.4, 0.2% CHAPS, 2% DMSO
Replicates: technical

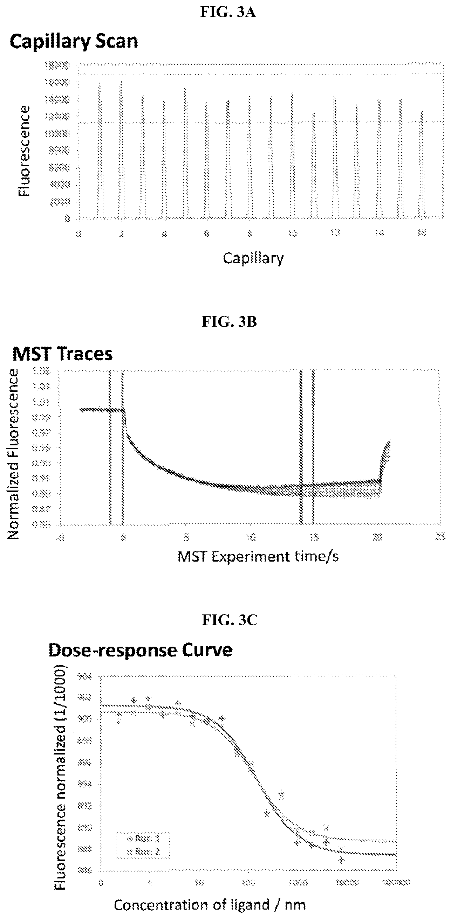

Data from the ACE2 Assay are shown in **FIG. 3****.**

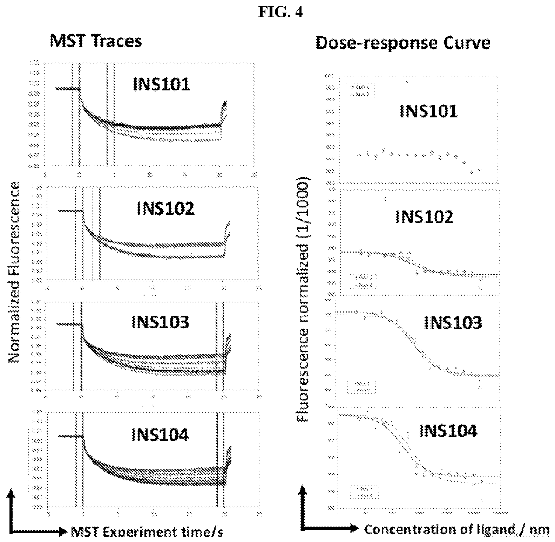

Data from the statin ligands assay are shown in **FIG. 4****.**

Data on ligand affinity and signal to noise (S/N) ratio are shown in **Table 1** below.

**Table 1. Ligand Affinity and Signal to Noise (S/N) Ratio**

| Ligand | Affinity (nM) | S/N | Remarks |
|---|---|---|---|
| hACE2 | 126-144 | 14.7 | Strong binding |
| INS-101 | NA | NA | No interaction/ weak binder |
| INS-102 | 468-951 | 5.3-6.2 | Good binding |
| INS-103 | 678-1005 | 14.4-17.3 | Good binding |
| INS-104 | 234-456 | 8.5-8.7 | Strong Binding |

Overall results of the affinity and docking score of the ACE2 receptor and statin ligands are shown in **Table 2** below.

**Table 2. Ligand Affinity and Docking Score**

| Compound | Affinity (nM) of binding to SARS-COV2 Spike protein | Docking Score |
|---|---|---|
| hACE2 - Human ACE2 receptor | 126-144 | |
| INS101 - Pravastatin Na | n.d. | - |
| INS102 - Simvastatin | 468-951 | -12.6 |
| INS102S - Hydroxy Simvastatin | n.d. | - |
| INS103 - Pitavastatin Ca | 678-1005 | -13.82 |
| INS104 - Atorvastatin | 234-456 | -24.16 |
| INS105 - Lovastatin | 529-929 | -13.6 |
| INS106 - Simvastatin Hydroxy acid | 1570-2600 | -13.58 |
| INS107 - Enalaprilat | n.d. | - |
| INS108 - MLN4760 | Not Tested | - |
| INS109 - Rosuvastatin Ca | Not Tested | -19.29 |

It is noted that INS-101 and INS-102S have weak binding indicated. For INS-107, no binding is indicated, although there may be very weak binding indicated. The order of affinity based on the binding assay is: hACE2>INS-104>INS-102>INS-105>INS-103>INS-106.

The docking score was generated by in silico modeling of the statin and the S-protein target. The more negative the docking score, the greater the binding affinity. The order of affinity based on the docking score therefore is: hACE2>INS-104>INS-109>INS-103>INS-105>INS-106>INS-102.

In conclusion, INS-102, INS-103, INS-104, the hydrophobic statins, bind with SARS-COV-2 S-protein at nanomolar (nM) concentrations. INS-101, which is hydrophilic, has none or very low affinity for the S-protein. INS-102, INS-103, and INS-104 may hinder the interactions between SARS-COV-2 and hACE2.

### EXAMPLE 5: ONCODESIGN STUDIES

The study evaluates two compounds on SARS-CoV-2 induced cytokine profile in human lung epithelial cell model during replication phase with SARS-CoV-2 virus of 2 compounds at 3 concentrations on human lung cell line, Calu-3. At the end of the experiment, supernatants were collected to assay IL6 by Elisa and 10-plex panel by multiplex technology. Last viral loads were also evaluated by RTqPCR technology.

**Test substance.** The two test substance compounds INS102 and INS103 were provided by the sponsor and stored at - 20°C until use. INS102 and INS103 were provided at 25mM in DMSO.

**Cell line.** The cell line used in this study is detailed below:

| **Cell line** | **Type** | **Species** | **Origin** |
|---|---|---|---|
| Calu-3 | Lung adenocarcinoma | Human | Elabscience |

**Cell culture conditions.** Calu-3 cell model is well described in the literature already for SARS-CoV (e.g. Tseng et al., 2005, J Virol, https://doi:10.1128/JVI.79.15.9470-9479). Calu-3 cells were grown as monolayer at 37°C in a humidified atmosphere (5% CO₂, 95% air) into the corresponding cell culture medium (MEM + 1% Pyruvate + 1% glutamine + 10% Fetal Bovine Serum). All the cells were adherent to plastic flasks. For cell passaging procedures, cells were detached from the culture flask by a 5-min treatment with trypsin-versene and neutralized by addition of complete culture medium. For the study, cells were coated on 96-well plates. The cells were counted and their viability is assessed by using V-cell counter.

**Virus isolate.** The virus strain was supplied through the European Virus Archive goes Global (Evag) platform (https://www.european-virus-archive.com/). For this study, Slovakia isolate was used (reference SARS-CoV-2 strain Slovakia/SK-BMC5/2020). Viral titer: SARS-CoV-2 was amplified and titered on Vero E6 TMPRSS2 cell line (origin NIBsc, UK) by Oncodesign.

The experimental goal is to evaluate anti-viral effects during replication phase on human lung epithelial cell line. All experiment are repeated once independently (N=2).

Protocol of infection and treatment: Calu-3 cells were counted and their viability assessed using Vi-Cell automatic apparatus. About two hours before testing (time necessary for cells to adhere at the bottom of the wellplate), cells were seeded in 96-well plate at the density of 30,000 cells per well. Cells were cultured to allow to reach confluence. The approach studies the effect of compound during the replication phase (after cell infection) - Briefly, the virus was prepared at one multiplicity of infection (M.O.I=~0.01). As the titer of virus stock is 5x10e4 pfu/mL, virus master mix is calculated and prepared according to the number of Calu3 cells/well at the time of the experiment. - The cell media contained in the plate is removed and 100 µL of the virus compound was add immediately to the dedicated wells. Plates were transferred to a 37°C incubator for 1 hour, and 80 µl of complete cell media added in all wells - The reference control (chloroquine diphosphate (#C6628, Sigma) was prepared at 300 µM in cell media: i.e 10x concentration, 20µl to be add to the cell). Final concentration on infected cells of compounds are described below.

| INS102 | INS103 |
|---|---|
| Conc (µM) Final | Conc (µM) Final |
| 10 | 5 |
| 1 | 1 |
| 0.1 | 0.2 |

Different cases were studied:
- Case A: One hour after infection, add a volume of 20 µL the compound at 10x concentration (compounds in contact for 72 hours with infected cells in a 37°C incubator)
- Case B: 24 hours after infection, add a volume of 20 µL the compound at 10x concentration (compounds in contact for 48 hours with infected cells in a 37°C incubator)
- Case C: 48 hours after infection, add a volume of 20 µL the compound at 10x concentration (compounds in contact for 24 hours with infected cells in a 37°C incubator)
- Case D: 6 hours before infection, add a volume of 20 µL the compound at 10x concentration (compound in contact for 72 hours with infected cells in a 37°C incubator, and the total statin exposure time is 78 hours)
- Case E: 24 hours before infection, add a volume of 20 µL the compound at 10x concentration (compound in contact for 72 hours with infected cells in a 37°C incubator, and the total statin exposure time is 96 hours)
- Case F: One hour before infection, add a volume of 20 µL the compound at 10x concentration (compound in contact for 72 hours with infected cells in a 37°C incubator, and the total statin exposure time is 73 hours)

At the end of the study, from cell plates with virus, all supernatant is collected and stored for viral load quantification by RT-qPCR.

**Cell viability.** Cell viability was measured by Cell Titer Glow Kit by Promega, which measures ATP in the cells. The protocol for the kit can be found on the Promega website under the CellTiter-Glo 2.0 Cell viability Assay page.

**Supernatant collection for IL6 assay by ELISA.** At the end of the experiment (72 hours post infection), supernatant from individual wells was collected, and split into 3 individual plates to avoid freeze/thaw cycles. One arm of the study is to assay the Interleukin-6 (IL6) by the Elisa^{®} technique. The protocol strictly followed the manufacturer's recommendations (#430507, LEGEND MAX^{™} Human IL-6 ELISA Kit, BioLegend).

**Supernatant collection for assay of 10-plex panel of cytokines.** The other arm of the study is to assay a 10-plex panel of cytokines at the end of the experiment (IL6, IL8, IL10, TNFα and IL1α, IL1β, IL18, Eotaxin-3, MCP-1, IP10). This approach simultaneously analyzes multiple cytokine and chemokines biomarkers with bead-based Multiplex Assays using the Luminex technology. The protocol strictly followed the manufacturer's recommendations (#MX3227W-PPX10, Life Technologies).

**Supernatant collection for RT-qPCR technology.** The third arm of the study is in quantification of viral load by RTqPCR at the end of the experiment. Among the targeted region, these assays include the use of IP2/IP4 gene (based on the protocol published by the French Pasteur Institut listed by the WHO: https://www.who.int/docs/default-source/coronaviruse/real-time-rt-pcr-assays-for-the-detection-of-sars-cov-2-institut-pasteur-paris.pdf?sfvrsn=3662fcb6_2).

Extraction of viral RNA was performed using the QIAamp Viral RNA Mini Kit (Qiagen) or similar. RNA was frozen at -20°C until RTqPCR; complete RT-PCR was run using SuperScript^{™} III One-Step QRT-PCR System kit (commercial kit#1732-020, Life Tehnologies) with primers and RT-PCR conditions targeted IP2/IP4 genes. Amplifications were performed using a Bio-Rad CFX96^{™} or Thermo instrument and adjoining software.

Results of the studies are provided below.

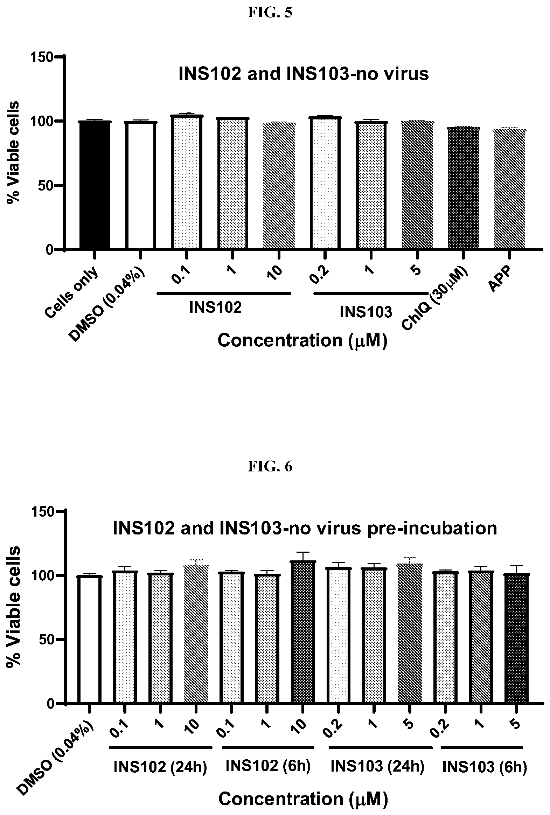

**Effect of INS-102 and INS-103 on the cell viability of Calu3 cells with no virus.** Cells were treated with the compounds for 72 hours. No loss of cell viability was observed with INS-102 and INS-103 using working concentrations as shown in **FIG. 5****.** Details of the cell set-up and treatment are provided below.

| | | | | |
|---|---|---|---|---|
| INS 102 72h TTT at 10 µM | INS103 72h TTT at 5 µM | DMSO 0.04% | Cells only | Cell medium |
| INS 102 72h TTT at 1 µM | INS 103 72h TTT at 1 µM | | | |
| INS 102 72h TTT at 0.1 µM | INS103 72h TTT at 0.2 µM | | | |
| 72h Chloroquine 30 µM | 72h Chloroquine 30 µM | | | |
| Apilimod 72h | Apilimod 72h | | | |
| Cells only | | | | |

APP refers to apilimod, which is an anti-proliferative agent and ChIQ refers to chloroquine. The concentration is reported in µM.

The cells were also pre-treated with compounds for 6 or 24 hours and then a DMSO containing medium is added for 72 hours. No loss of cell viability was observed with INS-102 and INS-103 using working concentrations as shown in **FIG. 6****.** Concentrations are reported in µM. Details of the cell set-up and treatment are provided below.

| | | | |
|---|---|---|---|
| INS 102 10 µM preinc 24h | INS 103 5 µM preinc 24h | Cells + DMSO 0.04% | Cell medium |
| INS 102 1 µM preinc 24h | INS 103 1 µM preinc 24h | | |
| INS 102 0.1 µM preinc 24h | INS 103 0.2 µM preinc 24h | | |
| INS 102 10 µM preinc 6h | INS 103 5 µM preinc 6h | | |
| INS 102 1 µM preinc 6h | INS 103 1 µM preinc 6h | | |
| INS 102 0.1 µM preinc 6h | INS 103 0.2 µM preinc 6h | | |

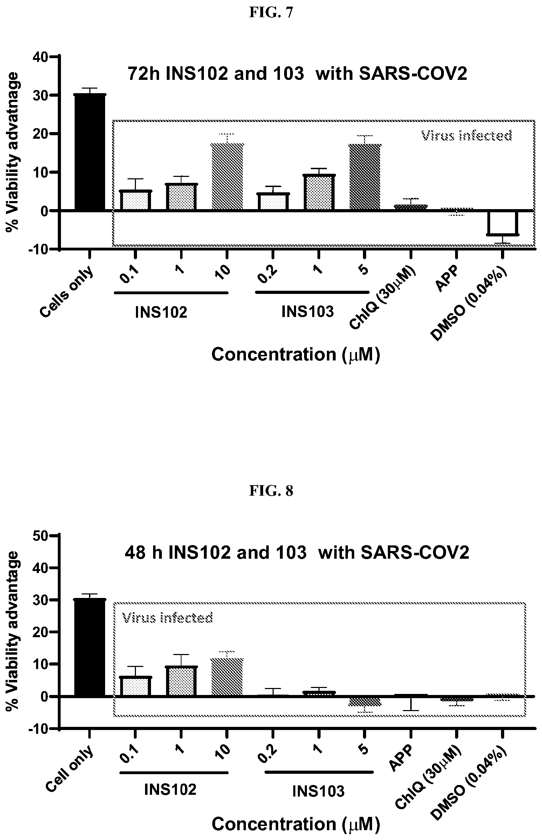

**Case A.** Cells were infected with virus for 1 hour, and then INS102 or INS103 was added, wherein the compound was in contact for 72 hours with the infected cell. It was found that there was dose-dependent protection against virus-induced loss of cell viability with INS102 and INS103 treatment as shown in **FIG. 7****.** The percent cell viability was calculated as [(value - mean value cell infected)/(mean value of cells)] x 100. Details of the cell set-up and treatment are provided below.

| | | | | |
|---|---|---|---|---|
| 72h INS102 at 10 µM | 48h INS 102 at 10 µM | 24h INS102 at 10 µM | Cells only | Cell medium |
| 72h INS 102 at 1 µM | 48h INS 102 at 1 µM | 24h INS 102 at 1 µM | | |
| 72h INS102 at 0.1 µM | 48h INS 102 at 0.1 µM | 24h INS 102 at 0.1 µM | | |
| 72h Chloroquine 30 µM | 48h Chloroquine 30 µM | 24h Chloroquine 30 µM | | |
| Apilimod 72h | Apilimod 48h | Apilimod 24h | | |
| Cells + virus (DMSO 0.4%) | | Cells + virus | | |

| | | | | |
|---|---|---|---|---|
| 72h INS103 at 5 µM | 48h INS 103 at 5 µM | 24h INS103 at 5 µM | Cells only | Cell medium |
| 72h INS103 at 1 µM | 48h INS 103 at 1 µM | 24h INS 103 at 1 µM | | |
| 72h INS 103 at 0.2 µM | 48h INS 103 at 0.2 µM | 24h INS 103 at 0.2 µM | | |
| 72h Chloroquine 30 µM | 48h Chloroquine 30 µM | 24h Chloroquine 30 µM | | |
| Apilimod 72h | Apilimod 48h | Apilimod 24h | | |
| Cells + virus (DMSO 0.4%) | | Cells + virus | | |

Table 3 below provides the statistics compared with DMSO treated samples.

**Table 3. t-test of INS102 and INS103 compared to DMSO for Case A**

| | **ttest compared to DMSO** | | | | | |
|---|---|---|---|---|---|---|
| | **INS102** | | | **INS103** | | |
| | 10 | 1 | 0.1 | 5 | 1 | 0.2 |
| Average | 17.47765806 | 7.303782644 | 5.50564 | 17.37485163 | 9.66047837 | 4.829509 |
| ttest comp | **0.00023591** | **0.00448677** | 0.119105 | **6.84303E-05** | **0.000118625** | **0.028439** |

| | | | | | | |
|---|---|---|---|---|---|---|
| Significant values are bolded. | | | | | | |

T-test results shown above demonstrated that INS102 doses of 10 uM and 1 uM and INS103 doses at 5 uM, 1 uM and 0.2uM were significant when compared to the DMSO control.

**Case B.** Cells were infected with virus for 24 hours, and then INS102 or INS103 was added, so that the compound was in contact for 48 hours with the infected cell. It was found that there was dose-dependent protection against virus-induced loss of cell viability with INS102 treatment as shown in **FIG. 8****.** The percent cell viability is calculated as described above.

Table 4 below provides the statistics compared with DMSO treated samples for Case B. T-test results demonstrated that INS102 doses of 10 uM and 1 uM were significant when compared to the DMSO control.

**Table 4. t-test of INS102 and INS103 compared to DMSO for Case B**

| | **ttest compared to DMSO** | | | | | |
|---|---|---|---|---|---|---|
| | **INS102** | | | **INS103** | | |
| | 10 | 1 | 0.1 | 5 | 1 | 0.2 |
| Average | 11.84593766 | 9.59690771 | 6.426665 | -3.02491288 | 1.723507577 | 0.524263 |
| ttest comp | **0.000904341** | **0.03736156** | 0.072083 | 0.195583434 | 0.2981665 | 0.825995 |

| | | | | | | |
|---|---|---|---|---|---|---|
| Significant values are bolded. | | | | | | |

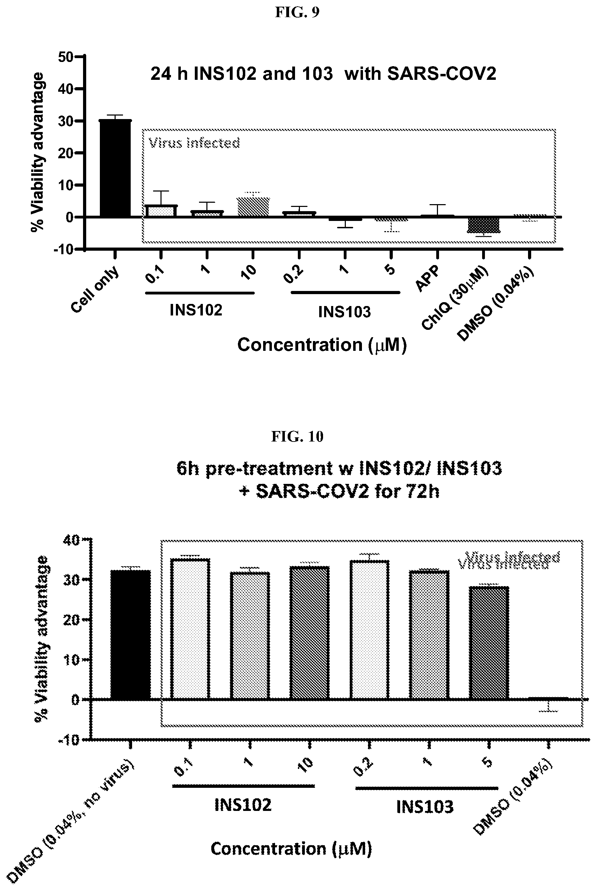

**Case C.** Cells were infected with virus for 48 hours, and then INS102 or INS103 was added, wherein the compound was in contact for 24 hours with the infected cell. Data is presented in **FIG. 9****.** The percent cell viability calculated as described above.

Table 5 below provides the statistics compared with DMSO treated samples for Case C. T-test results shown above demonstrated that INS102 a dose of 10 uM was significant when compared to the DMSO control.

**Table 5. t-test of INS102 and INS103 compared to DMSO for Case C**

| | **ttest compared to DMSO** | | | | | |
|---|---|---|---|---|---|---|
| | **INS102** | | | **INS103** | | |
| | 10 | 1 | 0.1 | 5 | 1 | 0.2 |
| Average | 6.116941423 | 2.160134928 | 3.966607 | -1.20825217 | -1.19209527 | 1.84658 |
| ttest comp | **0.009801443** | 0.463562459 | 0.401461 | 0.743479179 | 0.622662622 | 0.354776 |

| | | | | | | |
|---|---|---|---|---|---|---|
| Significant values are bolded. | | | | | | |

**Case D.** The cells were pre-treated with INS102 or INS103 for 6 hours and then infected with the virus for 72 hours, wherein the compound was in contact for 78 hours with the cell. It was found that there was dose-independent protection against virus-induced loss of cell viability with INS102 and INS103 treatment as shown in **FIG. 10****.** The percent cell viability was calculated as described above. Details of the cell set-up and treatment are provided below.

| | | | |
|---|---|---|---|
| INS 102 10 µM preinc 6h | INS 103 5 µM preinc 6h | Cells + DMSO 0.04% | Cell medium |
| INS 102 1 µM preinc 6h | INS 103 1 µM preinc 6h | | |
| INS 102 0.1 µM preinc 6h | INS 103 0.2 µM preinc 6h | | |
| Cells + virus (DMSO 0.04%) | | | |

Table 6 below provides the statistics compared with DMSO treated samples for Case D. T-test results shown above demonstrated that all doses of INS 102 and INS 103 were significant when compared to the DMSO control.

**Table 6. t-test of INS102 and INS103 compared to DMSO for Case D**

| | **ttest compared to DMSO** | | | | | | |
|---|---|---|---|---|---|---|---|
| | **INS102** | | | **INS103** | | | |
| | 10 | 1 | 0.1 | 5 | 1 | 0.2 | DMSO (0.4%) |
| Average | 33.31332212 | 31.90428836 | 35.2639316 | 28.2990113 | 32.21346923 | 34.82928603 | 0 |
| SEM | 0.977419087 | 0.982337629 | 0.712336889 | 0.53586786 | 0.361633162 | 1.534413934 | 2.88721954 |
| ttest comp | **3.42802 E-05** | **4.36773 E-05** | **3.63716 E-05** | **0.00014214** | **8.67483 E-05** | **1.63115 E-05** | |

| | | | | | | | |
|---|---|---|---|---|---|---|---|
| Significant values are bolded. | | | | | | | |

Table 7 below provides the 1-Way ANOVA Dunnett's multiple comparisons test.

**Table 7. Dunnett's multiple comparisons test for Case D**

| 1-Way ANOVA | |
|---|---|
| Dunnett's multiple comparisons test | Summary |
| DMSO (0.04%)+Virus vs. INS102_10 uM | **** |
| DMSO (0.04%)+Virus vs. INS102_1 uM | **** |
| DMSO (0.04%)+Virus vs. INS102_0.1 uM | **** |
| DMSO (0.04%)+Virus vs. INS103_5 uM | **** |
| DMSO (0.04%)+Virus vs. INS103_1 uM | **** |
| DMSO (0.04%)+Virus vs. INS103_0.2 uM | **** |
| DMSO (0.04%)+Virus vs. DMSO (0.04%, no virus) | **** |
| **** p<0.0001 | |

**Case E.** The cells were pre-treated with INS102 or INS103 for 24 hours, and then infected with the virus for 72 hours, wherein the compound was in contact for 96 hours with the cell. It was found that there was dose-dependent protection against virus-induced loss of cell viability with high and medium doses of INS102 and high dose of INS103 treatment. The percent cell viability was calculated as described above. Details of the cell set-up and treatment are provided below.

| | | | |
|---|---|---|---|
| INS 102 10 µM preinc 24h | INS 103 5 µM preinc 24h | Cells + DMSO 0.04% | Cell medium |
| INS 102 1 µM preinc 24h | INS 103 1 µM preinc 24h | | |
| INS 102 0.1 µM preinc 24h | INS103 0.2 µM preinc 24h | | |
| Cells + virus (DMSO 0.04%) | | | |

Table 8 below provides the statistics compared with DMSO treated samples for Case E. T-test results shown above demonstrated that INS102 at a dose of 10 uM and INS103 at a dose of 5 uM were significant when compared to the DMSO control.

**Table 8. t-test of INS102 and INS103 compared to DMSO for Case E**

| Student's test compared to Cell+Virus (DMSO 0.04%) | | | | | | | | |
|---|---|---|---|---|---|---|---|---|
| | | **INS102** | | | **INS103** | | **Cell+Virus (DMSO 0.04%)** | **Cell + DMSO 0.04%** |
| concentration (uM) | 10.0 | 1.0 | 0.1 | 5.0 | 1.0 | 0.2 | | |
| Average | 46.90 | 29.28 | -0.98 | 46.71 | -0.20 | -3.00 | -2.11 | 100.00 |
| SEM | 10.69 | 12.20 | 9.19 | 11.67 | 6.62 | 9.66 | 20.73 | 2.96 |
| ttest | 0.01697 | 0.11399 | 0.91685 | 0.00683 | 0.81394 | 0.93693 | | |

Table 9 below provides the 1-Way ANOVA Dunnett's multiple comparisons test.

**Table 9. Dunnett's multiple comparisons test for Case E**

| 1-Way ANOVA | |
|---|---|
| Dunnett's multiple comparisons test | Summary |
| DMSO (0.04%)+Virus vs. INS102_10 uM | *** |
| DMSO (0.04%)+Virus vs. INS102_1 uM | * |
| DMSO (0.04%)+Virus vs. INS102_0.1 uM | ns |
| DMSO (0.04%)+Virus vs. INS103_5 uM | *** |
| DMSO (0.04%)+Virus vs. INS103_1 uM | ns |
| DMSO (0.04%)+Virus vs. INS103_0.2 uM | ns |
| DMSO (0.04%)+Virus vs. DMSO (0.04%, no virus) | **** |
| **** p<0.0001; *** P<0.001; * p<0.05 | |

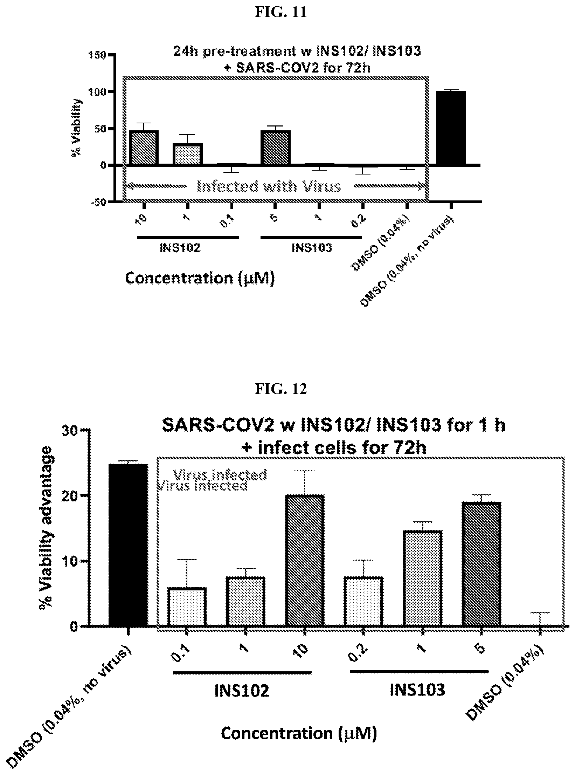

**Case F.** The cells were per-treated with INS102 or INS103 for 1 hour, and then infected with the virus for 72 hours, wherein the compound was in contact for 73 hours with the cell. It was found that there was dose-dependent anti-viral protection with high dose of INS102 and all doses of INS103 treatment as shown in **FIG. 12****.** The percent cell viability was calculated as described above. Details of the cell set-up and treatment are provided below.

| | | | |
|---|---|---|---|
| INS102 10 µM coinc 1h | INS103 5 µM coinc 1h | Cells + DMSO 0.04% | Cell medium |
| INS102 1 µM coinc 1h | INS103 1 µM coinc 1h | | |
| INS102 0.1 µM coinc 1h | INS 103 0.2 µM coinc 1h | | |
| Cells + virus (DMSO 0.04%) | | | |

Table 10 below provides the statistics compared with DMSO treated samples for Case E. T-test results shown above demonstrated that INS102 doses of 1 uM and INS103 doses at 5 µM, 1 µM and 0.2 µM were significant when compared to the DMSO control.

**Table 10. t-test of INS102 and INS103 compared to DMSO for Case F**

| | **ttest compared to DMSO** | | | | | | |
|---|---|---|---|---|---|---|---|
| | **INS102** | | | **INS103** | | | |
| | 10 | 1 | 0.1 | 5 | 1 | 0.2 | DMSO (0.4%) |
| Average | 19.3953702 | 8.72147798 | 10.1010452 | 19.9647454 | 15.8159441 | 9.88105928 | 1.66097239 |
| SEM | 5.01166203 | 0.83033759 | 1.54032519 | 0.76616811 | 0.91744314 | 1.52254329 | 1.60660458 |
| ttest compared to DMSO | 0.18993608 | **0.018238** | **0.04807105** | **0.00026152** | **0.00128653** | **0.04949338** | |

| | | | | | | | |
|---|---|---|---|---|---|---|---|
| Significant values are bolded. | | | | | | | |

Table 11 below provides the 1-Way ANOVA Dunnett's multiple comparisons test.

**Table 11. Dunnett's multiple comparisons test for Case F**

| 1-Way ANOVA | |
|---|---|
| Dunnett's multiple comparisons test | Summary |
| DMSO (0.04%)+Virus vs. INS102_10 uM | **** |
| DMSO (0.04%)+Virus vs. INS102_1 uM | ns |
| DMSO (0.04%)+Virus vs. INS102_0.1 uM | ns |
| DMSO (0.04%)+Virus vs. INS103_5 uM | **** |
| DMSO (0.04%)+Virus vs. INS103_1 uM | *** |
| DMSO (0.04%)+Virus vs. INS103_0.2 uM | ns |
| DMSO (0.04%)+Virus vs. DMSO (0.04%, no virus) | **** |
| **** p<0.00001; *** p<0.001 | |

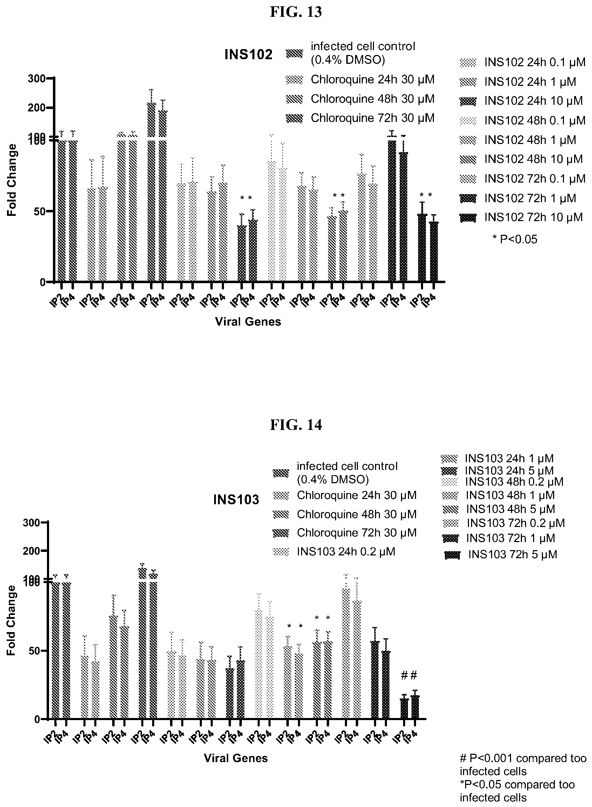

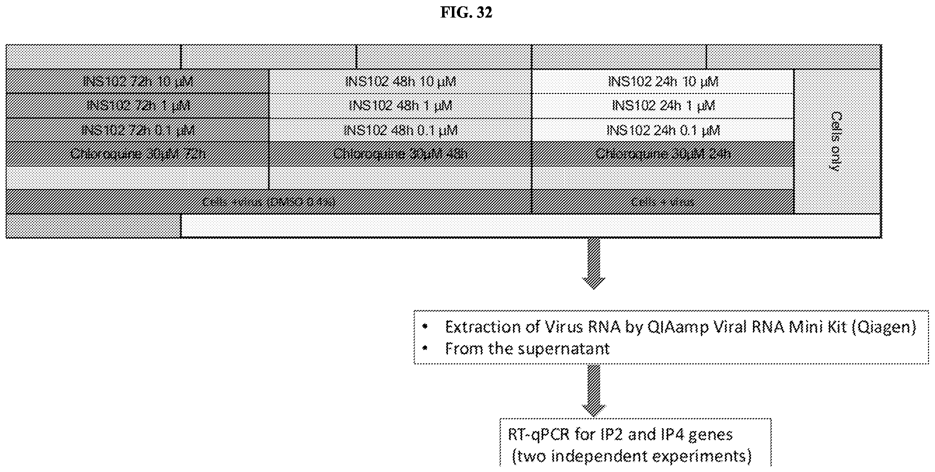

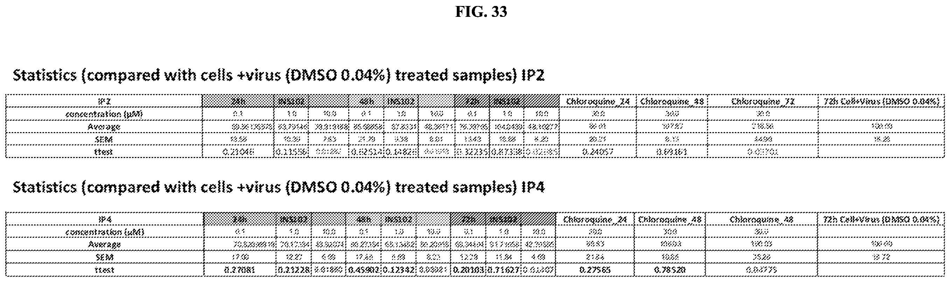

Effects of INS102 on SARS-CoV-2 viral load for Cases A-C are illustrated: INS-102 at 10 µM dose reduced viral load at 24, 48, and 72 hours as shown in **FIG. 13****.** Details of the cell set-up and treatment are shown in **FIG. 32****.** The statistical analysis is shown in **FIG. 33****.**

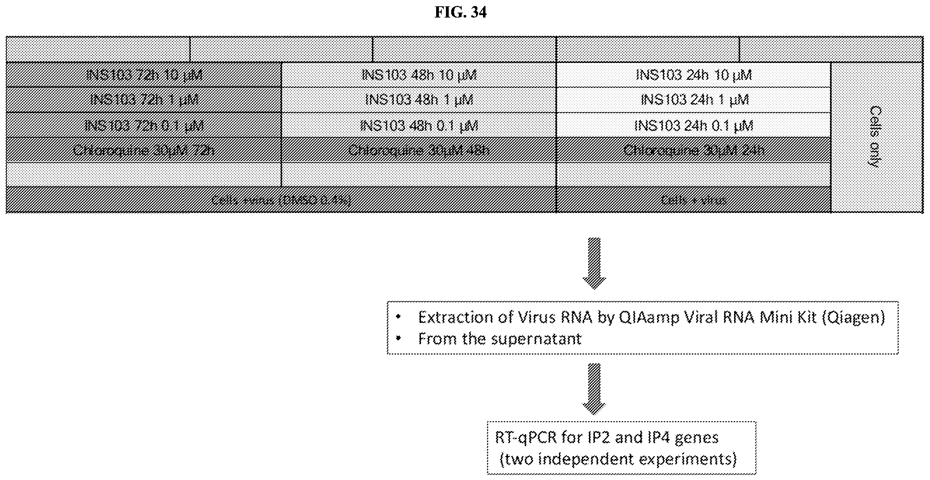

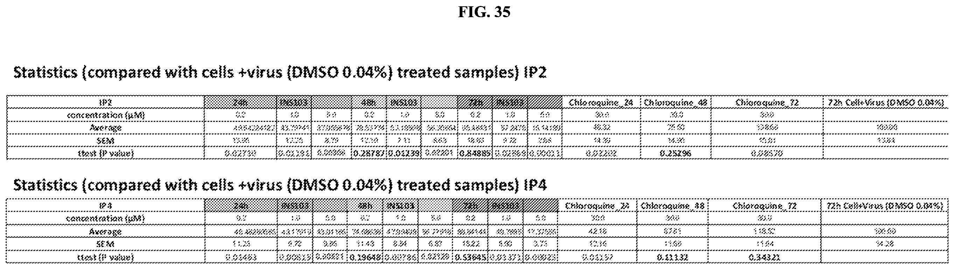

Effects of INS103 on SARS-CoV-2 viral load for Cases A-C are illustrated: INS-103 dose-dependently reduced viral load as shown in **FIG. 14****.** Details of the cell set-up and treatment are provided in **FIG. 34****.** The statistical analysis is shown in **FIG. 35****.**

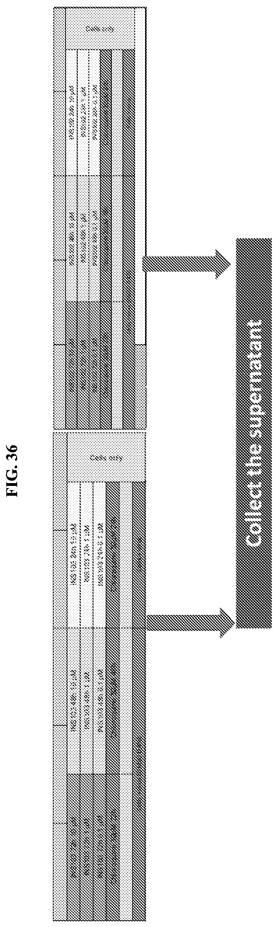

The cell studies set-up for INS102 and INS103 are shown in **FIG. 36****.**

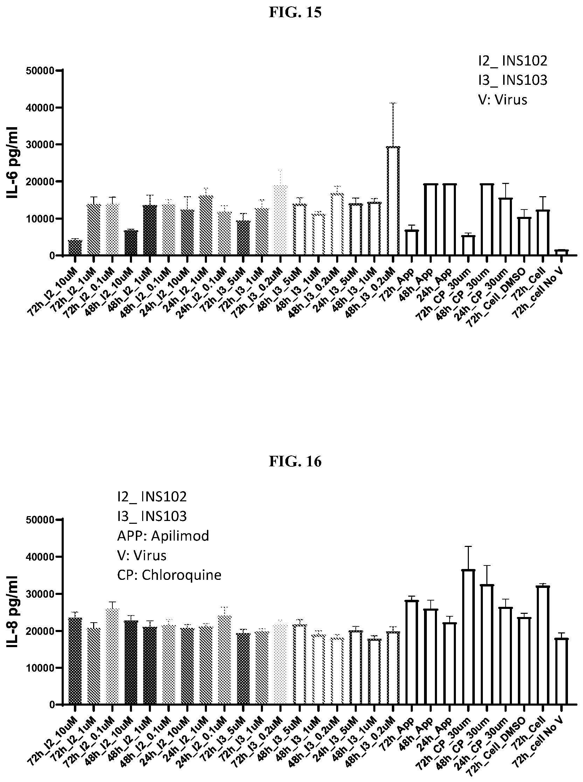

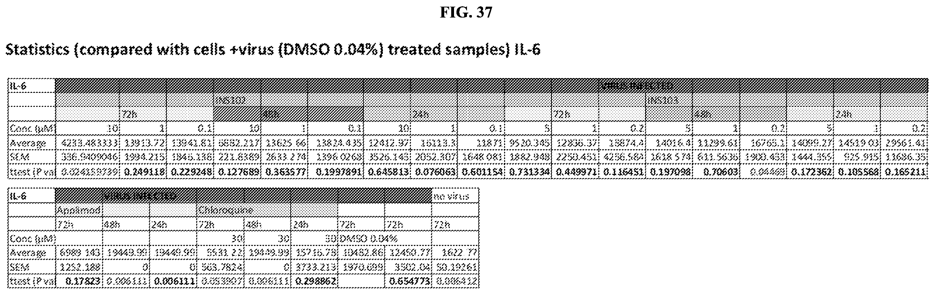

**Luminex.** The cytokines used in the Luminex experiment are as defined above. Results from the experiment on IL-6 production for Cases A-C are shown in **FIG. 15****.** The statistical analysis is shown in **FIG 37****.**

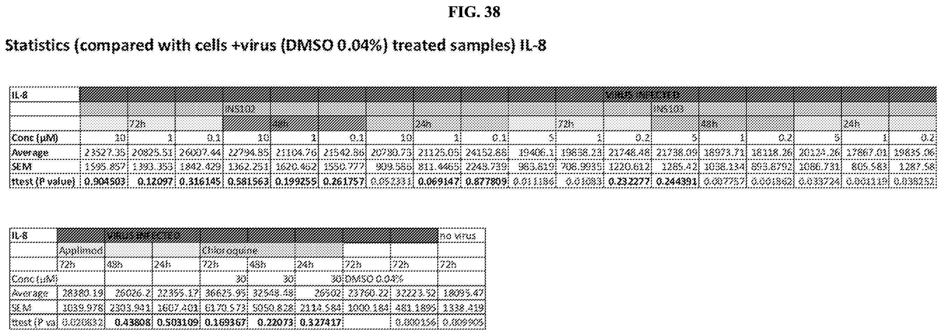

Results from the experiment on IL-8 production for Cases A-C are shown in **FIG. 16****.** The statistical analysis is shown in **FIG. 38****.**

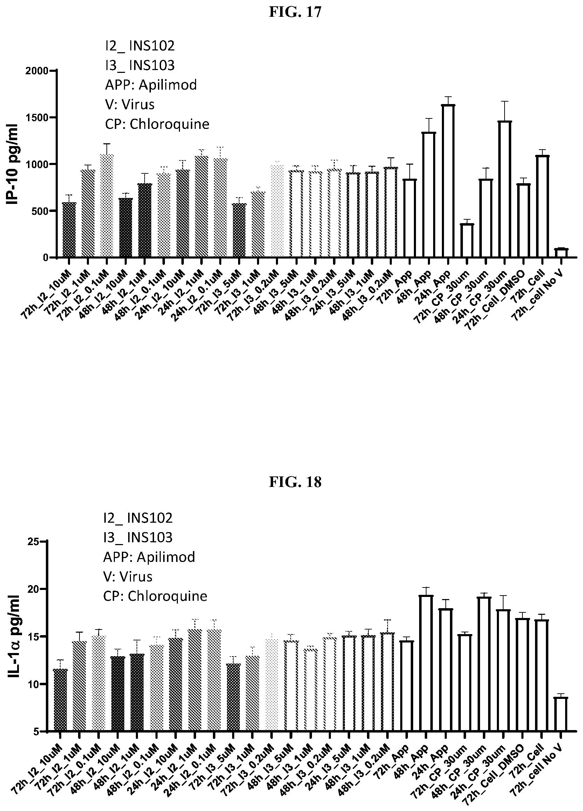

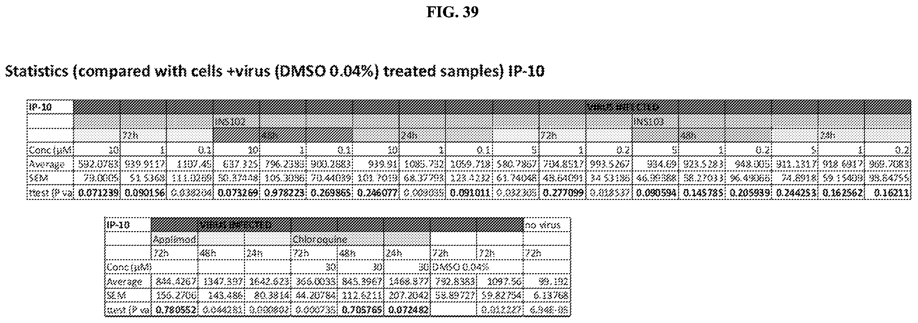

Results from the experiment on IL-10 production for Cases A-C are shown in **FIG. 17****.** The statistical analysis is shown in **FIG. 39****.**

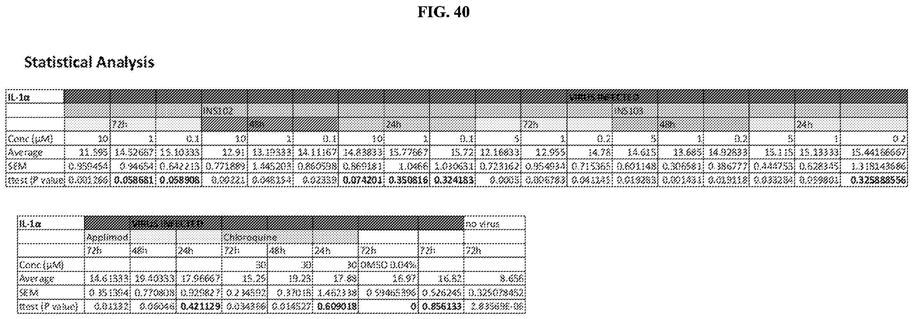

Results from the experiment on IL-1α production for Cases A-C are shown in **FIG. 18****.** The statistical analysis is shown in **FIG. 40****.**

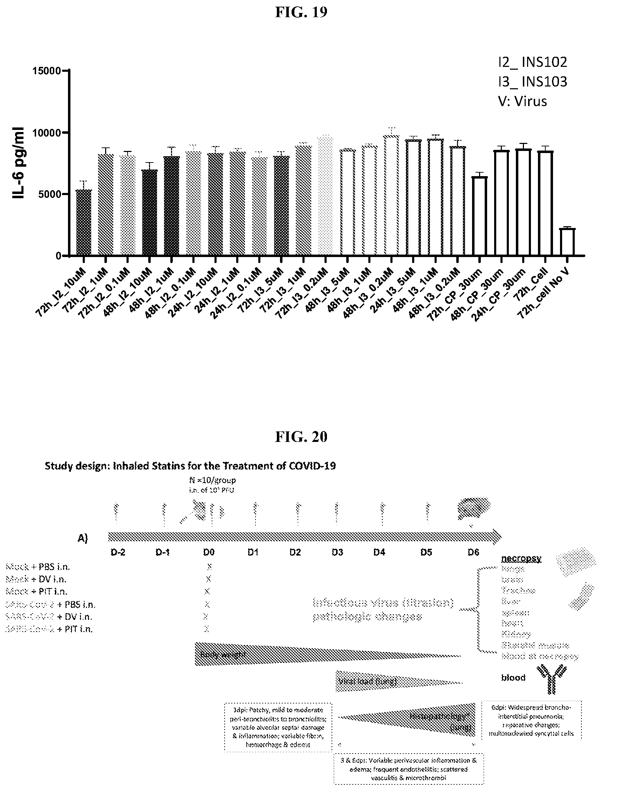

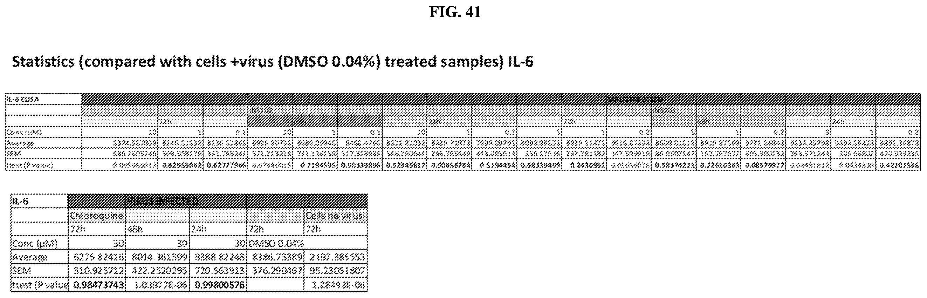

**ELISA.** The setup of the ELISA experiment is defined above. Results from the experiment on the IL-6 production for Cases A-C are shown in **FIG. 19****.** The statistical analysis is shown in **FIG. 41****.**

The Oncodesign studies have shown that SARS-CoV-2 reduces Calu-3 cell viability. As demonstrated herein, INS-102 and INS-103 were not cytotoxic to Calu-3 cells or Vero cells. INS-102 and INS-103 inhibited SARS-CoV-2-induced loss of cell viability in Calu-3 cells. INS-102 and INS-103 pre-treatment for 6 hrs inhibit SARS-CoV-2-induced loss of cell viability in Calu-3 cells. Incubation of SARS-CoV-2 with INS compounds inhibited SARS-CoV-2-induced loss of cell viability in Calu-3 cells. High doses (10 uM INS-102, 5 uM INS-103) reduced SARS-CoV-2 viral load. INS-102 and INS-103 both inhibited SARS-CoV-2-induced cytokine and chemokine production in Calu-3 cells.

### EXAMPLE 6: IN VIVO INHALATION TREATMENT OF SARS-COV-2

To test the effect of statin inhalation on SARS-CoV-2. a hamster model was chosen. Hamsters are obligate nasal breathers, and a model for respiratory diseases and treatments. A schematic of the overall study design is shown in **FIG. 20****.**

Adult male hamsters (7-9-week-old; Charles River ) were housed ABSL-3 containment, with 2 hamsters per cage. Beginning 2 days prior to infection and continuing through day 6 post-infection, hamsters were weighed daily. Also starting 2 days prior to infection, hamsters were treated with drug, PPBS or control vehicle. Drug (pitavastatin) was prepared in a citrate buffer formulation and control vehicle (DV) contained the formulation without the pitavastatin. Drug and DV samples were kept under light-protected conditions until use.

| Material | Amount (g) | Concentration (%) |
|---|---|---|
| Pitavastatin calcium | 4.18 | 0.40 (free acid) |
| Sodium citrate dihydrate | 2.42 | 0.24 |
| Citric acid | 0.34 | 0.03 |
| Sodium chloride | 7.6 | 0.76 |
| Deionized water (ml) | 1000 | |

On Day 0 (i.e., 2 days after start of treatment), a first set of 15 hamsters were inoculated intranasally with 10⁴ PFU SARS-CoV-2 in a volume of 30 µl and a second set of 15 hamsters were intranasally inoculated with the same volume of DPBS only. The inoculations (viral and control) were administered approximately 2 hours after the treatment of drug or control vehicle. Virus and control vehicle were prepared as set forth in Table 12. Prior to inoculation, hamsters were anesthetized with isoflurane in a bell jar system (2-5% saturation). Hamsters were allowed to recover from the anesthesia in an empty cage before returning to grouped housing with bedding present.

**Table 12. Virus and control groups**

| Group | Virus | Volume (virus) | Volume (diluent) |
|---|---|---|---|
| 4, 5, 6 | SARS-CoV-2 | 152 ul of 1/10 of (2.2x107 PFU/ml of UCD4 (Cov0386) | 848 µL |
| 1, 2, 3 | Mock (diluent) | 0 | 1000µl |

| | | | |
|---|---|---|---|
| Viruses were diluted to 333,333 PFU/mL (to obtain 10⁴ PFU/30 µl) | | | |

On Days 1-3 following viral infection, a throat swab was performed on each hamster. On Day 3, half of each treatment group was euthanized and the remainder of the animals were euthanized on day 6 post-infection. Treatment groups are shown in Table 13. For the throat swab, hamsters were anesthetized with isoflurane in a bell jar system (2-5% saturation) prior to swabbing. On days 3 and 6, hamsters were anesthetized with a cocktail of ketamine, xylazine, and acepromazine and then euthanized by cervical dislocation. Necropies were performed to harvest the tissues listed in Table 14.

**Table 13. Treatment Groups**

| Gr. | Virus On D₀ (10⁴ PFU in 30µl, i.n.) | Treatment (100µl, i.n.) on (D_{-2 to 5}) | Hamsters # (D3) | Hamsters # (D6) |
|---|---|---|---|---|
| 1 | DPBS | DPBS | 1M&1F | 1M&1F |
| 2 | DPBS | DV | 1M&1F | 1M&1F |
| 3 | DPBS | Pitavastatin (3 mg/kg) | 1M&1F | 1M&1F |
| 4 | SARS-CoV2 | DPBS | 2M&1F | 2M&1F |
| 5 | SARS-CoV2 | DV | 2M&1F | 2M&1F |
| 6 | SARS-CoV2 | Pitavastatin (3 mg/kg) | 2M&1F | 2M&1F |
| | Total | | 15(9M6F) | 15(9M6F) |

**Table 14. Sample collection and preservation:**

| Tissue | Snap frozen | Trizol | Formalin |
|---|---|---|---|
| Throat swab | yes | | |
| Trachea | yes | yes | yes |
| Lung | yes | yes | yes |
| brain | yes | yes | yes |
| Liver | yes | yes | yes |
| Spleen | yes | yes | yes |
| Heart | yes | yes | yes |
| kidnev | yes | yes | yes |
| Skeletal muscle | yes | yes | yes |
| Blood (serum) | yes | | |

Samples were assayed as from the harvested tissues as follows.

***Plaque Assay:*** Washes from tracheal swabs, serum, and lung and brain homogenates were thawed at 37°C and inocula were assayed directly without freezing. Samples were serially diluted 10-fold in DMEM with 1% bovine serum albumen (BSA) starting at an initial dilution of 1:8. 125 µL of each dilution was added to confluent Vero CCL-81 cells (ATCC) in 12-well cluster plates with cell culture media decanted. Virus was incubated on cells for 1 hour at 5% CO₂ in a humidified 37°C incubator. Cell monolayers were overlaid with 0.5% agarose dissolved in DMEM with 5% fetal bovine serum (FBS) and 1x antibiotic-antimycotic (Thermo Fisher) and incubated for 3 days at 5% CO₂ and 37°C in a humidified incubator. Cells were fixed for >30 minutes with 4% buffered formalin then agarose plugs were removed. Cells were stained with 0.05% crystal violet in 20% ethanol for 10 minutes then rinsed three times with water. Plates were inverted to dry completely then counted in duplicate wells. Viral titers were recorded by averaging the reciprocal of the highest dilution where plaques are noted and represented as PFU per swab or PFU per mg of solid tissues.

***Plaque Reduction Neutralization Test:*** Serum from hamsters at days 3 and 6 post inoculation was thawed at 37°C and 30 µL was heated in a water bath for 30 minutes at 56°C to inactivate complement proteins. Serum was diluted 4-fold with virus diluent consisting of PBS and 1% FBS, then samples were serially 2-fold diluted 11 times for a dynamic range of 1:4 to 1:4096. An equal volume of virus diluent containing 80 PFU of SARS-CoV-2 was added to each antibody dilution and a no-antibody control consisting of virus diluent only, resulting in a final dynamic range of 1:4 to 1:8192 with one no-antibody control. Antibody-virus dilution series were applied to confluent Vero CCL-81 cells in single-replicate and incubated for 1 hour at 5% CO2 and 37°C in a humidified incubator. Cells were overlaid, incubated, fixed, and stained as described above for plaque assays. Neutralizing titer is defined as the reciprocal of the dilution for which fewer than 20% of plaques were detected versus the no-antibody control (>80% neutralization).

***Statistics:*** All statistical tests were performed with GraphPad PRISM 9.0.2 (GraphPad Software). Logrank (Mantel-Cox) test for survival proportions were performed pairwise and p-values were adjusted with Bonferroni correction using R version 4.0.0 (R Project) p.adjust function. Correlation between mortality and positive virus detection was calculated by Fisher's exact test. Repeated measures Two-Way ANOVA was performed on log₁₀-transformed values and multiple comparisons were computed according to Tukey method. Main effect Two-Way ANOVA was performed on weights normalized to starting values at the time of virus challenge or log₁₀-transformed viral titers and multiple comparisons were computed with Tukey's method. Area under the curve (AUC) was calculated for longitudinally collected tracheal swabs and log10-transformed. ANOVA of grouped log₁₀-AUC was performed with multiple comparisons computed with Tukey's method. Kruskal-Wallis H test was performed on untransformed PRNT80 neutralization values and multiple comparisons were computed according to Dunn's method.

***Histopathology:*** At necropsy, lung was inflated with 10% buffered formalin (Thermo Fisher) and hamster tissues were fixed for 48 hours at room temperature in a 10-fold volume of 10% buffered formalin. Skulls were demineralized in a 10-fold volume of 0.5 M ethylenediamine tetraacetic acid (EDTA) (pH=7) at 4°C for 18 days, with EDTA solution exchanges every 5 days. Tissues were embedded in paraffin, thin-sectioned and stained routinely with hematoxylin and eosin (H&E). H&E slides were scanned to 40x magnification by whole-slide image technique using an Aperio slide scanner with a magnification doubler and a resolution of 0.25 µm/pixel. Image files were uploaded on a Leica hosted web-based site and a board certified veterinary anatomic pathologist blindly evaluated sections for SARS-CoV-2 induced histologic lesions. For quantitative assessment of lung inflammation, digital images were captured and analyzed using ImageJ software (Fiji) to estimate the area of inflamed tissue (visible to the naked eye at subgross magnification) as a percentage of the total surface area of the lung section.

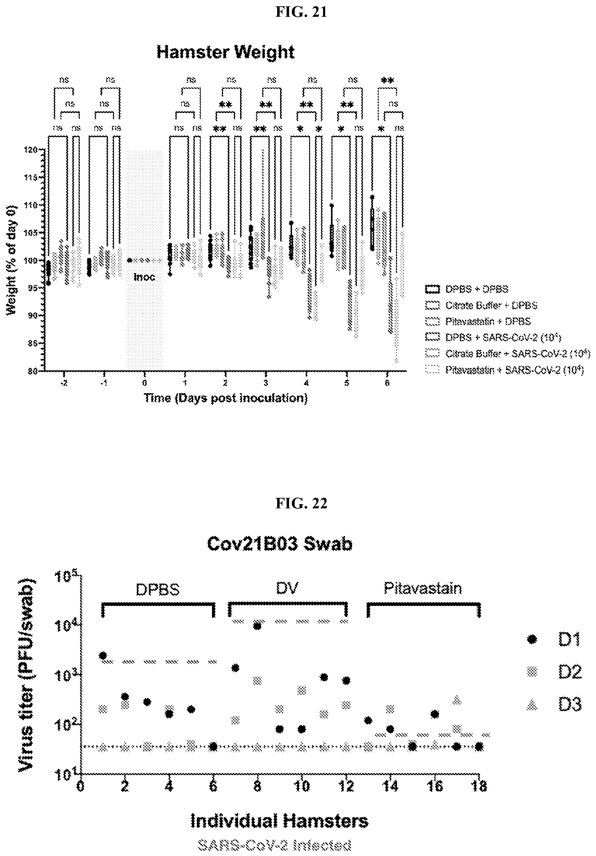

Results of the treatment study are shown in **FIGs. 21-28****.**

Protective effect of treatment with intranasally inhaled pitavastatin (as compared to control) were observed. As shown in **FIG. 21****,** animals treated with control (no virus) maintained relatively constant weight. Animals treated with the SARS-CoV-2 and no drug experienced weight loss starting at about day 3 and continuing to the end of the experiment at day 6. In contrast, animals that received the treatment of intranasally inhaled pitavastatin had less weight loss and this showed statistical significance at day 4.

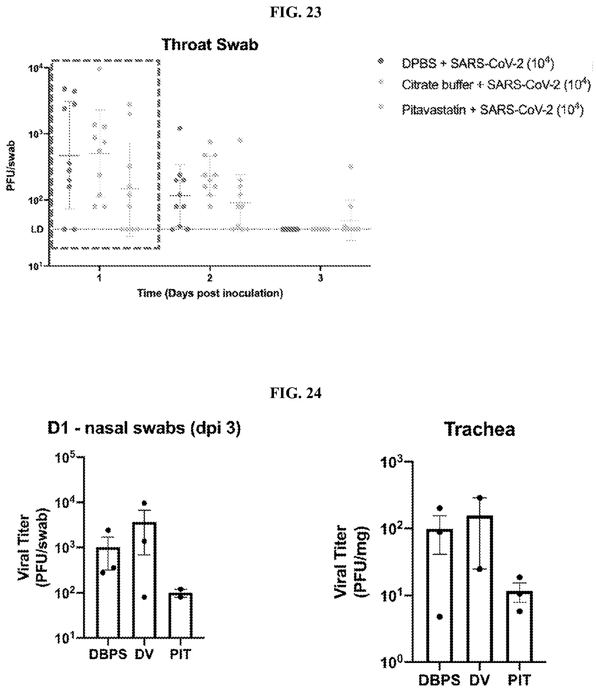

**FIG. 22** shows viral titers from the nasal swabs. Animals treated with the intranasally inhaled pitavastatin showed a trend of reduced viral titers as compared to animals treated with DPBS or control vehicle (DV). **FIG. 23** shows a comparison of the viral titers in each infection treatment group. Treatment with pitavasatin reduced viral titers in throat swabs at Day 1, but this trend was not observed at days 2 or 3. This may due to the fact that the virus is naturally cleared in upper airway and/or moves down into the lower airway of the animals.

**FIG. 24** shows the viral titer in the nasal swabs (Left panel) and tracheas (Right panel) of hamsters treated with intranasally inhaled pitavastatin and controls on Day 3 post-infection. A reduction of viral titer was seen in the pitavastatin-treated animals. This difference was not observed at the later time points.

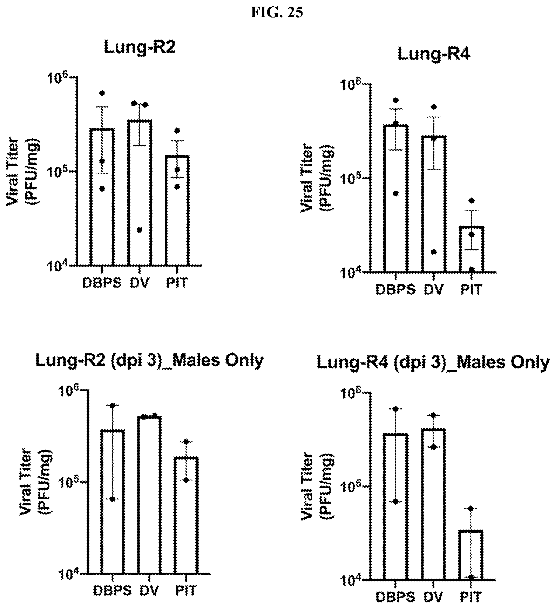

**FIG. 25** shows the viral titer in lung samples (R2 Left panels; R4 Right panel) of hamsters treated with intranasally inhaled pitavastatin and controls on Day 3 post-infection. A significant decrease in viral titer was seen in the animals that received the intranasally inhaled pitavastatin as compared to the controls in the R4 samples.

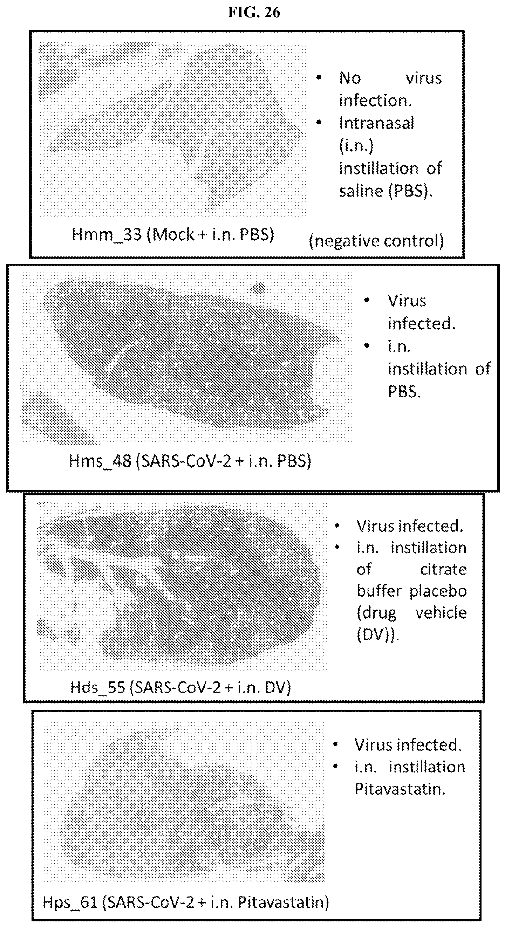

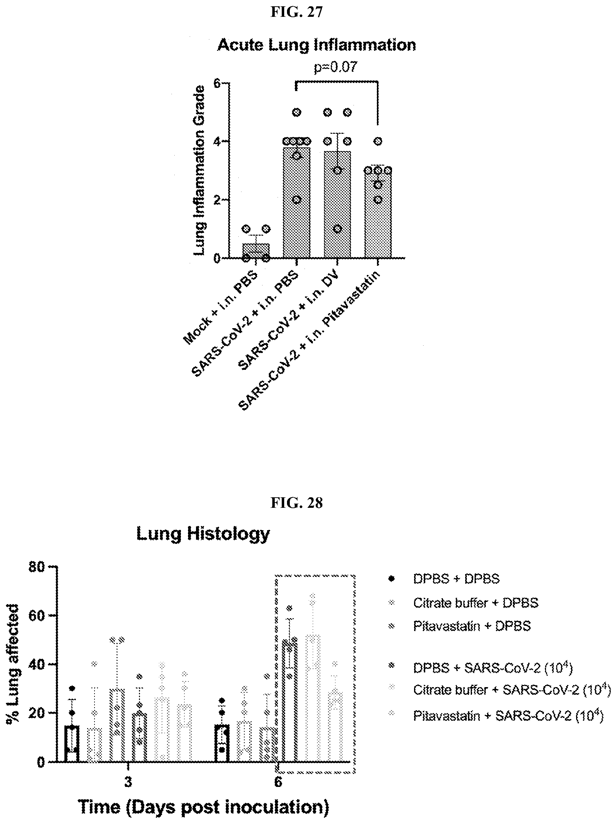

Histopathology of lungs from the treated and control samples is shown in **FIG 26****.** Hamsters treated with the intranasally inhaled pitavastatin showed a reduction in acute ling inflammation as compared to the controls. **FIG. 27** shows a semi-quantitative plot of the blinded scoring of lung inflammation grade of all the infected animals based the average +/-SEM of the lung histopathology graded according to severity of inflammation. The Mock group represents no virus infection and instillation of PBS intranasally (i.n.).

**FIG. 28** shows lung histopathology scoring based on the percentage of affected lung (i.e. the amount of lung affected by acute inflammation). All treatment groups (mock infected and virus-treated) for days 3 and 6. An examination of the animals at day 3 had only a low amount of inflammation due to viral infection (~25%). By day 6, virus-infected animals showed a clear increase in inflammation (~50%) due to SARS-CoV-2 infection. Treatment with the intranasally inhaled pitavastatin reduced the day 6 inflammation to ~30%. The dotted-lined box highlights the comparison of the virus-infected animals treated with drug and controls.

The results of the intranasally inhaled pitavastatin demonstrate a protective/therapeutic effect against SARS-CoV-2 (COVID-19) as shown by a statistically significant effect on preservation of hamster weights, trends of reduced nasal, airway, throat, and lung viral loads and a mild/moderate reduction in lung inflammation.

### EXAMPLE 7: COMBINATION THERAPY

Following the methods provided in Example 5 for infection of cells and application of therapeutic agents, the effects of statins in combination with either dexamethasone or remdesivir on viral infection were tested in Calu-3 human lung epithelial cells. Information on the cell line is provided below.

| **Cell line** | **Type** | **Species** | **Origin** |
|---|---|---|---|
| Calu-3 | Lung adenocarcinoma | Human | Elabscience |

**Cell culture conditions.** Calu-3 cell model is well described in the literature already for Sars-CoV (e.g. Tseng et al., 2005, J Virol, https://doi:10.1128/JVI.79.15.9470-9479). Calu-3 cells were grown as monolayer at 37°C in a humidified atmosphere (5% CO₂, 95% air) into the corresponding cell culture medium (MEM + 1% Pyruvate + 1% glutamine + 10% Fetal Bovine Serum). All the cells are adherent to plastic flasks. For cell passaging procedures, cells will be detached from the culture flask by a 5-min treatment with trypsin-versene and neutralized by addition of complete culture medium. For the study, cells were coated on 96-well plates. The cells were counted and their viability were assessed by using V-cell counter.

**Virus isolate.** The virus strain was supplied through the European Virus Archive goes Global (Evag) platform (https://www.european-virus-archive.com/). For this study, Slovakia isolate was used (reference SARS-CoV-2 strain Slovakia/SK-BMC5/2020). Viral titer: SARS-Cov2 was amplified and titered on Vero E6 TMPRSS2 cell line (origin NIBsc, UK) by Oncodesign.

Calu-3 cells were counted and their viability was assessed using Vi-Cell automatic apparatus. Cells were cultured to reach confluence in 96-well plates seeding. The approach will consist to study the effect of compounds during the infection and replication phase (two arms were completed: cell treated with compounds before infection and virus treated by compounds before cell infection). Prepare the virus at one multiplicity of infection (M.O.I=~0.01) based on what we obtained during experimental phase from virus stock. As the titer of virus stock is 1.5x10e6 pfu/mL, virus master mix was calculated and prepared according to the number of Calu3 cells/well at the time of the experiment.

**Case 1.** Cells were treated for 6 hours with one of the statins (INS-102, INS-103, or INS-104) as monotherapy or in combination with dexamethasone (Dex) or remdesivir (Remde) and incubated at 37 °C. After the 6 hour pre-treatment, the cells were infected at a MOI of 0.01 with the SARS-CoV-2 virus and incubated at 37 °C for 72 hr. Control and comparison treatments were also performed with vehicle only (DMSO), Dex alone and Remde alone. Statins were tested at 10 µM, 1 µM, and 0.1 µM or 5 µM, 1 µM, and 0.2 µM , Dex at 10 µM, 1 µM, and 0.1 µM and Remde at 1 nM, 10 nM and 100 nM.

**Case 2.** Cells were treated for 1 hours with one of the statins (INS-102, INS-103, or INS-104) as monotherapy or in combination with dexamethasone (Dex) or remdesivir (Remde) at room temperature. After the 1 hour pre-treatment, the cells were infected at a MOI of 0.01 with the SARS-CoV-2 virus and incubated at 37 °C for 72 hr. Control and comparison treatments were also performed with vehicle only (DMSO), Dex alone and Remde alone. Statins were tested at 10 µM, 1 µM, and 0.1 µM or 5 µM, 1 µM, and 0.2 µM , Dex at 10 µM, 1 µM, and 0.1 µM and Remde at 1 nM, 10 nM and 100 nM.

Cells were harvested 24 hrs post infection and viral Load measured by RT-PCR of relative gene expression for the open-reading frame 1ab (ORF1ab) gene, the largest one of SARS-CoV-2, encoding polyprotein PPlab and PP1a responsible for viral transcription and replication. Gene expression was assessed by RT-PCR with the following primers: RT PCR primers: ORF1ab_Fw CCGCAAGGTTCTTCTTCGTAAG; ORF1ab_Rv TGCTATGTTTAGTGTTCCAGTTTTC; ORF1ab_probe AAGGATCAGTGCCAAGCTCGTCGCC [5']HEX [3'] BHQ-1.

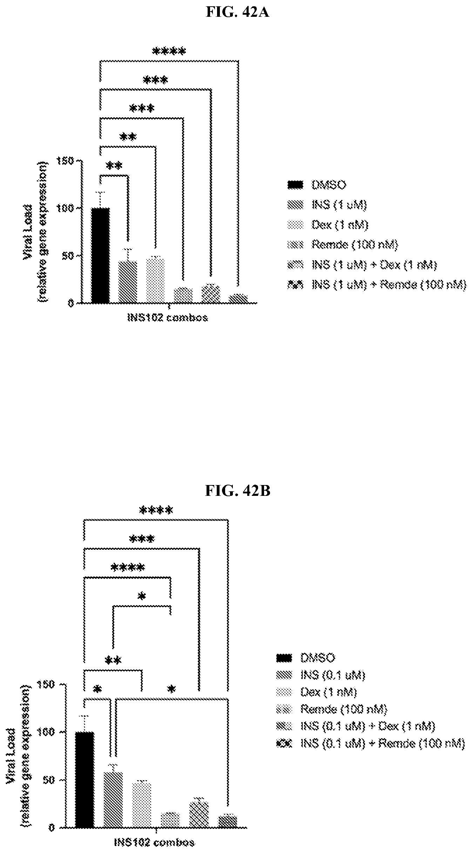

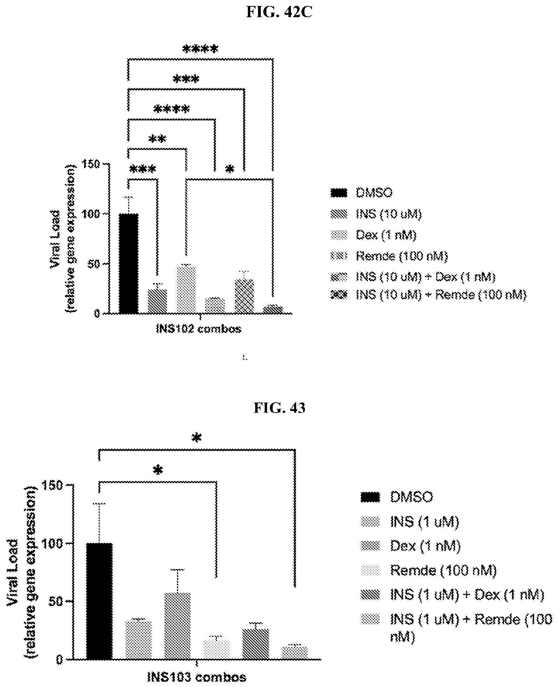

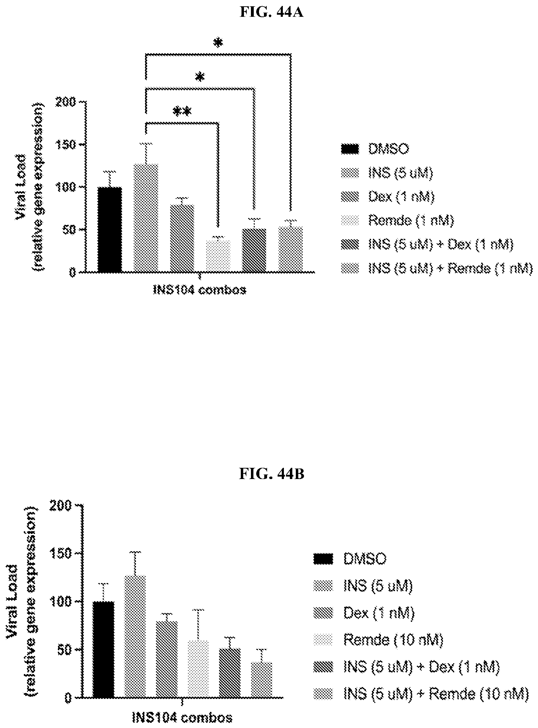

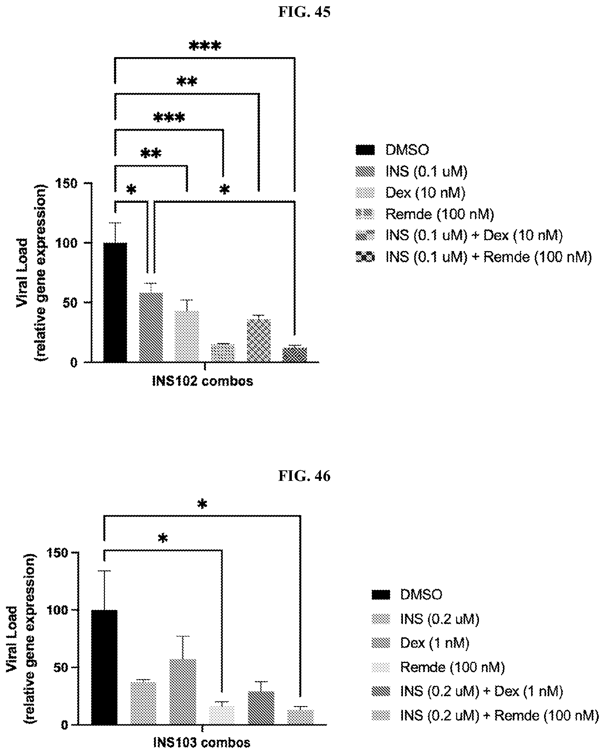

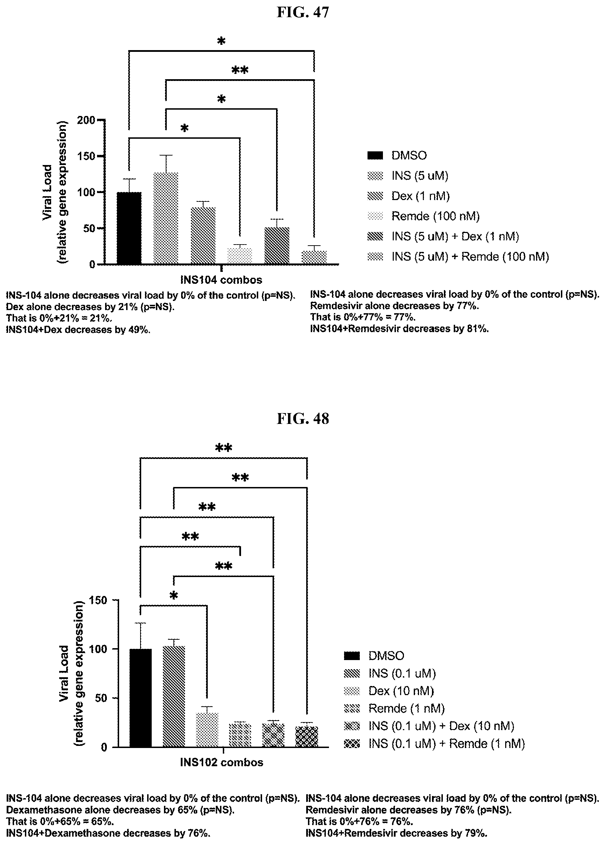

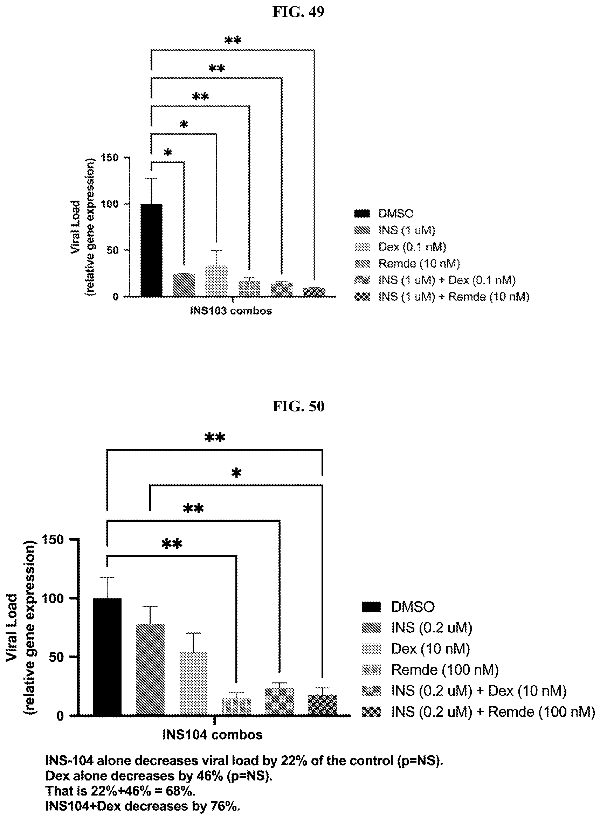

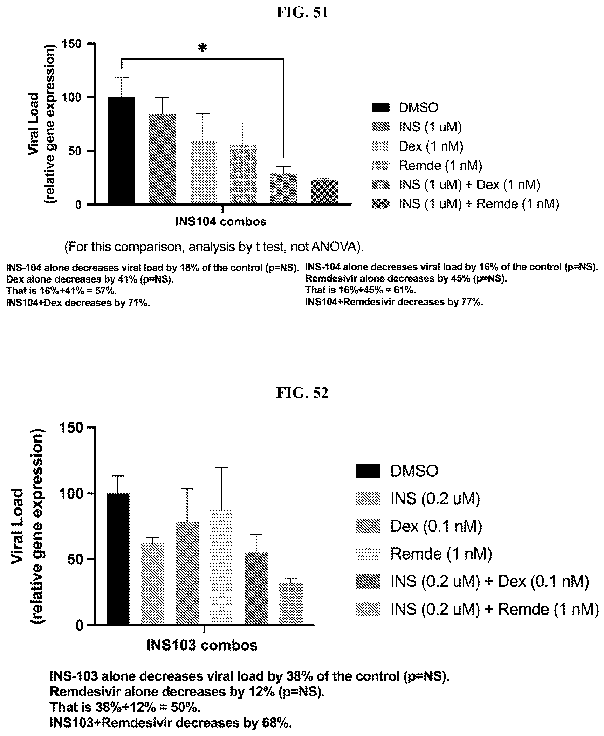

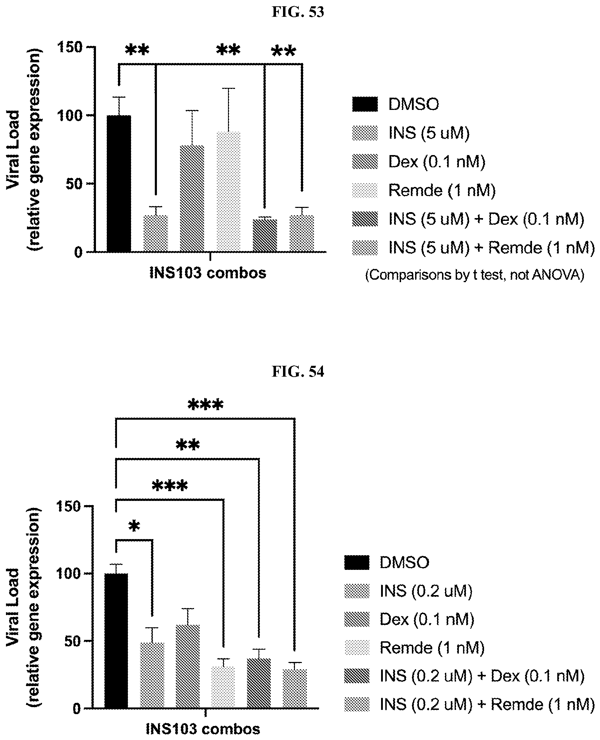

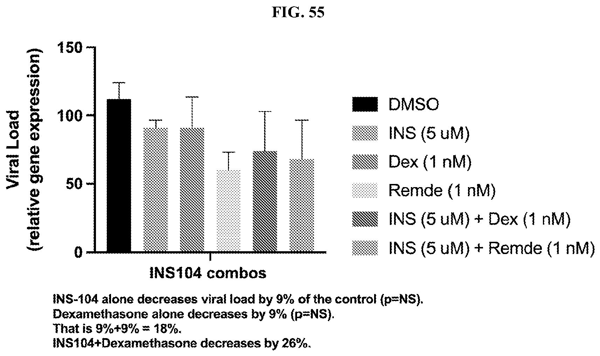

Results are shown in Tables 15-17 below and exemplary bar graphs are shown in **FIGs. 42-55****.**

Viral load was decreased as compared to DMSO control in all single agent treatments. Each combination that showed an enhanced decrease of viral load as compared with the two single agents is denoted by "E". Combinations that showed a slight trend towards an enhanced decrease of viral load as compared with the two single agents is denoted by "(e)".

The combination of INS-102 and dexamethasone showed an enhancement in the decrease of viral load as compared to the single agents with INS-102 at 1 µM and Dex at 0.1 µM and 1 µM, and also with INS-102 at 0.1 µM and Dex at 1 µM and 10 µM . The combination of INS-102 and remdesivir showed an enhancement in the decrease of viral load as compared to the single agents with INS-102 at 1 µM and 10 µM when remdesivir was provided at 100 nM.

**Table 15. Combination Data for INS-102**

| **INS-102** | **Dex 0.1 µM** | **Dex 1 µM** | **Dex 10 µM** |
|---|---|---|---|
| hi 10 µM | - | - | - |
| med 1 µM | E | E | - |
| low 0.1 µM | - | E | E |
| | | | |

| **INS-102** | **Remde 1** | **Remde 10** | **Remde 100** |
|---|---|---|---|
| hi 10 µM | - | - | E |
| med 1 µM | - | - | E |
| low 0.1 µM | - | - | - |

The combinations of INS-103 with the additional agents showed less enhancement on decreasing viral load. The combination of INS-103 at 1 µM and Dex at 1 µM demonstrated an enhancement in the decrease of viral load as compared to the single agents.

**Table 16. Combination Data for INS-103**

| **INS-103** | **Dex 0.1 µM** | **Dex 1 µM** | **Dex 10 µM** |
|---|---|---|---|
| hi 5 µM | E | - | - |
| med 1 µM | - | E | - |
| low 0.2 µM | - | (e) | - |
| | | | |

| **INS-103** | **Remde 1** | **Remde 10** | **Remde 100** |
|---|---|---|---|
| hi 5 µM | - | - | - |
| med 1 µM | - | - | - |
| low 0.2 µM | (e) | (e) | (e) |

The combination of INS-104 at 5µM and Dex at 1 µM showed a synergistic enhancement in the decrease of viral load as compared to the single agents and the enhancement was maintained at a lower level when Dex was at the 10 µM level. The combination of INS-104 with remdesivir had an enhancement in the decrease of viral load as compared to the single agents with INS-104 at 5 µM when remdesivir was provided at 10 nM.

**Table 17. Combination Data for INS-104**

| **INS-104** | **Dex 0.1 µM** | **Dex 1 µM** | **Dex 10 µM** |
|---|---|---|---|
| hi 5 µM | - | E | E |
| med 1 µM | - | E | (e) |
| low 0.2 uM | - | (e) | - |
| | | | |

| **INS-104** | **Remde 1** | **Remde 10** | **Remde 100** |
|---|---|---|---|
| hi 5 µM | - | E | - |
| med 1 µM | - | (e) | - |
| low 0.2 µM | - | - | (e) |

The data below shows the sequences for primers and probes.

| Primers and Probes | |
|---|---|
| Name | Sequences (5'-3') |
| ORF1ab gene / nCov | |
| ORF1ab_Fw | CCGCAAGGTTCTTCTTCGTAAG |
| ORF1ab_Rv | TGCTATGTTTAGTGTTCCAGTTTTC |
| ORF1ab_probe | AAGGATCAGTGCCAAGCTCGCC [5'] Hex [3'] BHQ-1 |

SARS-CoV-2 possesses an almost 30 kbp long genome. The genome contains open-reading fram 1ab (ORF1ab) gene, the largest one of SARS-CoV-2, encoding polyprotein PPlab and PP1a responsible for viral transicription and replication.

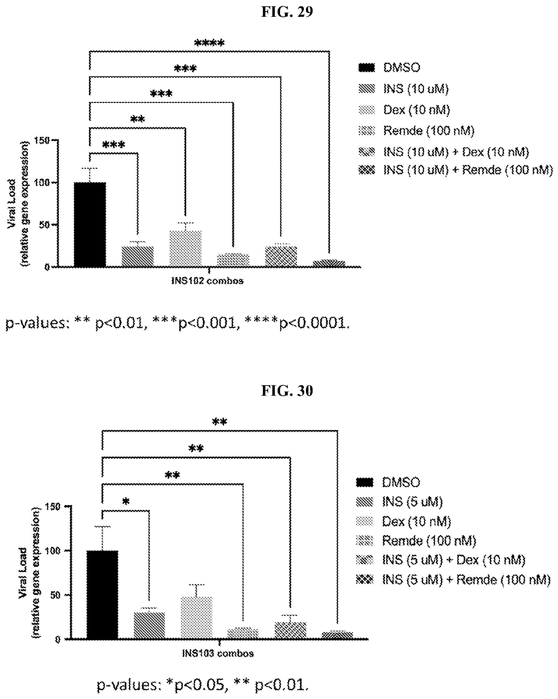

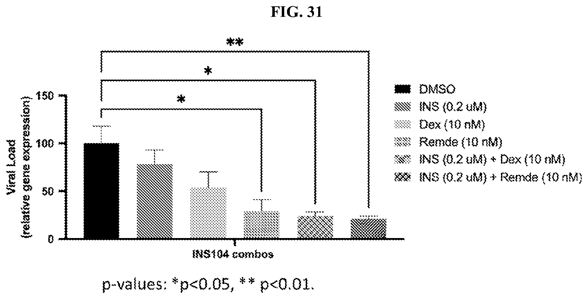

Results of the treatment study are shown in **FIGs. 29-31****.**

While particular alternatives of the present disclosure have been disclosed, it is to be understood that various modifications and combinations are possible and are contemplated within the true spirit and scope of the appended claims. There is no intention, therefore, of limitations to the exact abstract and disclosure herein presented.

## Claims

1. A formulation for use in a method for reducing viral respiratory infection in a subject in need thereof, the method comprising:
administering the formulation intranasally or by inhalation to the subject having a viral respiratory infection, wherein the viral respiratory infection is coronavirus or SARS-CoV-2, wherein the formulation comprises
a therapeutically effective amount of a statin; and
a pharmaceutically acceptable carrier.

2. The formulation for use of claim 1, wherein the viral respiratory infection is COVID-19.

3. The formulation for use of claim 1 or claim 2, wherein the formulation is administered intranasally.

4. The formulation for use of claim 1 or claim 2, wherein the formulation is administered by inhalation,
optionally wherein the administration is by a mechanical inhaler,
further optionally wherein the mechanical inhaler is a metered-dose powder inhaler, a pressurized aerosol inhaler, a dry powder inhaler, or a nebulizer.

5. The formulation for use of any one of claims 1 to 4, wherein the statin is selected from the group consisting of:
(i) simvastatin, pitavastatin, rosuvastatin, atorvastatin, lovastatin, fluvastatin, mevastatin, cerivastatin, tenivastatin, and pravastatin; or
(ii) simvastatin, pitavastatin, rosuvastatin, and atorvastatin; or
(iii) pitavastatin and simvastatin.

6. The formulation for use of any one of claims 1 to 5, wherein the therapeutically effective amount is:
(i) between about 0.005 µg and about 40 mg; or
(ii) between about 0.5 µg and about 15 mg; or
(iii) between about 1.0 µg and about 10 mg; or
(iv) between about 1.0 µg and about 5 mg.

7. The formulation for use of any one of claims 1 to 6, further comprising administering at least one additional therapeutic agent.

8. The formulation for use of claim 7, wherein each additional therapeutic agent is:
(i) independently selected from the group consisting of RNA polymerase inhibitors; inhibitors of a viral protease; inhibitors of a host protease; TMPRSS2 inhibitors; antiviral agents; chloroquine or a salt thereof, hydroxychloroquine or a salt thereof, amantadine, rimantadine, lopinavir, ritonavir, umifenovir, remdesivir, favipiravir, nelfinavir mesylate, azithromycin, bafilomycin, camostat or a salt thereof, darunavir, oseltamivir, ribavirin, convalescent plasma or antibodies extracted therefrom; selinexor; inhaled nitric oxide; exosomes and/or microvesicles; and cord blood regulatory T cells; and/or
(ii) nelfinavir mesylate, azithromycin, bafilomycin, camostat mesylate, camostat or a camostat salt, arbidol, amantadine, rimantadine, lopinavir, darunavir, ribavirin, remdesivir, favipirvir, chloroquine, hydroxychloroquine, tocilizumab, or sarilumab,
optionally wherein the additional therapeutic agent is remdesivir.

9. The formulation for use of claim 7, wherein:
(i) each additional therapeutic agent is selected from the group consisting of β-agonists; corticosteroids; muscarinic antagonists; RhoA inhibitors; GGTase-I or -II inhibitors; ROCK1 and/or ROCK2 inhibitors; soluble epoxide hydrolase inhibitors; fatty acid amide hydrolase inhibitors; leukotriene receptor antagonists; phosphodiesterase-4 inhibitors such as roflumilast; 5-lipoxygenase inhibitors such as zileuton; mast cell stabilizers such as nedocromil; squalene synthase inhibitors such as lapaquistat, zaragozic acid, and RPR 107393; inhibitors of farnesyl pyrophosphate synthase, including without limitation bisphosphonates such as alendronate, etidronate, clodronate, tiludronate, pamidronate, neridronate, olpadronate, ibadronate, risedronate, zoledronate; theophylline; anti-IL5 antibodies; anti-IgE antibodies; anti-IL5 receptor antibodies; anti-IL13/4 receptor antibodies; biologics such as mepolizumab, reslizumab, benralizumab, omalizumab, and dupilumab; β-agonist and muscarinic antagonist combinations, including both long- and short-acting formulations; β-agonist and corticosteroid combinations, including both long- and short-acting formulations; corticosteroids and muscarinic antagonist combinations, including both long- and short-acting formulations; and β-agonist, corticosteroid, and muscarinic antagonist combinations, including both long- and short-acting formulations,
optionally wherein each additional therapeutic agent is a β-agonist, a corticosteroid, a muscarinic antagonist, or any combination thereof; or
(ii) the additional agent is dexamethasone,
optionally further comprising remdesivir.

10. The formulation for use of any one of claims 7 to 9, wherein each additional therapeutic agent is administered:
(i) intranasally or by inhalation; and/or
(ii) at a sub-therapeutic dose.

11. A formulation for use in a method for treating a viral respiratory infection in a subject in need thereof, wherein the viral respiratory infection is coronavirus or SARS-CoV-2, the method comprising:
administering the formulation intranasally or by inhalation to the subject suffering from the viral respiratory infection or who may be exposed to the viral respiratory infection, wherein the formulation comprises
a therapeutically effective amount of a statin; and
a pharmaceutically acceptable carrier.

12. The formulation for use of claim 11, wherein the formulation is administered prophylactically.

13. The formulation for use of claim 12, wherein the formulation is administered prior to exposure to the viral respiratory infection,
optionally wherein the formulation is administered:
(i) between 1 hour and 7 days before exposure to the viral respiratory infection; or
(ii) between 1 hour and 24 hours before exposure to the viral respiratory infection; or
(iii) between 3 hours and 12 hours before exposure to the viral respiratory infection; or
(iv) about 6 hours before exposure to the viral respiratory infection.

14. The formulation for use of claim 13, wherein the formulation is administered from 1 hour to 24 hours before the potential exposure to the viral respiratory infection, wherein the statin comprises pitavastatin or simvastatin, and wherein the formulation further comprises at least one of remdesivir or dexamethasone.

15. A pharmaceutical formulation for use in the treatment of a viral respiratory disease, wherein the viral respiratory disease is coronavirus or SARS-CoV-2, the composition comprising:
a therapeutically effective amount of a statin, or an enantiomer, or diastereomer thereof, and
a pharmaceutically acceptable carrier suitable for administration by inhalation.

16. A formulation for use in a method selected from Method A, Method B and Method C:
**Method A:** a method for treating a SARS-CoV-2 virus infection in a subject in need thereof, the method comprising:
administering the formulation intranasally or by inhalation to a subject suffering from the viral respiratory infection, wherein the viral respiratory infection is COVID-19, wherein the formulation comprises
a therapeutically effective amount of a statin; and
a pharmaceutically acceptable carrier; or
**Method B:** a method for treating a SARS-CoV-2 virus infection in a subject in need thereof, the method comprising:
administering the formulation intranasally or by inhalation to a subject who may be exposed to a SARS-CoV-2 virus, wherein the formulation comprises
a therapeutically effective amount of a statin; and
a pharmaceutically acceptable carrier; or
Method C: a method for reducing the severity of COVID-19 in a subject infected with SARS-CoV-2, the method comprising:
administering the formulation intranasally or by inhalation to the infected subject, wherein the formulation comprises
a therapeutically effective amount of a statin; and
a pharmaceutically acceptable carrier.

## Patentansprüche

1. Formulierung zur Verwendung in einem Verfahren zum Reduzieren einer viralen Atemwegsinfektion bei einem Subjekt, das dessen bedarf, wobei das Verfahren Folgendes umfasst:
Verabreichen der Formulierung intranasal oder durch Inhalation an das Subjekt mit einer viralen Atemwegsinfektion, wobei die virale Atemwegsinfektion Coronavirus oder SARS-CoV-2 ist, wobei die Formulierung Folgendes umfasst:
eine therapeutisch wirksame Menge eines Statins; und
einen pharmazeutisch unbedenklichen Träger.

2. Formulierung zur Verwendung nach Anspruch 1, wobei die virale Atemwegsinfektion COVID-19 ist.

3. Formulierung zur Verwendung Anspruch 1 oder Anspruch 2, wobei die Formulierung intranasal verabreicht wird.

4. Formulierung zur Verwendung Anspruch 1 oder Anspruch 2, wobei die Formulierung durch Inhalation verabreicht wird,
wobei die Verabreichung optional durch einen mechanischen Inhalator erfolgt,
wobei der mechanische Inhalator ferner optional ein Dosierpulverinhalator, ein unter Druck stehender Aerosolinhalator, ein Trockenpulverinhalator oder ein Vernebler ist.

5. Formulierung zur Verwendung nach einem der Ansprüche 1 bis 4, wobei das Statin aus der Gruppe ausgewählt ist, die aus Folgendem besteht:
(i) Simvastatin, Pitavastatin, Rosuvastatin, Atorvastatin, Lovastatin, Fluvastatin, Mevastatin, Cerivastatin, Tenivastatin und Pravastatin; oder
(ii) Simvastatin, Pitavastatin, Rosuvastatin und Atorvastatin; oder
(iii) Pitavastatin und Simvastatin.

6. Formulierung zur Verwendung nach einem der Ansprüche 1 bis 5, wobei die therapeutisch wirksame Menge Folgendes beträgt:
(i) zwischen etwa 0,005 µg und etwa 40 mg; oder
(ii) zwischen etwa 0,5 µg und etwa 15 mg; oder
(iii) zwischen etwa 1,0 µg und etwa 10 mg; oder
(iv) zwischen etwa 1,0 µg und etwa 5 mg.

7. Formulierung zur Verwendung nach einem der Ansprüche 1 bis 6, ferner umfassend Verabreichen mindestens eines zusätzlichen therapeutischen Mittels.

8. Formulierung zur Verwendung nach Anspruch 7, wobei jedes zusätzliche therapeutische Mittel Folgendes ist:
(i) unabhängig aus der Gruppe ausgewählt, die aus Folgendem besteht: RNA-Polymerase-Inhibitoren; Inhibitoren einer viralen Protease; Inhibitoren einer Wirtsprotease; TMPRSS2-Inhibitoren; antiviralen Mitteln; Chloroquin oder einem Salz davon, Hydroxychloroquin oder einem Salz davon, Amantadin, Rimantadin, Iopinavir, Ritonavir, Umifenovir, Remdesivir, Favipiravir, Nelfinavir-Mesylat, Azithromycin, Bafilomycin, Camostat oder einem Salz davon, Darunavir, Oseltamivir, Ribavirin, Rekonvaleszenzplasma oder daraus extrahierten Antikörpern; Selinexor; inhaliertem Stickstoffmonoxid; Exosomen und/oder Mikrovesikel; und regulatorischen T-Zellen des Nabelschnurbluts; und/oder
(ii) Nelfinavir-Mesylat, Azithromycin, Bafilomycin, Camostat-Mesylat, Camostat oder ein Camostatsalz, Arbidol, Amantadin, Rimantadin, Iopinavir, Darunavir, Ribavirin, Remdesivir, Favipirvir, Chloroquin, Hydroxychloroquin, Tocilizumab oder Sarilumab, wobei das zusätzliche therapeutische Mittel optional Remdesivir ist.

9. Formulierung zur Verwendung nach Anspruch 7, wobei:
(i) jedes zusätzliche therapeutische Mittel aus der Gruppe ausgewählt ist, die aus Folgendem besteht: β-Agonisten; Kortikosteroiden; muskarinischen Antagonisten; RhoA-Inhibitoren; GGTase-I- oder -II-Inhibitoren; ROCK1- und/oder ROCK2-Inhibitoren; löslichen Epoxidhydrolase-Inhibitoren; Fettsäureamidhydrolase-Inhibitoren; Leukotrienrezeptor-Antagonisten; Phosphodiesterase-4-Inhibitoren, wie etwa Roflumilast; 5-Lipoxygenase-Inhibitoren, wie etwa Zileuton; Mastzellstabilisatoren, wie etwa Nedocromil; Squalensynthase-Inhibitoren, wie etwa Iapaquistat, Zaragozinsäure und RPR 107393; Inhibitoren der Farnesylpyrophosphat-Synthase, einschließlich unter anderem Bisphosphonaten, wie etwa Alendronat, Etidronat, Clodronat, Tiludronat, Pamidronat, Neridronat, Olpadronat, Ibadronat, Risedronat, Zoledronat; Theophyllin; anti-IL5-Antikörpern; Anti-IgE-Antikörpern; Anti-IL5-Rezeptor-Antikörpern; Anti-IL13-4-Rezeptor-Antikörpern; Biologien, wie etwa Mepolizumab, Reslizumab, Benralizumab, Omalizumab und Dupilumab; β-Agonisten- und Muscarin-Antagonisten-Kombinationen, einschließlich sowohl lang- als auch kurzwirksamer Formulierungen; β-Agonisten- und Kortikosteroid-Kombinationen, einschließlich sowohl langals auch kurzwirksamer Formulierungen; Kombinationen von Kortikosteroiden und muskarinischen Antagonisten, einschließlich sowohl lang- als auch kurzwirksamer Formulierungen; und Kombinationen von β-Agonisten, Kortikosteroiden und muskarinischen Antagonisten, einschließlich sowohl lang- als auch kurzwirksamer Formulierungen,
wobei jedes zusätzliche therapeutische Mittel optional ein β-Agonist, ein Kortikosteroid, ein Muscarin-Antagonist oder eine beliebige Kombination davon ist; oder
(ii) das zusätzliche Mittel Dexamethason ist,
optional ferner umfassend Remdesivir.

10. Formulierung zur Verwendung nach einem der Ansprüche 7 bis 9, wobei jedes zusätzliche therapeutische Mittel wie folgt verabreicht wird:
(i) intranasal oder durch Inhalation; und/oder
(ii) in einer subtherapeutischen Dosis.

11. Formulierung zur Verwendung in einem Verfahren zum Behandeln einer viralen Atemwegsinfektion bei einem Subjekt, das dessen bedarf, wobei die virale Atemwegsinfektion Coronavirus oder SARS-CoV-2 ist, wobei das Verfahren Folgendes umfasst:
Verabreichen der Formulierung intranasal oder durch Inhalation an das Subjekt, das an der viralen Atemwegsinfektion leidet oder das der viralen Atemwegsinfektion ausgesetzt sein kann, wobei die Formulierung Folgendes umfasst:
eine therapeutisch wirksame Menge eines Statins; und
einen pharmazeutisch unbedenklichen Träger.

12. Formulierung zur Verwendung nach Anspruch 11, wobei die Formulierung prophylaktisch verabreicht wird.

13. Formulierung zur Verwendung nach Anspruch 12, wobei die Formulierung vor der Exposition gegenüber der viralen Atemwegsinfektion verabreicht wird,
wobei die Formulierung optional wie folgt verabreicht wird:
(i) zwischen 1 Stunde und 7 Tagen vor der Exposition gegenüber der viralen Atemwegsinfektion; oder
(ii) zwischen 1 Stunde und 24 Stunden vor der Exposition gegenüber der viralen Atemwegsinfektion; oder
(iii) zwischen 3 Stunden und 12 Stunden vor der Exposition gegenüber der viralen Atemwegsinfektion; oder
(iv) etwa 6 Stunden vor der Exposition gegenüber der viralen Atemwegsinfektion.

14. Formulierung zur Verwendung nach Anspruch 13, wobei die Formulierung 1 Stunde bis 24 Stunden vor der potenziellen Exposition gegenüber der viralen Atemwegsinfektion verabreicht wird, wobei das Statin Pitavastatin oder Simvastatin umfasst und wobei die Formulierung ferner mindestens eines von Remdesivir oder Dexamethason umfasst.

15. Pharmazeutische Formulierung zur Verwendung bei der Behandlung einer viralen Atemwegserkrankung, wobei die virale Atemwegserkrankung Coronavirus oder SARS-CoV-2 ist, wobei die Zusammensetzung Folgendes umfasst:
eine therapeutisch wirksame Menge eines Statins oder eines Enantiomers oder Diastereomers davon und
einen pharmazeutisch unbedenklichen Träger, der zur Verabreichung durch Inhalation geeignet ist.

16. Formulierung zur Verwendung in einem Verfahren, das aus Verfahren A, Verfahren B und Verfahren C ausgewählt ist:
**Verfahren A:** ein Verfahren zum Behandeln einer SARS-CoV-2-Virusinfektion bei einem Subjekt, das dessen bedarf, wobei das Verfahren Folgendes umfasst:
Verabreichen der Formulierung intranasal oder durch Inhalation an ein Subjekt, das an der viralen Atemwegsinfektion leidet, wobei die virale Atemwegsinfektion COVID-19 ist, wobei die Formulierung Folgendes umfasst:
eine therapeutisch wirksame Menge eines Statins; und
einen pharmazeutisch unbedenklichen Träger; oder
**Verfahren B:** ein Verfahren zum Behandeln einer SARS-CoV-2-Virusinfektion bei einem Subjekt, das dessen bedarf, wobei das Verfahren Folgendes umfasst:
Verabreichen der Formulierung intranasal oder durch Inhalation an ein Subjekt, das einem SARS-CoV-2-Virus ausgesetzt sein kann, wobei die Formulierung Folgendes umfasst:
eine therapeutisch wirksame Menge eines Statins; und
einen pharmazeutisch unbedenklichen Träger; oder
**Verfahren C:** ein Verfahren zum Reduzieren des Schweregrads von COVID-19 bei einem Subjekt, das mit SARS-CoV-2 infiziert ist, wobei das Verfahren Folgendes umfasst:
Verabreichen der Formulierung intranasal oder durch Inhalation an das infizierte Subjekt, wobei die Formulierung Folgendes umfasst:
eine therapeutisch wirksame Menge eines Statins; und
einen pharmazeutisch unbedenklichen Träger.

## Revendications

1. Formulation destinée à être utilisée dans un procédé de réduction d'une infection respiratoire virale chez un sujet en ayant besoin, le procédé comprenant :
l'administration de la formulation par voie intranasale ou par inhalation au sujet présentant une infection respiratoire virale, ladite infection respiratoire virale étant le coronavirus ou le SARS-CoV-2, ladite formulation comprenant
une quantité thérapeutiquement efficace d'une statine ; et
un support pharmaceutiquement acceptable.

2. Formulation destinée à être utilisée selon la revendication 1, ladite infection respiratoire virale étant la COVID-19.

3. Formulation destinée à être utilisée selon la revendication 1 ou la revendication 2, ladite formulation étant administrée par voie intranasale.

4. Formulation destinée à être utilisée selon la revendication 1 ou la revendication 2, ladite formulation étant administrée par inhalation,
éventuellement ladite administration étant effectuée par un inhalateur mécanique,
en outre éventuellement, ledit inhalateur mécanique étant un inhalateur doseur de poudre, un inhalateur d'aérosol pressurisé, un inhalateur de poudre sèche ou un nébuliseur.

5. Formulation destinée à être utilisée selon l'une quelconque des revendications 1 à 4, ladite statine étant choisie dans le groupe constitué de :
(i) simvastatine, pitavastatine, rosuvastatine, atorvastatine, lovastatine, fluvastatine, mévastatine, cérivastatine, ténivastatine et pravastatine ; ou
(ii) simvastatine, pitavastatine, rosuvastatine et atorvastatine ; ou
(iii) pitavastatine et simvastatine.

6. Formulation destinée à être utilisée selon l'une quelconque des revendications 1 à 5, ladite quantité thérapeutiquement efficace étant :
(i) entre environ 0,005 µg et environ 40 mg ; ou
(ii) entre environ 0,5 µg et environ 15 mg ; ou
(iii) entre environ 1 µg et environ 10 mg ; ou
(iv) entre environ 1 µg et environ 5 mg.

7. Formulation destinée à être utilisée selon l'une quelconque des revendications 1 à 6, comprenant en outre l'administration d'au moins un agent thérapeutique supplémentaire.

8. Formulation destinée à être utilisée selon la revendication 7, chaque agent thérapeutique supplémentaire étant :
(i) indépendamment choisi dans le groupe constitué des inhibiteurs de l'ARN polymérase ; des inhibiteurs d'une protéase virale ; des inhibiteurs d'une protéase hôte ; des inhibiteurs de TMPRSS2 ; des agents antiviraux ; de la chloroquine ou un sel de celle-ci, de l'hydroxychloroquine ou un sel de celle-ci, de l'amantadine, de la rimantadine, de lopinavir, du ritonavir, de l'umifénovir, du remdésivir, du favipiravir, du mésylate de nelfinavir, de l'azithromycine, de la bafilomycine, du camostat ou un sel de celui-ci, du darunavir, de l'oseltamivir, de la ribavirine, du plasma de convalescence ou des anticorps extraits de ceux-ci ; du selinexor ; de l'oxyde nitrique inhalé ; des exosomes et/ou des microvésicules ; et des lymphocytes T régulateurs du sang de cordon ; et/ou
(ii) le mésylate de nelfinavir, l'azithromycine, la bafilomycine, le mésylate de camostat, le camostat ou un sel de camostat, l'arbidol, l'amantadine, la rimantadine, le lopinavir, le darunavir, la ribavirine, le remdésivir, le favipirvir, la chloroquine, l'hydroxychloroquine, le tocilizumab ou le sarilumab,
éventuellement ledit agent thérapeutique supplémentaire étant le remdésivir.

9. Formulation destinée à être utilisée selon la revendication 7 :
(i) chaque agent thérapeutique supplémentaire étant choisi dans le groupe constitué des β-agonistes ; des corticostéroïdes ; des antagonistes muscariniques ; des inhibiteurs de RhoA ; des inhibiteurs de GGTase-I ou -II ; des inhibiteurs de ROCK1 et/ou ROCK2 ; des inhibiteurs de l'époxyde hydrolase soluble ; des inhibiteurs de l'amide hydrolase d'acide gras ; des antagonistes des récepteurs du leucotriène ; des inhibiteurs de la phosphodiestérase-4 tels que le roflumilast ; des inhibiteurs de la 5-lipoxygénase tels que le zileuton ; des stabilisateurs de mastocytes tels que le nédocromil ; des inhibiteurs de la squalène synthase tels que le lapaquistat, l'acide zaragozique et le RPR 107393 ; des inhibiteurs de la farnésyl pyrophosphate synthase, y compris, sans limitation, des bisphosphonates tels que l'alendronate, l'étidronate, le clodronate, le tiludronate, le pamidronate, le néridronate, l'olpadronate, l'ibadronate, le risédronate, le zolédronate ; la théophylline ; des anticorps anti-IL5 ; des anticorps anti-IgE ; des anticorps anti-récepteurs de l'IL5 ; des anticorps anti-récepteurs de l'IL13/4 ; des produits biologiques tels que le mepolizumab, le reslizumab, le benralizumab, l'omalizumab et le dupilumab ; des combinaisons de β-agonistes et d'antagonistes muscariniques, y compris les formulations à action prolongée et à action rapide ; des combinaisons de β-agonistes et de corticostéroïdes, y compris les formulations à action prolongée et à action rapide ; des combinaisons de corticostéroïdes et d'antagonistes muscariniques, y compris les formulations à action prolongée et à action rapide ; et les combinaisons de β-agonistes, de corticostéroïdes et d'antagonistes muscariniques, y compris les formulations à action prolongée et à action rapide,
éventuellement chaque agent thérapeutique supplémentaire étant un β-agoniste, un corticostéroïde, un antagoniste muscarinique ou toute combinaison de ceux-ci ; ou
(ii) l'agent supplémentaire étant la dexaméthasone,
éventuellement comprenant en outre du remdésivir.

10. Formulation destinée à être utilisée selon l'une quelconque des revendications 7 à 9, chaque agent thérapeutique supplémentaire étant administré :
(i) par voie intranasale ou par inhalation ; et/ou
(ii) à une dose sous-thérapeutique.

11. Formulation destinée à être utilisée dans un procédé de traitement d'une infection respiratoire virale chez un sujet en ayant besoin, ladite infection respiratoire virale étant le coronavirus ou le SARS-CoV-2, le procédé comprenant :
l'administration de la formulation par voie intranasale ou par inhalation au sujet souffrant de l'infection respiratoire virale ou pouvant être exposé à l'infection respiratoire virale, ladite formulation comprenant
une quantité thérapeutiquement efficace d'une statine ; et
un support pharmaceutiquement acceptable.

12. Formulation destinée à être utilisée selon la revendication 11, ladite formulation étant administrée de manière prophylactique.

13. Formulation destinée à être utilisée selon la revendication 12, ladite formulation étant administrée avant l'exposition à l'infection respiratoire virale,
éventuellement, ladite formulation étant administrée :
(i) entre 1 heure et 7 jours avant l'exposition à l'infection respiratoire virale ; ou
(ii) entre 1 heure et 24 heures avant l'exposition à l'infection respiratoire virale ; ou
(iii) entre 3 heures et 12 heures avant l'exposition à l'infection respiratoire virale ; ou
(iv) environ 6 heures avant l'exposition à l'infection respiratoire virale.

14. Formulation destinée à être utilisée selon la revendication 13, ladite formulation étant administrée de 1 heure à 24 heures avant l'exposition potentielle à l'infection respiratoire virale, dans laquelle la statine comprend de la pitavastatine ou de la simvastatine, et ladite formulation comprenant en outre au moins l'un parmi le remdésivir ou la dexaméthasone.

15. Formulation pharmaceutique destinée à être utilisée dans le traitement d'une maladie respiratoire virale, ladite maladie respiratoire virale étant le coronavirus ou le SARS-CoV-2, la composition comprenant :
une quantité thérapeutiquement efficace d'une statine, ou d'un énantiomère, ou d'un diastéréomère de celle-ci, et
un support pharmaceutiquement acceptable adapté à l'administration par inhalation.

16. Formulation destinée à être utilisée dans un procédé choisi parmi le procédé A, le procédé B et le procédé C :
**Procédé A :** procédé de traitement d'une infection par le virus SARS-CoV-2 chez un sujet en ayant besoin, le procédé comprenant :
l'administration de la formulation par voie intranasale ou par inhalation à un sujet souffrant de l'infection respiratoire virale, ladite infection respiratoire virale étant la COVID-19, ladite formulation comprenant
une quantité thérapeutiquement efficace d'une statine ; et
un support pharmaceutiquement acceptable ; ou
**Procédé B :** procédé de traitement d'une infection par le virus SARS-CoV-2 chez un sujet en ayant besoin, le procédé comprenant :
l'administration de la formulation par voie intranasale ou par inhalation à un sujet pouvant être exposé à un virus SARS-CoV-2, ladite formulation comprenant
une quantité thérapeutiquement efficace d'une statine ; et
un support pharmaceutiquement acceptable ; ou
**Procédé C :** procédé de réduction de la gravité de la COVID-19 chez un sujet infecté par le SARS-CoV-2, le procédé comprenant :
l'administration de la formulation par voie intranasale ou par inhalation au sujet infecté, ladite formulation comprenant
une quantité thérapeutiquement efficace d'une statine ; et
un support pharmaceutiquement acceptable.
